# EUROPÄISCHE PATENTSCHRIFT

(11) **EP 2 822 924 B1**
(45) Veröffentlichungstag und Bekanntmachung des Hinweises auf die Patenterteilung: **04.12.2019**
(21) Anmeldenummer: 13710324.8
(22) Anmeldetag: 07.03.2013
(51) Int. Cl.: C07C 209/68, C07C 211/45, C07C 211/52, C07C 213/08, C07C 217/84, C07C 253/30, C07C 255/58, C07C 217/80

(54) **VERFAHREN ZUR SYNTHESE VON AMINOBIPHENYLEN UNTER VERWENDUNG VON ARYLHYDRAZINEN**
PROCESS FOR THE SYNTHESIS OF AMINOBIPHENYLS USING ARYLHYDRAZINES
PROCÉDÉ POUR LA SYNTHÈSE DES AMINOBIPHÉNYLS EN UTILISANT DES ARYLHYDRAZINES

(30) Priorität: 07.03.2012 EP 12001532
(43) Veröffentlichungstag der Anmeldung: 14.01.2015
(73) Patentinhaber: BASF SE, 67056 Ludwigshafen am Rhein (DE)
(72) Erfinder: HEINRICH, Markus, 91094 Langensendelbach (DE); JASCH, Hannelore, 91052 Erlangen (DE); HÖFLING, Sarah, 85757 Karlsfeld (DE)
(86) Internationale Anmeldenummer: PCT/EP2013/054605
(87) Internationale Veröffentlichungsnummer: WO 2013/132006

(56) Entgegenhaltungen:
- WO-A1-2010/000856
- NEFEDOV, V.A. ET AL.: "Arylation of azulene", ZHURNAL ORGANICHESKOI KHIMII, MAIK NAUKA, MOSCOW, RU, Bd. 23, Nr. 1, 1987, Seiten 172-181, XP009170648, ISSN: 0514-7492
- HÖFLING, S.B. ET AL.: "4-Substituted tert-Butyl Phenylazocarboxylates-Synthetic Equivalents for the para-Phenyl Radical Cation", ANGEWANDTE CHEMIE INTERNATIONAL EDITION, Bd. 49, Nr. 50, 10. Dezember 2010 (2010-12-10), Seiten 9769-9772, XP55068261, ISSN: 1433-7851, DOI: 10.1002/anie.201004508 -& HÖFLING, S.B. ET AL.: "4-Substituted tert-Butyl Phenylazocarboxylates-Synthetic Equivalents for the para-Phenyl Radical Cation; Supporting Information", ANGEWANDTE CHEMIE INTERNATIONAL EDITION, Bd. 49, Nr. 50, 10. Dezember 2010 (2010-12-10), Seiten S1-S91, XP55068360, ISSN: 1433-7851, DOI: 10.1002/anie.201004508
- DEMIR, A.S. ET AL.: "Potassium permanganate/carboxylic acid/organic solvent: a powerful reagent for enone oxidation and aryl coupling reactions", TETRAHEDRON, Bd. 64, Nr. 27, 30. Juni 2008 (2008-06-30) , Seiten 6196-6201, XP022695590, ISSN: 0040-4020, DOI: 10.1016/J.TET.2008.05.004 [gefunden am 2008-05-03] in der Anmeldung erwähnt
- DEMIR, A.S. ET AL.: "Manganese(III) acetate-mediated oxidative coupling of phenylhydrazines with benzene: a novel method for biaryl coupling", JOURNAL OF THE CHEMICAL SOCIETY, PERKIN TRANSACTIONS 1, Nr. 22, 15. November 2001 (2001-11-15), Seiten 3042-3045, XP55068468, ISSN: 1472-7781, DOI: 10.1039/b105119a in der Anmeldung erwähnt
- WETZEL, A. ET AL.: "Synthesis of amino- and hydroxybiphenyls by radical chain reaction of arenediazonium salts", ANGEWANDTE CHEMIE. INTERNATIONAL EDITION, Bd. 47, Nr. 47, 2008, Seiten 9130-9133, XP002544312, ISSN: 1433-7851, DOI: 10.1002/ANIE.200803785

## Beschreibung

Die vorliegende Erfindung beschreibt ein Verfahren zur Synthese von Aminobiphenylen sowie Derivaten davon. Dieses Verfahren ist kostengünstig durchführbar und beruht auf selektiven Umsetzungen. Funktionalisierte Biphenylverbindungen sind insbesondere als Pharmazeutika und Pflanzenschutzmittel sowie als Vorstufen solcher Wirkstoffe von großem Interesse.

Eine breite Palette metallorganischer Methoden steht heute zur milden und effizienten Synthese von Biarylverbindungen zur Verfügung. [L. Ackermann, Modern Arylation Methods, 1st ed., Wiley-VCH, Weinheim, 2009.]

Die bekannten metallorganischen Methoden sind allerdings auch mit Nachteilen behaftet. Ihre Attraktivität wird beispielsweise durch hohe Kosten der Ausgangsmaterialien, insbesondere bei Palladium-katalysierten Umsetzungen, oder mangelnde Umweltverträglichkeit, wie im Falle des Nickels, gemindert. Katalytische Verfahren unter Verwendung von Cobalt- und Eisenverbindungen sind bisher nur eingeschränkt anwendbar. Einfachere Ausgangsmaterialien können eingesetzt werden, wenn die Biarylkupplung über eine CH-Bindungsaktivierung am Aromaten erfolgt. [G. Dyker, Handbook of C-H Transformations, Wiley-VCH, Weinheim, 2005.] Trotz zahlreicher aktueller Arbeiten auf diesem Forschungsgebiet ist das verwendbare Substratspektrum bisher noch sehr begrenzt.

Verglichen mit der Vielfalt an metallorganischen Transformationen, die im Wesentlichen in den letzten zwei Jahrzehnten entwickelt wurden, kommen Additionsreaktionen von Arylradikalen an aromatische Substrate aktuell nur selten zum Einsatz.

Dabei liegen die Pionierarbeiten von Pschorr, Gomberg und Bachmann auf dem Gebiet der radikalischen Biarylsynthese, in der traditionell Aryldiazoniumsalze als Radikalvorläufer eingesetzt werden, bereits lange zurück. [M. Gomberg, W. E. Bachmann, J. Am. Chem. Soc. 1924, 46, 2339-2343, R. Pschorr, Chem. Ber. 1896, 29, 496-501] Ein grundlegender Nachteil der intermolekularen Reaktionsführung besteht allerdings darin, dass die Addition von Arylradikalen an gängige Substrate wie substituierte Benzole meist nur langsam erfolgt, was in der Folge Nebenreaktionen begünstigt. [J. C. Scaiano, L. C. Stewart, J. Am. Chem. Soc. 1983, 105, 3609-3614] Der Erfolg radikalischer Biarylsynthesen ist deshalb häufig an spezielle Bedingungen geknüpft, wobei das Substrat als Lösungsmittel eingesetzt [A. Nunez, A. Sanchez, C. Burgos, J. Alvarez-Builla, Tetrahedron 2004, 60, 6217-6224, P. T. F. McLoughlin, M. A. Clyne, F. Aldabbagh, Tetrahedron 2004, 60, 8065-8071] oder die Reaktion intramolekular durchführt wird [M. L. Bennasar, T. Roca, F. Ferrando, Tetrahedron Lett. 2004, 45, 5605-5609]. Eine Verbesserung der klassischen Gomberg-Bachmann Reaktion konnte auch durch eine Reaktionsführung unter Phasentransferbedingungen erzielt werden. [J. R. Beadle, S. H. Korzeniowski, D. E. Rosenberg, B. J. Garcia-Slanga, G. W. Gokel, J. Org. Chem. 1984, 49, 1594-1603]

Aus kürzlich erschienen Übersichtsartikeln zur radikalischen Biarylsynthese geht hervor, dass in der aktuellen Forschung die klassischen Aryldiazoniumsalze [C. Galli, Chem. Rev. 1988, 88, 765-792] zunehmend durch Arylchloride, -bromide und -iodide als Radikalvorläufer ersetzt werden. [A. Studer, M. Brossart in Radicals in Organic Synthesis, Eds. P. Renaud, M. P. Sibi, 1st ed., Wiley-VCH, Weinheim, 2001, Vol. 2, 62-80; G. Pratsch, M. R. Heinrich, Topics in Current Chemistry, Vol. 320, Eds. M. R. Heinrich, A. Gansäuer] Allerdings müssen zur Generierung von Arylradikalen aus Arylhalogeniden meist toxische Organozinn- oder teure Organosiliciumverbindungen eingesetzt werden. Als Alternativen dazu wurden im Zusammenhang mit Arylbromiden und - iodiden kürzlich auch organokatalytische Biarylsynthesen beschrieben [A. Studer, D. Curran, Angew: Chem. Int. Ed. 2011, 50, 5018-5022].

Wesentlich seltener wurden bisher Arylhydrazine als Vorläufer für Arylradikale eingesetzt. Im Unterschied zu den oben genannten Reaktionen mit Aryldiazoniumsalzen, Arylbromiden und Aryliodiden erfolgt die Generierung der Arylradikale hierbei unter oxidativen Bedingungen. Neben Mangan(III)-acetat [A. S. Demir, Ö. Reis, E. Özgül-Karaaslan, J. Chem. Soc. Perkin Trans. 1 2001, 3042] wurde in Biarylsynthesen bisher Kaliumpermanganat

[A. S. Demir, H. Findik, Tetrahedron 2008, 64, 6196] als Oxidationsmittel beschrieben. In diesen Arbeiten kommen als Reaktionspartner für die Arylhydrazine, bzw. die daraus entstehenden Arylradikale, jedoch nur Benzol, Brombenzol, Toluol und einfache Heterocyclen wie Thiophen und Furan zum Einsatz. [A. S. Demir, Ö. Reis, M. Emrullaho 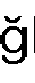 lu *Tetrahedron* **2002,** *58,* 8055].

Im Zusammenhang mit radikalischen Arylierungsreaktionen stellen aber vor allem Aniline eine bedeutende Substratgruppe dar, da aus ihnen 2-Aminobiphenyle hergestellt werden können. Derart funktionalisierte Biphenylverbindungen sind insbesondere als Pharmazeutika und Pflanzenschutzmittel sowie als Vorstufen solcher Wirkstoffe von großem Interesse.

V.A. Nefedov et al. beschreiben in Zhurnal Organicheskoi Khimii 1987, 23(1), 172-181 die Umsetzung von N,N-Dimethylanilin mit p-Nitrophenylhydrazin in Gegenwart von CuF₂ zu einem Gemisch aus 4'-Nitro-2-(N,N-dimethylamino)-biphenyl und 4'-Nitro-4-(N,N-dimethylamino)-biphenyl.

In der Supporting Information zum Artikel von S.B. Höfling et al. in Angew. Chem. Int. Ed. 2010, 49(50), 9769-9772 wird 5,4'-Difluorbiphenyl-2-amin durch Umsetzung von tert-Butyl-2-(2-fluorphenyl)-diazocarboxylat mit 4-Fluoranilin in Gegenwart von H₂O₂ und NaOH hergestellt. tert-Butyl-2-(2-fluorphenyl)-diazocarboxylat wird wiederum durch Oxidation von tert-Butyl-2-(4-fluorphenyl)-hydrazincarboxylat mit MnO₂ hergestellt. tert-Butyl-2-(4-fluorphenyl)hydrazincarboxylat wird durch Umsetzung von 4-Fluorphenylhydrazin mit Boc-Anhydrid gewonnen. Dieses Verfahren benötigt drei separate Schritte, um vom Hydrazin und Anilin zum Biphenylamin zu kommen und muss außerdem Schutzgruppentechniken anwenden.

WO 2010/000856 betrifft ein Verfahren zu Herstellung von Aminobiphenylen durch Umsetzung eines Diazoniumsalzes mit einem Anilin.

Ähnlich beschreiben auch A. Wetzel et al. in Angew. Chem. Int. Ed. 2008, 47(47), 9130-9133 die Umsetzung von Diazoniumsalzen mit Anilinen zu Aminobiphenylen.

Der vorliegenden Erfindung lag die Aufgabe zu Grunde, ein Verfahren zur Herstellung von 2-Aminobiphenylen und Derivaten bereitzustellen, das vorzugsweise auf der radikalischen Arylierung von Anilinverbindungen beruht. Aus Kostengründen sollen als Arylradikalvorläufer einfach zugängliche Arylhydrazine zusammen mit einem günstigen Oxidationsmittel eingesetzt werden.

Die Aufgabe wird durch die im Folgenden näher beschriebenen Verfahren zur Herstellung von 2-Aminobiphenylen gelöst.

Ausführungsformen der Erfindung sind:

Verfahren zur Herstellung einer Verbindung der Formel **3** 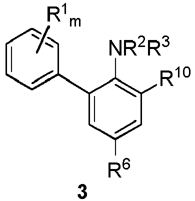 wobei eine Verbindung der Formel **1** 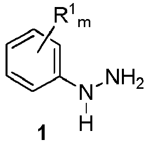 mit einer Verbindung der Formel **2** 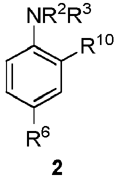 umgesetzt wird, wobei
- m: für 0, 1, 2, 3, 4 oder 5 steht;
- jedes R¹: jeweils unabhängig für Halogen, Alkyl, Haloalkyl, Hydroxy, Hydroxyalkyl, Alkoxy, Haloalkoxy, Alkylthio, Cycloalkyl, Haloalkylthio, Alkenyl, Alkinyl, Amino, Nitro, Cyano, -SO₃R⁵, -SO₂NH₂, -SO₂NHR⁴, -SO₂NR⁴R⁵, -COOR⁴, -CONHR⁴, -CONR⁴R⁵, -COR⁴, -OCOR⁴,-NR⁴R⁵, -NR⁴COR⁵, -NR⁴SO₂R⁵, Alkylcarbonyl, Haloalkylcarbonyl, Alkenylcarbonyl, Alkoxycarbonyl, Haloalkoxycarbonyl, Alkenyloxycarbonyl, Alkylsulfonyl, Haloalkylsulfonyl, Alkylimino, Aryl, Aryloxy, Arylcarbonyl, Arylalkyl, Heteroarylalkyl, Arylalkoxycarbonyl, Arylalkylimino oder Heteroaryl steht;
- R² und R³: für Wasserstoff stehen,
- n: jeweils unabhängig für 1, 2, 3, 4, 5, 6, 7, 8, 9 oder 10 steht;
- R⁴: jeweils unabhängig für Wasserstoff, Alkyl, Cycloalkyl, Haloalkyl, Arylalkyl, Heteroarylalkyl, Aryl oder Heteroaryl steht;
- R⁵: jeweils unabhängig für Wasserstoff, Alkyl, Cycloalkyl, Haloalkyl, Arylalkyl, Heteroarylalkyl, Aryl oder Heteroaryl steht;
- R⁶: jeweils unabhängig für Wasserstoff, Halogen, Alkyl, Alkenyl, Alkinyl, Cycloalkyl, Arylalkyl, Heteroarylalkyl, Haloalkyl, Hydroxyalkyl, Alkoxyalkyl, Aryloxyalkyl, Heteroaryloxyalkyl, Aminoalkyl, -(CH₂)ₙ-NR⁴R⁵, -COOH, -CHO, -CN, -COR⁴, Alkylcarbonyl, Haloalkylcarbonyl, Cycloalkylcarbonyl, Arylalkylcarbonyl, Alkenylcarbonyl, Arylcarbonyl, Heteroarylcarbonyl, -COOR⁴, Alkoxycarbonyl, Haloalkoxycarbonyl, Cycloalkoxycarbonyl, Arylalkoxycarbonyl, Alkenyloxycarbonyl, Aryloxycarbonyl, Heteroaryloxycarbonyl, -CONHR⁴,-CONR⁴R⁵, Amino, Nitro, -NHR⁴, -NR⁴R⁵, 1-Pyrrolidino, 1-Piperidino, 1-Morpholino, Alkylimino, Cycloalkylimino, Haloalkylimino, Arylalkylimino, -NR⁴COR⁵, NR⁴COOR⁵,-NR⁴SO₂R⁵, Hydroxy, Alkoxy, Haloalkoxy, Cycloalkoxy, Arylalkyloxy, Aryloxy, Heteroaryloxy, -OCOR⁴, Alkylcarbonyloxy, Haloalkylcarbonyloxy, Cycloalkylcarbonyloxy, Arylalkylcarbonyloxy, Arylcarbonyloxy, Heteroarylcarbonyloxy, -OCONR⁴R⁵, -O-(CH₂)ₙ-OR⁴, -O-(CH₂)ₙ-NR⁴R⁵, -O-(CH₂)ₙ-NR⁴COR⁵, -O-(CH₂),-NR⁴COOR⁵, -O-(CH₂)ₙ-COOR⁴, -O-(CH₂)ₙ-CONHR⁴, -O-(CH₂)ₙ-CONR⁴R⁵, -O-(CH2)n-SO3R⁴, -O-(CH2),-SO₂R⁴, -O-(CH2)ₙ-CN, -SH, Alkylthio, Haloalkylthio, Cycloalkylthio, Arylalkylthio, Arylthio, Heteroarylthio, Alkylsulfonyl, Haloalkylsulfonyl, Cycloalkylsulfonyl, Arylalkylsulfonyl, Arylsulfonyl, Heteroarylsulfonyl, -SO₂NH₂, -SO₂NHR⁴, -SO₂NR⁴R⁵,-SO₃R⁵, Aryl oder Heteroaryl steht;
- R¹⁰: jeweils unabhängig für Wasserstoff, Halogen, Alkyl, Haloalkyl, Hydroxyalkyl, Cycloalkyl, Arylalkyl, Heteroarylalkyl, (CH₂)_{q}-NR⁴R⁵, -(CH₂)_{q}-NR⁴COR⁵, -(CH₂)_{q}-NR⁴COOR⁵, -(CH₂)_{q}-COOR⁴, -(CH₂)_{q}-CONHR⁴,-(CH₂)_{q}-CONR⁴R⁵, -(CH₂)_{q}-SO₃R⁴, -(CH₂)_{q}-CN, Aryl oder Heteroaryl steht;
- q: jeweils unabhängig für 1, 2, 3, 4, oder 5 steht,
wobei der Alkylrest in Alkoxy, Alkycarbonyl, Alkycarbonyloxy, Alkoxycarbonyl, Alkylsulfonyl, Hydroxyalkyl, Alkoxyalkyl, Aryloxyalkyl, Heteroaryloxyalkyl, Aminoalkyl, Arylalkylcarbonyl, Arylalkoxycarbonyl, Arylalkyloxy, Arylalkyl, Heteroarylalkyl, Arylalkylcarbonyloxy, Alkylthio, Arylalkylthio und Arylalkylsulfonyl gegebenenfalls 1, 2 oder 3 Substituenten trägt, die ausgewählt sind unter Halogen, Alkyl, Haloalkyl, Cycloalkyl, Alkoxy und Haloalkoxy;
wobei Cycloalkyl gegebenenfalls 1, 2 oder 3 Substituenten trägt, die ausgewählt sind unter Alkyl, Alkoxy und Halogen;
wobei der Cycloalkylrest in Cycloalkoxy und Cycloalkylthio gegebenenfalls 1, 2 oder 3 Substituenten trägt, die ausgewählt sind unter Alkyl und Halogen;
wobei der Cycloalkylrest in Cycloalkylcarbonyl, Cycloalkoxycarbonyl, Cycloalkylcarbonyloxy und Cycloalkylsulfonyl gegebenenfalls 1, 2 oder 3 Substituenten trägt, die ausgewählt sind unter Halogen, Alkyl, Haloalkyl, Cycloalkyl, Alkoxy und Haloalkoxy; wobei der Alkenylrest in Alkenylcarbonyl und Alkenyloxycarbonyl gegebenenfalls 1, 2 oder 3 Substituenten trägt, die ausgewählt sind unter Halogen, Alkyl, Haloalkyl, Cycloalkyl, Alkoxy und Haloalkoxy;
wobei der Cycloalkylidenrest in Cycloalkylimino gegebenenfalls 1, 2 oder 3 Substituenten trägt, die ausgewählt sind unter Halogen, Alkyl, Haloalkyl, Cycloalkyl, Alkoxy und Haloalkoxy; und
wobei der Alkylenrest in Alkylimino gegebenenfalls 1, 2 oder 3 Substituenten trägt, die ausgewählt sind unter Halogen, Alkyl, Haloalkyl, Cycloalkyl, Alkoxy und Haloalkoxy;
dadurch gekennzeichnet, dass die Umsetzung in Gegenwart mindestens eines Oxidationsmittels durchgeführt wird, wobei das Oxidationsmittel Sauerstoff, Luftsauerstoff, MnO₂, Mn(OAc)₃, KMnO₄, NalO₄, K₃[Fe(CN)₆], KO₂ oder H₂O₂ ist.

Die Verbindungen der Struktur **3** können beispielsweise als Zwischenprodukte zur Herstellung biologisch aktiver Verbindungen verwendet werden. Beispiele sind die Pflanzenschutzmittel der Strukturen **4, 5** und **6,** die nach bekannten Verfahren aus Verbindungen der Strukturen **7, 8** und **9** gewonnen werden können [Literatur für 7 **→ 4:** DE19735224, 1999, DE1953 1813, 1997; Literatur für **8 → 5:** US 2010174094A1, WO 2006024388A1, US 2008269263A1, US 2010069646A1; Literatur für **9 → 6:** WO20097344A1].

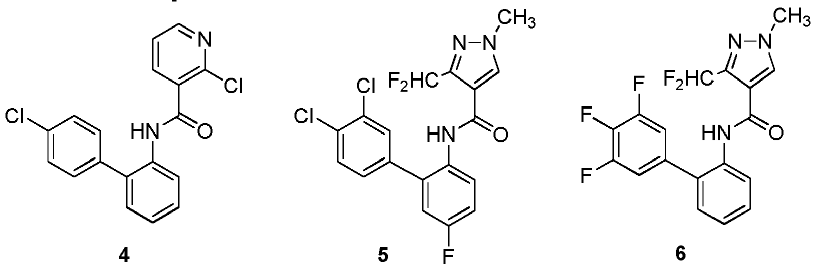

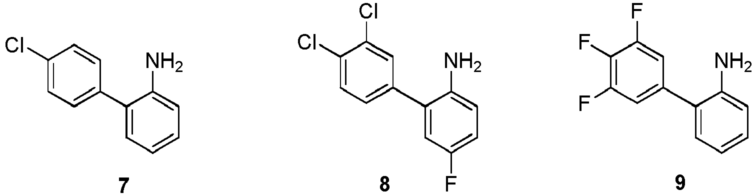

Metallorganische Synthesen der Verbindung der Struktur **8,** die allerdings deutlich teurere Ausgangsmaterialien benötigen als das im Folgenden vorgestellte erfindungsgemäße Verfahren, sind ebenfalls kürzlich beschrieben worden [WO2007138089A1, US2010185015A1].

Ausgehend von der Verbindung der Struktur **10** kann z. B. ein y-Sekretase-lnhibitor der Struktur **11** (LY411575) hergestellt werden [X. Pan, C. S. Wilcox, J. Org. Chem. 2010, 75, 6445-6451].

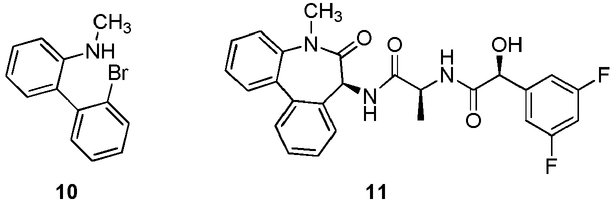

Des Weiteren wird ein Verfahren zur Synthese einer Verbindung der Formel **13** beschrieben, dadurch gekennzeichnet, dass in einem ersten Schritt eine Verbindung der Formel **1** mit einer Verbindung der Formel **2,** wobei R² und R³ = H, zu einer Verbindung der Formel **3,** wobei R² und R³ = H, (vorzugsweise wie oben beschrieben) umgesetzt wird und in einem weiteren Schritt die Verbindung der Formel **3** in eine Verbindung der Formel **12** überführt wird. Die Herstellung von divers funktionalisierten Verbindungen der Formel **12** gelingt unter den allgemein bekannten Bedingungen zur Diazotierung aromatischer Amine.

In einem weiteren Schritt werden die Verbindungen der Formel **12** nach literaturbekannten Verfahren in Verbindungen der Formel **13** überführt. Die folgenden Reaktionen wurden von C. Galli in Chem. Rev. 1988, 88, 765-792 zusammenfasst und beschrieben. Beispielsweise kann die reduktive Dediazotierung zu **13** (R¹¹ = H) unter verschiedensten Reaktionsbedingungen durchgeführt werden. Alternativ sind Halogenverbindungen, Thiole, Thioether, Nitrile, Nitroverbindungen, Sulfonsäurehalogenide und Carbonsäurehalogenide der Formel **13** (R¹¹ = Halogen, -SH, -SAlkyl, -SHaloalkyl,-SAryl, -SHeteroaryl, Cyano, Nitro, -SO₂Hal, -COHal) unter den Bedingungen der Sandmeyer-Reaktion zugänglich [siehe auch: F. Minisci, F. Fontana, E. Vismara, Gazz. Chim. Ital. 1993, 123, 9-18]. Für die Einführung von Fluor (R¹¹ = F) eignet sich speziell die Balz-Schiemann-Reaktion.

Umsetzungen von **12** unter den Bedingungen der Heck-Reaktion liefern alkenylsubstituierte Verbindungen der Formel **13** (R¹¹ = -CR¹⁴=CR¹⁵-COOH, -CR¹⁴=CR¹⁵-COOAlkyl, -CR¹⁴=CR¹⁵-COOHaloalkyl, -CR¹⁴=CR¹⁵-CN) [A. Roglans, A. Pla-Quintana, M. Moreno-Manas, Chem. Rev. 2006, 106, 4622-4643]. Reaktionsbedingungen für die Umsetzungen von Verbindungen der Formel **12** mit aromatischen Substraten unter Erhalt von Verbindungen der Formel **13** (R¹¹ = Aryl, Heteroaryl) werden in der Einleitung und in den folgenden Ausführungen vielfach beschrieben und können entweder radikalisch oder metallkatalysiert (z.B. durch Palladium) durchgeführt werden [Chem. Rev. 2006, 106, 4622-4643].

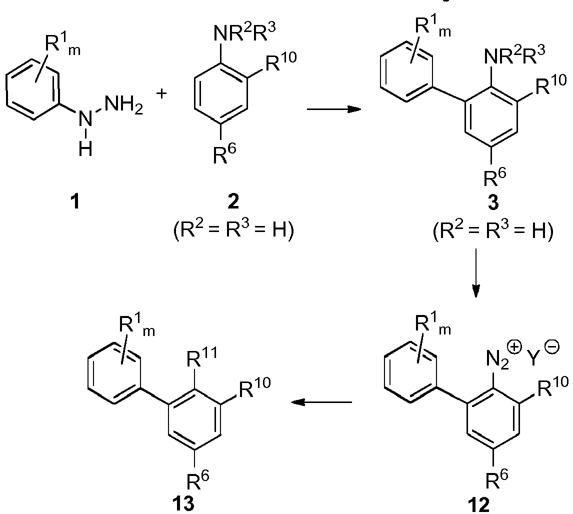

Dabei stehen
- R¹¹: für Wasserstoff, -OH, -SH, -SAlkyl, -SHaloalkyl, -SCycloalkyl, -SO₂Cl, -SO₂Br, -SO₂l, -COCI, -COBr, -COI, Nitro, -S-(CH₂)_{q}-Aryl, -S-(CH₂)_{q}-Heteroaryl, -SAryl, -SHeteroaryl, Halogen, Cyano, -CR¹⁴=CR¹⁵-COOH, -CR¹⁴=CR¹⁵-COOAlkyl, -CR¹⁴=CR¹⁵-COOHaloalkyl, -CR¹⁴=CR¹⁵-CN, -CR¹⁴=CR¹⁵-Aryl, -CR¹⁴=CR¹⁵-Heteroaryl, -SO₂R⁴, Aryl oder Heteroaryl;
- R¹⁴: für Wasserstoff, Alkyl oder Haloalkyl;
- R¹⁵: für Wasserstoff, Alkyl, Haloalkyl, -COOH, -COOAlkyl, -COOHaloalkyl, Cyano, Aryl, Heteroaryl, -NHCOAlkyl oder-NHCOOAlkyl;
- Y-: für Halogenid, Hydrogensulfat, Sulfat, Tetrafluoroborat, Acetat, Trifluoracetat, Hexa-fluorophosphat, Hexafluoroantimonat, das Anion eines aromatischen 1,2-Dicarbonsäureimids oder das Anion eines aromatischen 1,2-Disulfonsäureimids;
und alle weiteren Reste sind wie oben definiert.

Im Rahmen der vorliegenden Erfindung haben die generisch verwendeten Begriffe die folgenden Bedeutungen:
Das Präfix Cₓ-C_{y} bezeichnet im jeweiligen Fall die Anzahl möglicher Kohlenstoffatome.

Der Begriff "Halogen" bezeichnet jeweils Fluor, Brom, Chlor oder Iod, speziell Fluor, Chlor oder Brom, besonders bevorzugt Fluor oder Chlor.

Der Begriff "Alkyl" bezeichnet einen linearen oder verzweigten gesättigten Alkylrest, umfassend 1 bis 20 Kohlenstoffatome, wie Methyl, Ethyl, Propyl, 1-Methylethyl (Isopropyl), Butyl, 1-Methylpropyl (sec-Butyl), 2-Methylpropyl (Isobutyl), 1,1-Dimethylethyl (tert-Butyl), Pentyl, Hexyl, Heptyl, Octyl, 2-Ethylhexyl, Nonyl, Decyl oder 2-Propylheptyl und Stellungsisomere davon, bevorzugt Methyl, Ethyl oder Propyl.

Der Begriff "Haloalkyl", wie hierin und in den Haloalkyleinheiten von Haloalkoxy verwendet, beschreibt geradkettige oder verzweigte gesättigte Alkylgruppen mit 1 bis 10 Kohlenstoffatomen, wobei die Wasserstoffatome dieser Gruppen teilweise oder vollständig durch Halogenatome ersetzt sind. Beispiele hierfür sind Chlormethyl, Brommethyl, Dichlormethyl, Trichlormethyl, Fluormethyl, Difluormethyl, Trifluormethyl, Chlorofluormethyl, Dichlorfluormethyl, Chlordifluormethyl, 1-Chlorethyl,1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2 Dichlor-2-fluorethyl, 2,2,2-Trichlorethyl, Pentafluorethyl, 3,3,3-Trifluorprop-1-yl, 1,1,1-Trifluorprop-2-yl, 3,3,3-Trichlorprop-1-yl, Heptafluorisopropyl, 1-Chlorbutyl, 2-Chlorbutyl, 3-Chlorbutyl, 4-Chlorbutyl, 1-Fluorbutyl, 2-Fluorbutyl, 3-Fluorbutyl, 4-Fluorbutyl und dergleichen, bevorzugt Fluormethyl, 2-Fluorethyl oder Trifluormethyl.

Der Begriff "Alkyliden" oder "Alkylen" bezeichnet über eine Doppelbindung gebundene Alkylreste, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele sind Methyliden (=CH₂), Ethyliden (=CHCH₃), 1-Propyliden (=CHCH₂CH₃) oder 2-Propyliden [=C(CH₃)_{2]}. Im Speziellen ist der Alkylrest unsubstituiert.

Der Begriff "Cycloalkyliden" oder "Cycloalkylen" bezeichnet über eine Doppelbindung gebundene Cycloalkylreste, wobei der Cycloalkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele sind Cyclopentyliden, Cyclohexyliden oder Cycloheptyliden. Im Speziellen ist der Cycloalkylrest unsubstituiert.

Der Begriff "Haloalkyliden" oder "Haloalkylen" bezeichnet über eine Doppelbindung gebundene Haloalkylreste. Beispiele sind Fluormethylen (=CHF), 2-Chlorethyliden (=CH-CH₂Cl), 3-Brom-1-propyliden (=CH₂-CH₂-CH₂Br).

Der Begriff "Arylalkyliden" oder "Arylalkylen" bezeichnet über eine Alkylideneinheit gebundene Arylreste, wobei die Arylgruppe gegebenenfalls 1, 2, 3, 4 oder 5 Substituenten trägt, die ausgewählt sind unter Halogen, Alkyl, Haloalkyl, Alkoxy oder Haloalkoxy. Beispiele sind Benzyliden (=CH-Phenyl), 1-Naphthyliden (=CH-Naphthyl) oder =CH-CH₂-Phenyl.

Der Begriff "Alkenyl" bezeichnet einen einfach ungesättigten, linearen oder verzweigten aliphatischen Rest mit 3 bis 8 Kohlenstoffatomen. Beispiele hierfür sind Propen-1-yl, Propen-2-yl (Allyl), But-1-en-1-yl, But-1-en-2-yl, But-1-en-3-yl, But-1-en-4-yl, But-2-en-1-yl, But-2-en-2-yl, But-2-en-4-yl, 2-Methylprop-1-en-1-yl, 2-Methylprop-2-en-1-yl und dergleichen, bevorzugt Propenyl oder But-1-en-4-yl.

Der Begriff "Cycloalkyl" bezeichnet einen gesättigten alicyclischen Rest mit 3 bis 10 Kohlenstoffatomen als Ringglieder. Beispiele sind Cyclopropyl, Cyclobutyl, Cyclopentyl, Cyclohexyl, Cycloheptyl, Cyclooctyl, Cyclononyl und Cyclodecyl. Die Cycloalkylreste können 1, 2 oder 3 Substituenten tragen, die ausgewählt sind unter Alkyl, Alkoxy oder Halogen, bevorzugt Cyclopropyl, Cyclobutyl, Cyclopentyl oder Cyclohexyl. Im Speziellen ist der Cycloalkylrest unsubstituiert.

Der Begriff "Alkoxy" bezeichnet geradkettige oder verzweigte gesättigte Alkylgruppen, umfassend 1 bis 10 Kohlenstoffatome, die über ein Sauerstoffatom gebunden sind, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele für Alkoxy sind Methoxy, Ethoxy, n-Propoxy, 1-Methylethoxy (Isopropoxy), n-Butoxy, 1-Methylpropoxy (sec-Butoxy), 2-Methylpropoxy (Isobutoxy) und 1,1-Dimethylethoxy (tert-Butoxy), bevorzugt Methoxy, Ethoxy, n-Propoxy, oder -OCH₂-*cyclo*-Pentyl. Im Speziellen ist der Alkylrest unsubstituiert.

Der Begriff "Haloalkoxy" beschreibt geradkettige oder verzweigte gesättigte Haloalkylgruppen, umfassend 1 bis 10 Kohlenstoffatome, die über ein Sauerstoffatom gebunden sind. Beispiele hierfür sind Chlormethoxy, Brommethoxy, Dichlormethoxy, Trichlormethoxy, Fluormethoxy, Difluormethoxy, Trifluormethoxy, Chlorofluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, 1,1,2,2-Tetrafluorethoxy, 1-Chlor-1,2,2-trifluorethoxy, Pentafluorethoxy, 3,3,3-Trifluorprop-1-oxy, 1,1,1-Trifluorprop-2-oxy, 3,3,3-TrichIorprop-1-oxy, 1-Chlorbutoxy, 2-Chlorbutoxy, 3-Chlorbutoxy, 4-Chlorbutoxy, 1-Fluorbutoxy, 2-Fluorbutoxy, 3-Fluorbutoxy, 4-Fluorbutoxy und dergleichen, bevorzugt sind Fluormethoxy, Difluormethoxy oder Trifluormethoxy.

Der Begriff "Cycloalkoxy" bezeichnet einen Cycloalkylrest mit 3 bis 10 Kohlenstoffatomen als Ringglieder, die über ein Sauerstoffatom gebunden sind. Beispiele sind Cyclopropyloxy, Cyclobutyloxy, Cyclopentyloxy, Cyclohexyloxy, Cycloheptyloxy, Cyclooctyloxy, Cyclononyloxy und Cyclodecyloxy. Die Cycloalkylreste können 1, 2 oder 3 Substituenten tragen, die ausgewählt sind unter Alkyl und Halogen. Bevorzugte Cycloalkoxyreste sind Cyclopropyloxy, Cyclobutyloxy, Cyclopentyloxy oder Cyclohexyloxy. Im Speziellen ist der Cycloalkylrest unsubstituiert.

Der Begriff "Alkylcarbonyl" bezeichnet über eine Carbonylgruppe gebundene Alkylreste mit 1 bis 10 Kohlenstoffatomen, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Methylcarbonyl (Acetyl), Ethylcarbonyl, Propylcarbonyl, Isopropylcarbonyl, n-Butylcarbonyl, sec-Butylcarbonyl, Isobutylcarbonyl und tert-Butylcarbonyl, bevorzugt Methylcarbonyl oder Ethylcarbonyl. Im Speziellen ist der Alkylrest unsubstituiert

Der Begriff "Haloalkylcarbonyl" bezeichnet über eine Carbonylgruppe gebundene Haloalkylreste mit 1 bis 10 Kohlenstoffatomen. Beispiele hierfür sind Fluormethylcarbonyl, Difluormethylcarbonyl, Trifluormethylcarbonyl, 1-Fluorethylcarbonyl, 2-Fluorethylcarbonyl, 1,1-Difluorethylcarbonyl, 2,2-Difluorethylcarbonyl, 2,2,2-Trifluorethylcarbonyl, Pentafluor-ethylcarbonyl und dergleichen, bevorzugt sind Fluormethylcarbonyl, Difluormethylcarbonyl oder Trifluormethylcarbonyl.

Der Begriff "Alkylcarbonyloxy" bezeichnet über eine Carbonyloxygruppe gebundene Alkylreste mit 1 bis 10 Kohlenstoffatomen, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Methylcarbonyloxy (Acetoxy), Ethylcarbonyloxy, Propylcarbonyloxy und Isopropylcarbonyloxy, bevorzugt Methylcarbonyloxy oder Ethylcarbonyloxy. Im Speziellen ist der Alkylrest unsubstituiert.

Der Begriff "Haloalkylcarbonyloxy" bezeichnet über eine Carbonyloxygruppe gebundene Haloalkylreste mit 1 bis 10 Kohlenstoffatomen. Beispiele hierfür sind Fluormethylcarbonyloxy, Difluormethylcarbonyloxy, Trifluormethylcarbonyloxy, 1-Fluorethylcarbonyloxy, 2-Fluorethylcarbonyloxy, 1,1-Difluorethylcarbonyloxy, 2,2-Difluorethylcarbonyloxy, 2,2,2-Trifluorethylcarbonyloxy, Pentafluorethylcarbonyloxy und dergleichen, bevorzugt sind Fluormethylcarbonyloxy, Difluormethylcarbonyloxy oder Trifluormethylcarbonyloxy.

Der Begriff "Alkenylcarbonyl" bezeichnet über eine Carbonylgruppe gebundene Alkenylreste mit 3 bis 6 Kohlenstoffatomen, wobei der Alkenylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Propen-1-ylcarbonyl, Propen-2-ylcarbonyl (Allylcarbonyl), But-1-en-1-ylcarbonyl, But-1-en-2-ylcarbonyl, But-1-en-3-ylcarbonyl, But-1-en-4-ylcarbonyl, But-2-en-1-ylcarbonyl, But-2-en-2-ylcarbonyl, But-2-en-4-ylcarbonyl, 2-Methylprop-1-en-1-ylcarbonyl, 2-Methylprop-2-en-1-ylcarbonyl und dergleichen, bevorzugt sind Propen-1-ylcarbonyl, Propen-2-ylcarbonyl oder But-1-en-4-ylcarbonyl. Im Speziellen ist der Alkylrest unsubstituiert.

Der Begriff "Alkoxycarbonyl" bezeichnet über eine Carbonylgruppe gebundene Alkoxyreste mit 1 bis 10 Kohlenstoffatomen, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Methoxycarbonyl, Ethoxycarbonyl, Propoxycarbonyl, Isopropoxycarbonyl, n-Butoxycarbonyl, sec-Butoxycarbonyl, Isobutoxycarbonyl und tert-Butoxycarbonyl, bevorzugt sind Methoxycarbonyl, Ethoxycarbonyl, Propoxycarbonyl oder Isopropoxycarbonyl. Im Speziellen ist der Alkylrest unsubstituiert.

Der Begriff "Haloalkoxycarbonyl" bezeichnet über eine Carbonylgruppe gebundene Haloalkoxyreste mit 1 bis 10 Kohlenstoffatomen. Beispiele hierfür sind Fluormethoxycarbonyl, Difluormethoxycarbonyl, Trifluormethoxycarbonyl, 1-Fluorethoxycarbonyl, 2-Fluorethoxycarbonyl, 1,1-Difluorethoxycarbonyl, 2,2-Difluorethoxycarbonyl, 2,2,2-Trifluorethoxycarbonyl, Pentafluorethoxycarbonyl und dergleichen, bevorzugt sind Fluormethoxycarbonyl, Difluormethoxycarbonyl oder Trifluormethoxycarbonyl.

Der Begriff "Alkenyloxycarbonyl" bezeichnet Alkenyloxyreste mit 3 bis 8 Kohlenstoffatomen, die über eine Carbonylgruppe gebunden sind, wobei der Alkenylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Allyloxycarbonyl und Methallyloxycarbonyl, bevorzugt Allyloxycarbonyl. Im Speziellen ist der Alkenylrest unsubstituiert.

Der Begriff "Alkylsulfonyl" bezeichnet über eine Sulfonylgruppe (SO₂) gebundene Alkylreste mit 1 bis 10 Kohlenstoffatomen, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Methylsulfonyl, Ethylsulfonyl, Propylsulfonyl, Isopropylsulfonyl, n-Butylsulfonyl, sec-Butylsulfonyl, Isobutylsulfonyl und tert-Butylsulfonyl, bevorzugt Methylsulfonyl, Ethylsulfonyl, Propylsulfonyl oder Isopropylsulfonyl. Im Speziellen ist der Alkylrest unsubstituiert.

Der Begriff "Haloalkylsulfonyl" bezeichnet über eine Sulfonylgruppe (SO₂) gebundene Haloalkylreste mit 1 bis 10 Kohlenstoffatomen. Beispiele hierfür sind Fluormethylsulfonyl, Difluormethylsulfonyl, Trifluormethylsulfonyl, 1-Fluorethylsulfonyl, 2-Fluorethylsulfonyl, 1,1-Difluorethylsulfonyl, 2,2-Difluorethylsulfonyl, 2,2,2-Trifluorethylsulfonyl, Pentafluorethylsulfonyl und dergleichen, bevorzugt Fluormethylsulfonyl, Difluormethylsulfonyl oder Trifluormethylsulfonyl.

Der Begriff "Aryl", wie hierin und beispielsweise in den Arylalkyleinheiten von Arylalkyl verwendet, bezeichnet carbocyclische aromatische Reste mit 6 bis 14 Kohlenstoffatomen, wobei die Arylgruppe gegebenenfalls 1, 2, 3, 4 oder 5 Substituenten trägt, die ausgewählt sind unter Halogen, Cyano, Nitro, Alkyl, Haloalkyl, Alkoxy, Alkoxycarbonyl oder Haloalkoxy. Beispiele hierfür umfassen Phenyl, 4-Chlorphenyl, 4-Methoxyphenyl, Naphthyl, Fluorenyl, Azulenyl, Anthracenyl und Phenanthrenyl. Bevorzugt steht Aryl für Phenyl oder Naphthyl und insbesondere Phenyl.

Der Begriff "Heteroaryl", wie hierin und beispielsweise in den Heteroarylalkyleinheiten von Heteroarylalkyl verwendet, bezeichnet aromatische Reste mit 1 bis 4 Heteroatomen als Ringgliedern, die ausgewählt sind unter O, N, S und SO₂, wobei die Heteroarylgruppe gegebenenfalls 1, 2, 3 oder 4 Substituenten trägt, die ausgewählt sind unter Halogen, Nitro, Cyano, Alkyl, Haloalkyl, Alkoxy, Alkoxycarbonyl oder Haloalkoxy. Beispiele hierfür sind 5- und 6-gliedrige Heteroarylreste mit 1, 2, 3 oder 4 Heteroatomen ausgewählt unter O, N, S und SO₂ wie Pyrrolyl, 5-Methyl-2-pyrrolyl, Furanyl, 3-Methyl-2-furanyl, Thienyl, Pyrazolyl, Imidazolyl, Oxazolyl, Isoxazolyl, Thiazolyl, Isothiazolyl, Triazolyl, Tetrazolyl, Pyridyl, Pyrazinyl, Pyridazinyl, Pyrimidyl oder Triazinyl.

Der Begriff "Arylcarbonyl" bezeichnet Arylreste, die über eine Carbonylgruppe gebunden sind, wobei die Arylgruppe gegebenenfalls 1, 2, 3, 4 oder 5 Substituenten trägt, die ausgewählt sind unter Halogen, Alkyl, Haloalkyl, Alkoxy oder Haloalkoxy. Beispiele hierfür sind Phenylcarbonyl, 4-Nitrophenylcarbonyl, 2-Methoxyphenylcarbonyl, 4-Chlorphenylcarbonyl, 2,4-Dichlorphenylcarbonyl, 4-Nitrophenylcarbonyl oder Naphthylcarbonyl, bevorzugt Phenylcarbonyl.

Der Begriff "Arylalkyl" bezeichnet Arylreste, die über eine Alkylgruppe gebunden sind, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Arylgruppe gegebenenfalls 1, 2, 3, 4 oder 5 Substituenten trägt, die ausgewählt sind unter Halogen, Alkyl, Haloalkyl, Alkoxy oder Haloalkoxy. Beispiele hierfür sind 4-Methoxybenzyl, Benzyl, 2-Phenylethyl (Phenethyl) und dergleichen, bevorzugt Benzyl und Phenethyl. Im Speziellen ist der Alkylrest unsubstituiert.

Der Begriff "Alkylimino" bezeichnet einen Rest der Formel -N=R, der über den Stickstoff gebunden ist, worin R für Alkylen steht, wie =CH₂, =CHCH₃, =CHCH₂CH₃, =C(CH₃)₂, =CHCH₂CH₂CH₃, =C(CH₃)CH₂CH₃ oder =CHCH(CH₃)₂. Der Alkylenrest von "Alkylimino" trägt gegebenenfalls 1, 2 oder 3 Substituenten, die ausgewählt sind unter Halogen, Alkyl, Haloalkyl, Cycloalkyl, Alkoxy, Haloalkoxy. Im Speziellen ist der Alkylenrest unsubstituiert.

Der Begriff "Arylalkylimino" bezeichnet einen Rest der Formel -N=R, der über den Stickstoff gebunden ist, worin R für Arylalkylen, wie Benzyliden (R = CH-Phenyl) steht. Die Arylgruppe in "Arylalkylimino" kann gegebenenfalls 1, 2, 3, 4 oder 5 Substituenten tragen, die ausgewählt sind unter Halogen, Alkyl, Haloalkyl, Alkoxy oder Haloalkoxy.

Der Begriff "Hydroxyalkyl" bezeichnet einen über eine Alkylgruppe gebundene Hydroxygruppe, wobei die Alkylgruppe gegebenenfalls 1, 2 oder 3 Substituenten trägt, die ausgewählt sind unter Halogen, Alkyl, Haloalkyl, Cycloalkyl, Alkoxy, Haloalkoxy. Beispiele sind -CH₂OH, -(CH₂)₂OH oder -(CH₂)₃OH. Im Speziellen ist der Alkylrest unsubstituiert.

Der Begriff "Alkinyl" bezeichnet einen in Form einer Kohlenstoff-Kohlenstoff-Dreifachbindung zweifach ungesättigten, linearen oder verzweigten aliphatischen Rest mit 3 bis 8 Kohlenstoffatomen. Beispiele hierfür sind Propin-3-yl, But-1-in-1-yl, But-1-in-3-yl, But-1-in-4-yl, But-2-in-1-yl, But-2-in-4-yl und dergleichen, bevorzugt Propin-3-yl oder But-1-in-4-yl.

Der Begriff "Heteroarylalkyl" bezeichnet Heteroarylreste, die über eine Alkylgruppe gebunden sind, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Heteroarylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind 4-Pyridylmethyl, 1-(4-Pyridyl)ethyl, 2-(4-Pyridyl)ethyl, 2-Furanylmethyl, 1-(2-Furanyl)ethyl, 2-(2-Furanyl)ethyl und dergleichen, bevorzugt 4-Pyridylmethyl. Im Speziellen ist der Alkylrest unsubstituiert.

Der Begriff "Alkoxyalkyl" bezeichnet über eine Alkylgruppe gebundene Alkoxyreste mit 1 bis 10 Kohlenstoffatomen, wobei die Alkylreste gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Methoxymethyl, Ethoxymethyl, n-Propoxymethyl, Isopropoxymethyl, n-Butoxymethyl, sec-Butoxymethyl, Isobutoxymethyl, tert-Butoxymethyl, Methoxyethyl, Ethoxyethyl, Propoxyethyl, Isopropoxyethyl, n-Butoxyethyl, sec-Butoxyethyl, Isobutoxyethyl, tert-Butoxyethyl und dergleichen, bevorzugt sind Methoxymethyl, Ethoxymethyl, n-Propoxymethyl, Isopropoxymethyl, Methoxyethyl, Ethoxyethyl, Propoxyethyl oder Isopropoxyethyl. Im Speziellen ist der Alkylrest unsubstituiert.

Der Begriff "Aryloxyalkyl" bezeichnet über eine Alkylgruppe gebundene Aryloxyreste mit 6 bis 14 Kohlenstoffatomen, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Die Arylgruppe in "Aryloxyalkyl" trägt gegebenenfalls 1, 2, 3, 4 oder 5 Substituenten, die ausgewählt sind unter Halogen, Alkyl, Haloalkyl, Alkoxy oder Haloalkoxy. Beispiele hierfür sind Phenoxymethyl, Phenoxyethyl, Phenoxypropyl, Phenoxybutyl, 1-Naphtyloxymethyl, 1-(1-Naphtyloxy)ethyl, 2-(1-Naphtyloxy)ethyl, 1-(1-Naphtyloxy)propyl, 2-(1-Naphtyloxy)propyl, 3-(1-Naphtyloxy)propyl und dergleichen, bevorzugt sind Phenoxymethyl und Phenoxyethyl. Im Speziellen ist der Alkylrest unsubstituiert.

Der Begriff "Heteroaryloxyalkyl" bezeichnet über eine Alkylgruppe gebundene Heteroaryloxyreste mit 1 bis 4 Heteroatomen als Ringgliedern, die ausgewählt sind unter O, N, S und SO₂, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Die Heteroarylgruppe in "Heteroaryloxyalkyl" trägt gegebenenfalls 1, 2, 3 oder 4 Substituenten, die ausgewählt sind unter Halogen, Alkyl, Haloalkyl, Alkoxy oder Haloalkoxy. Beispiele hierfür sind 4-Pyridyloxymethyl, 1-(4-Pyridyloxy)ethyl, 2-(4-Pyridyloxy)ethyl, 1-(4-Pyridyloxy)propyl, 2-(4-Pyridyloxy)propyl, 3-(4-Pyridyloxy)propyl, 2-Furanyloxymethyl, 1-(2-Furanyloxy)ethyl, 2-(2-Furanyloxy)ethyl, 1-(2-Furanyloxy)propyl, 2-(2-Furanyloxy)propyl, 3-(2-Furanyloxy)propyl und dergleichen, bevorzugt sind 4-Pyridyloxymethyl oder 1-(4-Pyridyloxy)ethyl. Im Speziellen ist der Alkylrest unsubstituiert.

Der Begriff "Aminoalkyl" bezeichnet einen über eine Alkylgruppe gebundenen - NH₂-Rest, wobei die Alkylgruppe gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Aminomethyl [-CH₂NH₂], Aminoethyl [-(CH₂)₂NH₂] und dergleichen, bevorzugt sind -CH₂NH₂, -(CH₂)₂NH₂ oder -(CH₂)₃NH₂. Im Speziellen ist der Alkylrest unsubstituiert.

Der Begriff "Alkylaminoalkyl" bezeichnet einen über eine Alkylgruppe gebundenen - NHR⁴ oder -NR⁴R⁵ -Rest, wobei R⁴ und R⁵ wie oben definiert sind und die Alkylgruppe gegebenenfalls 1, 2 oder 3 Substituenten trägt, die ausgewählt sind unter Halogen, Alkyl, Haloalkyl, Cycloalkyl, Alkoxy, Haloalkoxy. Beispiele sind Methylaminomethyl [-CH₂-NH-CH_{3]}, N,N-Dimethylaminomethyl [-CH₂-N(CH₃)₂], N,N-Dimethylaminoethyl [-(CH₂)₂-N(CH₃)₂] und dergleichen, bevorzugt -CH₂-N(CH₃)₂ oder -(CH₂)₂-N(CH₃)₂. Im Speziellen ist der Alkylrest unsubstituiert.

Der Begriff "Cycloalkylcarbonyl" bezeichnet über eine Carbonylgruppe gebundene Cycloalkylreste mit 3 bis 10 Kohlenstoffatomen als Ringglieder, wobei der Cycloalkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Cyclopropylcarbonyl, Cyclobutylcarbonyl, Cyclopentylcarbonyl, Cyclohexylcarbonyl und dergleichen, bevorzugt Cyclopropylcarbonyl, Cyclopentylcarbonyl oder Cylcohexylcarbonyl. Im Speziellen ist der Cycloalkylrest unsubstituiert.

Der Begriff "Arylalkylcarbonyl" bezeichnet über eine Carbonylgruppe gebundene Arylalkylreste, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Arylgruppe gegebenenfalls 1, 2, 3, 4 oder 5 Substituenten trägt, die ausgewählt sind unter Halogen, Alkyl, Haloalkyl, Alkoxy oder Haloalkoxy. Beispiele hierfür sind Benzylcarbonyl, 2-Phenylethylcarbonyl und dergleichen, bevorzugt Benzylcarbonyl oder 2-Phenylethylcarbonyl. Im Speziellen ist der Alkylrest unsubstituiert.

Der Begriff "Heteroarylalkylcarbonyl" bezeichnet über eine Carbonylgruppe gebundene Heteroarylalkylreste mit 1 bis 4 Heteroatomen als Ringgliedern, die ausgewählt sind unter O, N, S und SO₂, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Heteroarylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind 4-Pyridylmethylcarbonyl, 1-(4-Pyridyl)ethylcarbonyl, 2-Furanylmethylcarbonyl, 1-(2-Furanyl)ethylcarbonyl und dergleichen, bevorzugt 4-Pyridylmethylcarbonyl. Im Speziellen ist der Alkylrest unsubstituiert.

Der Begriff "Cycloalkoxycarbonyl" bezeichnet über eine Carbonylgruppe gebundene Cycloalkoxyreste mit 3 bis 10 Kohlenstoffatomen als Ringglieder, wobei der Cycloalkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Cyclopropyloxycarbonyl, Cyclobutyloxycarbonyl, Cyclopentyloxycarbonyl, Cyclohexyloxycarbonyl, Cycloheptyloxycarbonyl, Cyclooctyloxycarbonyl, Cyclononyloxycarbonyl und dergleichen, bevorzugt sind Cyclopropyloxycarbonyl, Cyclopentyloxycarbonyl oder Cyclohexyloxycarbonyl. Im Speziellen ist der Cycloalkylrest unsubstituiert.

Der Begriff "Arylalkoxycarbonyl" bezeichnet über eine Carbonylgruppe gebundene A-rylalkoxyreste mit 6 bis 14 Kohlenstoffatomen, wobei der Alkoxyrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Benzyloxycarbonyl, 2-Phenylethyloxycarbonyl und der gleichen, bevorzugt ist Benzyloxycarbonyl. Im Speziellen ist der Alkoxylrest unsubstituiert.

Der Begriff "Aryloxy" bezeichnet einen Arylrest, der über ein Sauerstoffatom gebunden ist, wobei die Arylgruppe gegebenenfalls 1, 2, 3, 4 oder 5 Substituenten trägt, die ausgewählt sind unter Halogen, Alkyl, Haloalkyl, Alkoxy oder Haloalkoxy. Beispiele hierfür sind Phenyloxy (Phenoxy), Naphtyloxy, Fluorenyloxy und der gleichen, bevorzugt ist Phenoxy.

Der Begriff "Aryloxycarbonyl" bezeichnet über eine Carbonylgruppe gebundene Aryloxyreste mit 6 bis 14 Kohlenstoffatomen, wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Phenyloxycarbonyl (Phenoxycarbonyl), Naphtyloxycarbonyl, Fluorenyloxycarbonyl und dergleichen, bevorzugt ist Phenoxycarbonyl.

Der Begriff "Heteroaryloxy" bezeichnet über ein Sauerstoffatom gebundene Heteroarylreste mit 1 bis 4 Heteroatomen als Ringgliedern, die ausgewählt sind unter O, N, S und SO₂, wobei die Heteroarylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Pyrrolyloxy, Furanyloxy, Thienyloxy, Pyrazolyloxy, Imidazolyloxy, Oxazolyloxy, Thiazolyloxy, Pyridyloxy, Pyrazinyloxy, Pyridazinyloxy, Pyrimidyloxy und dergleichen, bevorzugt Pyrazolyloxy oder Pyridyloxy.

Der Begriff "Heteroaryloxycarbonyl" bezeichnet über eine Carbonylgruppe gebundene Heteroaryloxyreste mit 1 bis 4 Heteroatomen als Ringgliedern, die ausgewählt sind unter O, N, S und SO₂, wobei die Heteroarylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Pyrrolyloxycarbonyl, Furanyloxycarbonyl, Thienyloxycarbonyl, Pyrazolyloxycarbonyl, Imidazolyloxycarbonyl, Oxazolyloxycarbonyl, Thiazolyloxycarbonyl, Pyridyloxycarbonyl, Pyrazinyloxycarbonyl, Pyridazinyloxycarbonyl, Pyrimidyloxycarbonyl und der gleichen, bevorzugt Imidazolyloxycarbonyl oder Oxazolyloxycarbonyl.

Der Begriff "Arylalkyloxy" bezeichnet über ein Sauerstoffatom gebundene Arylalkylreste, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Benzyloxy, 2-Phenylethyloxy (Phenethyloxy) und dergleichen, bevorzugt ist Benzyloxy. Im Speziellen ist der Alkylrest unsubstituiert.

Der Begriff "Cycloalkylimino" bezeichnet einen Rest der Formel -N=R, der über den Stickstoff gebunden ist, worin R für Cycloalkylidenreste steht, die gegebenenfalls 1, 2 oder 3 Substituenten tragen, die ausgewählt sind unter Halogen, Alkyl, Haloalkyl, Cycloalkyl, Alkoxy, Haloalkoxy. Beispiele für R sind Cyclopentyliden, Cyclohexyliden, Cycloheptyliden und dergleichen. Im Speziellen ist der Cycloalkylidenrest unsubstituiert.

Der Begriff "Haloalkylimino" bezeichnet einen Rest der Formel -N=R, der über den Stickstoff gebunden ist, worin R für Haloalkylidenreste steht, wobei die Wasserstoffatome dieser geradkettige oder verzweigte Alkylengruppen teilweise oder vollständig durch Halogenatome ersetzt sind. Beispiele für R sind Chlormethylen, Brommethylen, Dichlormethylen, Fluormethylen, Difluormethylen, Chlorofluormethylen, 1-Chlorethylen, 1-Bromethylen, 1-Fluorethylen, 2-Fluorethylen, 2,2-Difluorethylen, 2,2,2-Trifluorethylen, 2-Chlor-2-fluorethylen, 2-Chlor-2,2-difluorethylen, 2,2-Dichlor-2-fluorethylen, 2,2,2-Trichlorethylen und dergleichen.

Der Begriff "Cycloalkylcarbonyloxy" bezeichnet über eine Carbonyloxygruppe gebundene Cycloalkylreste mit 3 bis 10 Kohlenstoffatomen als Ringglieder, wobei der Cycloalkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Cyclopropylcarbonyloxy, Cyclobutylcarbonyloxy, Cyclopentylcarbonyloxy, Cyclohexylcarbonyloxy, Cycloheptylcarbonyloxy, Cyclooctylcarbonyloxy, Cyclononylcarbonyloxy und dergleichen, bevorzugt Cyclopentylcarbonyloxy oder Cyclohexylcarbonyloxy. Im Speziellen ist der Cycloalkylrest unsubstituiert.

Der Begriff "Arylalkylcarbonyloxy" bezeichnet über eine Carbonyloxygruppe gebundene Arylalkylreste, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Benzylcarbonyloxy, 2-Phenylethylcarbonyloxy (Phenethylcarbonyloxy) und dergleichen, bevorzugt Benzylcarbonyloxy. Im Speziellen ist der Alkylrest unsubstituiert.

Der Begriff "Arylcarbonyloxy" bezeichnet über eine Carbonyloxygruppe gebundene Arylreste, wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Phenylcarbonyloxy, Naphthylcarbonyloxy, Fluorenylcarbonyloxy, Anthracenylcarbonyloxy und dergleichen, bevorzugt Phenylcarbonyloxy.

Der Begriff "Heteroarylcarbonyloxy" bezeichnet über eine Carbonyloxygruppe gebundene Heteroarylreste mit 1 bis 4 Heteroatomen als Ringliedern, die ausgewählt sind unter O, N, S und SO₂, wobei die Heteroarylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind 2-Pyrrolylcarbonyloxy, 2-Furanylcarbonyloxy, 2-Thienylcarbonyloxy, 3-Pyrazolylcarbonyloxy, 2-lmidazolylcarbonyloxy, 2-Oxazolylcarbonyloxy, 2-Thiazolylcarbonyloxy, 4-Triazolylcarbonyloxy, 4-Pyridylcarbonyloxy und dergleichen.

Der Begriff "Heteroarylcarbonyl" bezeichnet über eine Carbonylgruppe gebundene Heteroarylreste mit 1 bis 4 Heteroatomen als Ringgliedern, die ausgewählt sind unter O, N, S und SO₂, wobei die Heteroarylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind 2-Pyrrolylcarbonyl, 2-Furanylcarbonyl, 2-Thienylcarbonyl, 3-Pyrazolylcarbonyl, 2-Imidazolylcarbonyl, 2-Oxazolylcarbonyl, 2-Thiazolylcarbonyl, 4-Triazolylcarbonyl, 4-Pyridylcarbonyl und dergleichen.

Der Begriff "Alkylthio" bezeichnet Alkylreste, die über ein Schwefelatom gebunden sind, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Methylthio, Ethylthio, n-Propylthio, 1-Methylethylthio (Isopropylthio), n-Butylthio, 1-Methylpropylthio (sec-Butylthio), 2-Methylpropylthio (Isobutylthio) und 1,1-Dimethylethylthio (tert-Butylthio) und der gleichen, bevorzugt Methylthio, Ethylthio oder n-Propylthio. Im Speziellen ist der Alkylrest unsubstituiert.

Der Begriff "Haloalkylthio" beschreibt Haloalkylgruppen, die über ein Schwefelatom gebunden sind. Beispiele hierfür sind Chlormethylthio, Brommethylthio, Dichlormethylthio, Trichlormethylthio, Fluormethylthio, Difluormethylthio, Trifluormethylthio, Chlorofluormethylthio, Dichlorfluormethylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio, 1,1,2,2-Tetrafluorethylthio, 1-Chlor-1,2,2-trifluorethylthio, Pentafluorethylthio, 3,3,3-Trifluorprop-1-ylthio, 1,1,1-Trifluorprop-2-ylthio, 3,3,3-Trichlorprop-1-ylthio, 1-Chlorbutylthio, 2-Chlorbutylthio, 3-Chlorbutylthio, 4-Chlorbutylthio, 1-Fluorbutylthio, 2-Fluorbutylthio, 3-Fluorbutylthio, 4-Fluorbutylthio und dergleichen, bevorzugt sind Fluormethylthio, 2-Fluorethylthio oder Trifluormethylthio.

Der Begriff "Cycloalkylthio" bezeichnet Cycloalkylreste, die über ein Schwefelatom gebunden sind. Beispiele sind Cyclopropylthio, Cyclobutylthio, Cyclopentylthio, Cyclohexylthio, Cycloheptylthio, Cyclooctylthio, Cyclononylthio und Cyclodecylthio. Die Cycloalkylreste können 1, 2 oder 3 Substituenten tragen, die ausgewählt sind unter Alkyl und Halogen. Bevorzugte Cycloalkylthioreste sind Cyclopentylthio oder Cyclohexylthio. Im Speziellen ist der Cycloalkylrest unsubstituiert.

Der Begriff "Arylthio" bezeichnet Arylreste, die über ein Schwefelatom gebunden sind, wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Phenylthio, Naphtylthio, Fluorenylthio und der gleichen, bevorzugt Phenylthio.

Der Begriff "Heteroarylthio" bezeichnet über ein Schwefelatom gebundene Heteroarylreste mit 1 bis 4 Heteroatomen als Ringgliedern, die ausgewählt sind unter O, N, S und SO₂, wobei die Heteroarylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind 2-Pyrrolylthio, 3-Furanylthio, 3-Thienylthio, 2-Pyridylthio und dergleichen, bevorzugt 2-Pyridylthio oder 4-Pyridylthio.

Der Begriff "Arylalkylthio" bezeichnet über ein Schwefelatom gebundene Arylalkylreste, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Benzylthio, 2-Phenylethylthio und dergleichen, bevorzugt Benzylthio. Im Speziellen ist der Alkylrest unsubstituiert.

Der Begriff "Cycloalkylsulfonyl" bezeichnet über eine Sulfonylgruppe (SO₂) gebundene Cycloalkylreste mit 3 bis 10 Kohlenstoffatomen als Ringglieder, wobei der Cycloalkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Cyclopropylsulfonyl, Cyclobutylsulfonyl, Cyclopentylsulfonyl, Cyclohexylsulfonyl und dergleichen, bevorzugt Cyclopropylsulfonyl, Cyclopentylsulfonyl oder Cyclohexylsulfonyl. Im Speziellen ist der Cycloalkylrest unsubstituiert.

Der Begriff "Arylsulfonyl" bezeichnet über eine Sulfonylgruppe (SO₂) gebundene Arylreste, wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Phenylsulfonyl, Naphtylsulfonyl, Fluorenylsulfonyl und dergleichen, bevorzugt Phenylsulfonyl.

Der Begriff "Heteroarylsulfonyl" bezeichnet über eine Sulfonylgruppe (SO₂) gebundene Heteroarylreste mit 1 bis 4 Heteroatomen als Ringgliedern, die ausgewählt sind unter O, N, S und SO₂, wobei die Heteroarylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind 2-Pyrrolylsulfonyl, 2-Furanylsulfonyl, 2-Thienylsulfonyl, 3-Pyrazolylsulfonyl, 2-Imidazolylsulfonyl, 2-Oxazolylsulfonyl, 4-Pyridylsulfonyl und dergleichen, 2-Pyrrolylsulfonyl, 2-Furanylsulfonyl oder 4-Pyridylsulfonyl.

Der Begriff "Arylalkylsulfonyl" bezeichnet über eine Sulfonylgruppe (SO₂) gebundene Arylalkylreste, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy, und wobei die Arylgruppe gegebenenfalls 1, 2, 3 oder 4 Substituenten trägt, die ausgewählt sind unter Halogen, Alkyl, Haloalkyl, Alkoxy oder Haloalkoxy. Beispiele hierfür sind Benzylsulfonyl, 2-Phenylethylsulfonyl und dergleichen, bevorzugt Benzylsulfonyl. Im Speziellen ist der Alkylrest unsubstituiert.

Die nachfolgend gemachten Ausführungen zu bevorzugten Ausgestaltungen der erfindungsgemäßen Verfahren, insbesondere zu bevorzugten Ausgestaltungen der Reste der verschiedenen Edukte und Produkte und der Reaktionsbedingungen der erfindungsgemäßen Verfahren, gelten sowohl allein für sich genommen als auch insbesondere in jeder denkbaren Kombination miteinander.

Die hierin beschriebenen Umsetzungen werden in für derartige Reaktionen üblichen Reaktionsgefäßen durchgeführt, wobei die Reaktionsführung sowohl kontinuierlich, semi-kontinuierlich als auch diskontinuierlich ausgestaltet werden kann. In der Regel wird man die jeweiligen Reaktionen unter Atmosphärendruck durchführen. Die Reaktionen können jedoch auch unter vermindertem (z.B. 0.1 bis 1.0 bar) oder erhöhtem Druck (z. B. 1.0 bis 100 bar) durchgeführt werden.

Insbesondere ist es bevorzugt die Ausführungsformen in jeder beliebigen Kombination miteinander zu kombinieren.

Im Rahmen der vorliegenden Erfindung steht m bevorzugt für 0, 1, 2, 3 oder 4, insbesondere für 0, 1, 2 oder 3, besonders bevorzugt für 1, 2 oder 3.

Im Rahmen der vorliegenden Erfindung steht n bevorzugt für 1, 2, 3, 4 oder 5 insbesondere für 1, 2 oder 3.

Im Rahmen der vorliegenden Erfindung steht q bevorzugt für 1, 2, 3 oder 4, insbesondere für 1, 2 oder 3.

Im Rahmen der vorliegenden Erfindung steht R¹ bevorzugt für Halogen, Alkyl, Haloalkyl, Alkoxy, Haloalkoxy, Nitro, Cyano, Aryl, Aryloxy oder Heteroaryl. Besonders bevorzugt steht R¹ für Halogen, Alkoxy, Haloalkoxy, Cyano oder gegebenenfalls mit Halogen, Alkyl oder Alkoxy substituiertes Aryloxy und stärker bevorzugt für Chlor, Brom, Fluor, Cyano, Alkoxy oder Phenoxy oder alternativ stärker bevorzugt für Halogen, C₁-C₄-Alkyl oder C₁-C₄-Alkoxy (besonders bevorzugt für Chlor, Brom, Fluor, Methyl oder Methoxy und speziell bevorzugt für Chlor, Brom, Fluor oder Methoxy). Insbesondere steht R¹ für 2-Me, 3-Me, 4-Me, 2-F, 3-F, 4-F, 2-Cl, 3-Cl, 4-Cl, 2-Br, 3-Br, 4-Br, 4-CN, 2-Methoxy, 3-Methoxy, 4-Methoxy, 2-CF₃, 3-CF₃, 4-CF₄, 2-OCF₃, 3-OCF₃, oder 4-OCF₃. Die Positionsangaben beziehen sich dabei auf die 1-Position, über die der sich von der Verbindung der Formel **1** ableitende Arylrest an den Anilinring der Verbindung der Formel **3** gebunden ist bzw. auf die 1-Position des Hydrazinrestes in der Verbindung der Formel **1.**

Im Rahmen der vorliegenden Erfindung steht Y- bevorzugt für ein Halogenid, wie Fluorid, Chlorid, Bromid, lodid, BF₄⁻, PF₆⁻, Hydrogensulfat, Sulfat (½ SO₄²⁻), Acetat, das Anion eines aromatischen 1,2-Dicarbonsäureimids oder das Anion eines aromatischen 1,2-Disulfonimids. Das Anion entsteht in den beiden zuletzt genannten Fällen durch Abstraktion des Protons am Imid-Stickstoffatom. Besonders bevorzugt steht Y- für ein Halogenid, wie Chlorid oder Bromid, BF₄⁻ oder Sulfat (½ SO₄²⁻).

Im Rahmen der vorliegenden Erfindung steht R² für Wasserstoff.

Im Rahmen der vorliegenden Erfindung steht R³ für Wasserstoff.

Im Rahmen der vorliegenden Erfindung steht R⁴ bevorzugt für Wasserstoff, Alkyl, Haloalkyl, Cycloalkyl, Arylalkyl, Aryl oder Heteroaryl.

Im Rahmen der vorliegenden Erfindung steht R⁵ bevorzugt für Wasserstoff, Alkyl, Haloalkyl, Cycloalkyl, Arylalkyl, Aryl oder Heteroaryl.

Im Rahmen der vorliegenden Erfindung steht R¹⁰ bevorzugt für Wasserstoff, Halogen, Alkyl, Haloalkyl, Cycloalkyl, Aryl oder Heteroaryl. Besonders bevorzugt steht R¹⁰ für Wasserstoff, Halogen, C₁-C₆ Alkyl oder C₁-C₆ Haloalkyl. Insbesondere bevorzugt steht R¹⁰ für Wasserstoff.

Im Rahmen der vorliegenden Erfindung steht R¹¹ bevorzugt für Wasserstoff, Halogen, Hydroxy, Cyano, Aryl oder Heteroaryl.

Im Rahmen der vorliegenden Erfindung steht R¹⁴ bevorzugt für Wasserstoff, Alkyl oder Haloalkyl.

Im Rahmen der vorliegenden Erfindung steht R¹⁵ bevorzugt für Wasserstoff, Alkyl, Haloalkyl, Cyano, Aryl oder Heteroaryl.

Im Rahmen der vorliegenden Erfindung steht R⁶ bevorzugt für Wasserstoff, Halogen, Alkyl, Haloalkyl, Cycloalkyl, Alkoxy, Cyano, Nitro, Haloalkoxy, Cycloalkoxy, Alkylcarbonyloxy, -COOR4, -COR⁴, -CONR⁴R⁵, -NR⁴COR⁵, -NR⁴COOR⁵, Haloalkylcarbonyloxy, Aryloxy, Aryl oder Heteroaryl. Stärker bevorzugt steht R⁶ für Wasserstoff, Halogen, CN, C₁-C₄-Alkyl oder C₁-C₄-Alkoxy. Für R⁶ besonders bevorzugt sind: Wasserstoff, Fluor, Chlor, Brom, Cyano, Methoxy, Ethoxy, Methyl oder Ethyl, und insbesondere Wasserstoff, Fluor, Chlor, Brom, Cyano, Methoxy, Ethoxy, Methyl. Insbesondere bevorzugt ist R⁶ Wasserstoff, Fluor, Chlor, Brom, CN, Methoxy oder Ethoxy und speziell Wasserstoff, Fluor, Chlor, Brom oder Cyano.

Vorzugsweise sind Verfahrensmaßnahmen ausgewählt unter folgenden:
- Durchführung in wenigstens einem Lösungsmittel bzw. Lösungsmittelsystem;
- Durchführung in wenigstens einem Lösungsmittel bzw. Lösungsmittelsystem und in Gegenwart wenigstens einer Base, wenn die Verbindung der Formel 1 als Salz oder Säureaddukt eingesetzt wird;
- Durchführung in Gegenwart wenigstens eines Oxidationsmittels (erfindungsgemäß zwingende Maßnahme);
- und einer Kombination von wenigstens zwei dieser Maßnahmen.

Beschrieben wird außerdem die Durchführung unter den Bedingungen einer elektrochemischen Oxidation.

Der Begriff "Oxidationsmittel" bezeichnet diejenigen Elemente und Verbindungen, die als Elektronenakzeptoren [auch Elektronen-Akzeptor-Komplexe] bestrebt sind, durch die Aufnahme von Elektronen in einen energieärmeren Zustand überzugehen, v. a. unter Bildung stabiler Elektronenschalen. Ein Maß für die Stärke eines Oxidationsmittels ist das Redoxpotential.

Beispiele für Oxidationsmittel sind die Elemente Schwefel, Sauerstoff, Fluor, Chlor, Brom und Iod sowie anorganische Verbindungen wie Sauerstoffdifluorid. Weitere Beispiele sind Wasserstoffperoxid und entsprechende Addukte wie Natriumpercarbonat und Natriumperborat. Als Oxidationsmittel sind weiterhin sauerstoffhaltige Anionen (Oxoanionen) von Übergangsmetallen in hohen Oxidationsstufen wie Permangant (MnO₄⁻), Dichromat (Cr₂O₇²⁻) und Metalloxide wie Chrom(VI)-oxid (CrO₃) gebräuchlich. Desweiteren kommen Metallionen wie Mangan(III) und Cer(IV) oder Edelmetallionen wie Silber(I) und Kupfer(II) zum Einsatz. Starke Oxidationsmittel sind ebenfalls die Anionen von Halogensauerstoffsäuren wie Bromat (BrO₃⁻), Chlorat (CIO₃⁻ ) oder Hypochlorit (CIO⁻).

Die Reaktion wird in Gegenwart eines Oxidationsmittels durchgeführt und erfolgt vorzugsweise so, dass die Verbindung der Formel 2 und das Oxidationsmittel, vorzugsweise in einem Lösungsmittel oder Lösungsmittelsystem gelöst oder dispergiert, vorgelegt und mit der Verbindung der Formel 1 sukzessive versetzt werden. Bezüglich Zugabegeschwindigkeit, Reaktionstemperatur und Lösungsmittel oder Lösungsmittelsystem wird auf die folgenden Ausführungen verwiesen.

Bevorzugte Oxidationsmittel sind im Reaktionsmedium wenigstens teilweise löslich. Die Löslichkeit der Oxidationsmittel ist aber keine Bedingung für die Durchführung, da die Oxidation der Verbindung der Formel **1** auch an der Oberfläche des ungelösten Oxidationsmittels ablaufen kann.

Die Oxidationsmittel sind ausgewählt unter Sauerstoff, Luftsauerstoff, MnO₂, Mn(OAc)₃, KMnO₄, NalO₄, K₃[Fe(CN)₆], KO₂ und H₂O₂; speziell MnO₂.

Vorzugsweise setzt man das/die oxidierenden Metall(e) oder Metallsalz(e) in einer Gesamtmenge von 0,01 bis 20 Mol, besonders bevorzugt von 0,05 bis 8 Mol, stärker bevorzugt von 0,1 bis 5 Mol bezogen auf **1** Mol der Verbindung der Formel 1 ein.

Wird die Reaktion in entgasten (d.h. von Sauerstoff befreiten) Lösungsmitteln oder Lösungsmittelsystemen und unter einer Inertgasatmosphäre, wie Stickstoff oder Argon, durchgeführt, so muss das oxidierende Metallsalz in größeren Mengen eingesetzt werden, beispielsweise in einer Menge von 0,2 bis 10 Mol bezogen auf 1 Mol der Verbindung der Formel **1.**

In einer Ausführungsform der Erfindung wird die Umsetzung in Gegenwart mindestens eines (von Sauerstoff oder Luft verschiedenen) Oxidationsmittels unter Schutzgas, wie Stickstoff oder Argon, durchgeführt.

Wird die Reaktion dagegen unter Luftsauerstoff oder unter reiner Sauerstoffatmosphäre durchgeführt, so kann auf den Zusatz eines weiteren Oxidationsmittels verzichtet werden, da Sauerstoff, wie z.B. auch in Luftsauerstoff enthalten, im Rahmen der Erfindung als Oxidationsmittel betrachtet wird.

Zusätzlich beschrieben wird die Durchführung des Verfahrens unter den Bedingungen einer elektrochemischen Oxidation (nicht erfindungsgemäß). Bei dieser Vorgehensweise werden aus der Verbindung der Formel 1 durch anodische Oxidation Arylradikale generiert, was den Zerfall der oben genannten Verbindungen initiiert.

Die oben genannten Oxidationsmittel sowie die Bedingungen der elektrochemischen Oxidation führen zur Umwandlung der Verbindung der Formel **1** in ein instabiles Aryldiazen, welches sich unter Bildung eines Arylradikals zersetzt. Untersuchungen zur Stabilität von Aryldiazenen und deren Zersetzung unter Bildung von Arylradikalen findet man in der Literatur [P. C. Huang, E. M. Kosower, J. Am. Chem. Soc. 1967, 89, 3910-3911.]

Die Durchführung der elektrochemischen Oxidation erfolgt beispielsweise so, dass in das Reaktionsgefäß, welches die in einem geeigneten Lösungsmittel oder Lösungsmittelsystem vorgelegte Verbindung der Formel **2** enthält, Kathode und Anode angeordnet werden und während der sukzessiven Zugabe der Verbindung der Formel **1** Spannung angelegt wird. Die zu wählende Spannung und Stromdichte hängt von diversen Faktoren, wie Zugabegeschwindigkeit und Lösungsmittel bzw. Lösungsmittelsystem ab und muss im Einzelfall bestimmt werden, was beispielsweise mit Hilfe von Vorversuchen gelingt. Die Lösungsmittel oder Lösungsmittelsysteme werden geeigneterweise so gewählt, dass sie unter den gegebenen Reaktionsbedingungen möglichst keine Konkurrenzreaktion an den Elektroden eingehen. Da die anodische Oxidation von Wasser unter Bildung von Sauerstoff auch bei sehr geringen Stromdichten und Spannungen nur schwer zu vermeiden ist, werden bevorzugt nichtprotische, polare Lösungsmittel, wie Acetonitril, Dimethylformamid oder Aceton, eingesetzt.

Erfindungsgemäß erfolgt die Durchführung in Gegenwart von wenigstens einem der oben genannten Oxidationsmittel.

Zusätzlich kann das Verfahren zur Herstellung von Verbindungen der Struktur **3** dadurch erfolgen, dass die Reaktion unter Bestrahlung mit elektromagnetischer Strahlung im sichtbaren und/oder ultravioletten Bereich erfolgt. Bevorzugt wird elektromagnetische Strahlung mit einer Wellenlänge im Bereich von 100 bis 400 nm, besonders bevorzugt im Bereich von 200 bis 380 nm und insbesondere im Bereich von 250 bis 360 nm eingesetzt.

Die Durchführung unter Bestrahlung erfolgt bevorzugt in der Weise, dass die Verbindung der Formel **2** in einem geeigneten Lösungsmittel oder Lösungsmittelsystem vorgelegt wird und während der sukzessiven Zugabe der Verbindung der Formel **1** bestrahlt wird.

Zusätzlich kann das Verfahren zur Herstellung von Verbindungen der Formel **3** dadurch erfolgen, dass die Reaktion unter Anwendung von Ultraschall durchgeführt wird. Wie alle Schallwellen erzeugt auch Ultraschall eine periodische Kompression und Dehnung des Mediums; die Moleküle werden zusammengedrückt und gedehnt. Es bilden sich kleine Bläschen, die anwachsen und sofort wieder implodieren. Dieses Phänomen wird Kavitation genannt. Jedes implodierende Bläschen sendet Schockwellen und winzige Flüssigkeitsstrahlen mit einer Geschwindigkeit von etwa 400 km/h aus, die auf die nähere Umgebung einwirken. Kavitation kann beispielsweise ausgenutzt werden, um chemische Reaktionen zu beschleunigen und die Löslichkeit von Produkten in einem bestimmten Medium zu erhöhen.

Die Durchführung unter Anwendung von Ultraschall kann beispielsweise so erfolgen, dass sich das Reaktionsgefäß, in welchem die Verbindung der Formel **2** in einem geeigneten Lösungsmittel oder Lösungsmittelsystem vorgelegt ist, in einem Ultraschallbad befindet und das Reaktionsgemisch während der sukzessiven Zugabe der Verbindung der Formel 1 Ultraschall ausgesetzt wird. Anstelle der Verwendung eines Ultraschallbads kann in das Reaktionsgefäß, in welchem die Verbindung der Formel **2** in einem geeigneten Lösungsmittel oder Lösungsmittelsystem vorgelegt ist, eine Sonotrode (= Vorrichtung, welche die von einem Schallwandler erzeugten Ultraschallschwingungen an das zu beschallende Material weiterleitet) angebracht werden. Letztere Alternative bietet sich insbesondere für größere Ansätze an. Bezüglich Zugabegeschwindigkeit, Reaktionstemperatur und Lösungsmittel bzw. Lösungsmittelsystem müssen Vorversuche durchgeführt werden.

Die Anwendung von elektromagnetischer Strahlung oder von Ultraschall ist vor allem dann bevorzugt, wenn die Oxidation der Verbindung der Formel **1** durch das eingesetzte Oxidationsmittel nur langsam erfolgt. Eine schnellere Oxidation der Verbindung der Formel **1,** wie sie dann durch elektromagnetische Strahlung oder Ultraschall erreicht werden kann, ist für den Reaktionsverlauf grundsätzlich günstig. Wird die Verbindung der Formel 1 nach Zugabe zur Reaktionsmischung nur langsam oxidiert, so entstehen bevorzugt Homokupplungsprodukte. Unter Homokupplungsprodukten versteht man Verbindungen, die aus der Addition der aus der Verbindung der Formel **1** hervorgehenden Arylradikale an unumgesetzte Verbindungen der Formel **1** entstehen. Erwünscht ist dagegen die Addition der aus der Verbindung der Formel **1** hervorgehenden Arylradikale an Verbindungen der Formel **2.**

Die Oxidation der Verbindung der Formel **1** unter Bildung von Arylradikalen kann beispielsweise langsam verlaufen, wenn Luftsauerstoff als Oxidationsmittel verwendet wird. Desweiteren verläuft die Oxidation der Verbindung der Formel **1** langsam, wenn die Verbindung der Formel **1** in der Form eines Salzes, d.h. eines Säureadduktes, ohne zusätzliche Base eingesetzt wird. In diesen Fällen kann sich der Einsatz von Ultraschall oder elektromagnetischer Strahlung als vorteilhaft erweisen.

Der Begriff "Lösungsmittel" (Solvens, Lösemittel) bezeichnet im weitesten Sinne Stoffe, die andere auf physikalischem Wege ganz oder teilweise zur Lösung bringen können, im engeren Sinne anorganische und organische Flüssigkeiten, die andere gasförmige, flüssige oder feste Stoffe zu lösen vermögen. Voraussetzung für die Eignung als Lösungsmittel ist, dass sich beim Lösungsvorgang weder der lösende noch der gelöste Stoff chemisch verändern, dass also die Komponenten der Lösung durch physikalische Trennverfahren wie Destillation, Kristallisation, Sublimation, Verdunstung, Absorption in der Originalgestalt wiedergewonnen werden können.

Man kennt anorganische und organische Lösungsmittel, die hier gruppenweise vorgestellt werden.

Bei *anorganischen Lösungsmitteln* unterscheidet man Protonen-haltige (protische) wie z.B. H₂O, flüssiges NH₃, H₂S, HF, HCN, HNO₃, und Protonen-(Wasserstoff-)freie (aprotische) Lösungsmittel, wie z.B. flüssiges SO₂, N₂O₄, NOCl, SeOCl₂, ICl, BrF₃, AsCl₃, zum anderen wässrige und nichtwässrige Lösungsmittel (alle außer H₂O, mit den Untergruppen aprotische, amphiprotische, protogene, protophile Lösungsmittel).

Die letzerwähnten Einteilungen lassen sich natürlich erst recht auf die organischen Lösungsmittel anwenden, von denen hier nur die wichtigsten aufgeführt sein können: Alkohole (z.B. Methanol, Ethanol usw.) Glykole (z. B. Ethylenglykol), Ether und Glykolether (z. B. Diethylether, Dioxan, Tetrahydrofuran), Ketone (z.B. Aceton), Amide, Nitrile und andere Stickstoff-Verbindungen (z.B. Dimethylformamid, Acetonitril), Schwefelverbindungen (z. B. Schwefelkohlenstoff, Sulfolan), Nitroverbindungen (z. B. Nitromethan), Halogenkohlenwasserstoffe (z. B. Dichlormethan, Chloroform), Kohlenwasserstoffe (z. B. Benzine, Petrolether, Cyclohexan).

Geeignete Lösungsmittel oder Lösungsmittelsysteme hängen im Einzelnen von der Wahl der jeweiligen Reaktionsbedingungen für die Zersetzung der Verbindung der Formel **1,** wie beispielsweise den Reaktionspartnern, ab. Es hat sich jedoch allgemein als vorteilhaft erwiesen, als Lösungsmittel oder Lösungsmittelsysteme für die Umsetzung der Verbindung der Formel **1** und der Verbindung der Formel **2** ein Lösungsmittel oder Lösungsmittelsystem zu verwenden, in dem die Verbindung der Formel **2** eine gute Löslichkeit besitzt. Unter guter Löslichkeit wird verstanden, dass die Verbindung der Formel **2** idealerweise vollständig gelöst vorliegt, mindestens sollte jedoch eine Menge der Verbindung der Formel **2** gelöst vorliegen, die 0,1 Mol bezogen auf 1 Mol der eingesetzten Verbindung 1 entspricht.

Im Rahmen der vorliegenden Erfindung ist es nicht erforderlich, dass die Verbindungen der Formeln **1** und **2** vollständig im gewählten Lösungsmittel oder Lösungsmittelsystem löslich sind. Es hat sich jedoch als vorteilhaft erwiesen, wenn sowohl die Verbindung der Formel **1** als auch die Verbindung der Formel **2** im gewählten Lösungsmittel oder Lösungsmittelsystem vollständig löslich sind.

Im Rahmen der vorliegenden Erfindung sind bevorzugte organische Lösungsmittel beispielsweise kurzkettige Nitrile, wie Acetonitril oder Propionitril, Amide, wie Formamid und Dimethylformamid, kurzkettige ein- oder mehrwertige Alkohole, wie Methanol, Ethanol, Propanol oder Trifluorethanol, kurzkettige Carbonsäuren, wie Eisessig, Trifluoressigsäure, kurzkettige Ketone, wie Aceton, und Dimethylsulfoxid oder Mischungen dieser organischen Lösungsmittel untereinander. Unter diesen ist Acetonitril bevorzugt.

Ist die Löslichkeit des Oxidationsmittels im gewählten Lösungsmittel oder Lösungsmittelsystem zu gering, und erfolgt die Oxidation der Verbindung der Formel **1** deswegen nur langsam, so kann die Löslichkeit des Oxidationsmittels durch den Zusatz von Wasser gegebenenfalls erhöht werden.

Geeignet sind besonders solche Lösungsmittel bzw. Lösungsmittelsysteme, die keine leicht abstrahierbaren Wasserstoffatome besitzen, da sie ein gebildetes Arylradikal bestmöglich vor Nebenreaktionen schützen.

Beispiele für besonders geeignete Lösungsmittel bzw. Lösungsmittelsysteme sind vergleichsweise inerte organische Lösungsmittel bzw. Lösungsmittelsysteme, wie Acetonitril, Aceton, Trifluorethanol und/oder Dimethylsulfoxid, aber auch Alkohole ohne Wasserstoffatome in der α-Position, wie tert-Butanol.

Ein Zusatz von Wasser wirkt allgemein stabilisierend auf die entstehenden Arylradikale, da diese mit Wasser praktisch keine Nebenreaktionen eingehen. Jedoch sollte der Reaktionsverlauf durch Wasser nicht in dem Sinne negativ beeinflusst werden, dass die Löslichkeit der eingesetzten Verbindungen der Formel **1,** der Formel **2** und des Oxidationsmittels abnimmt.

Wenn Lösungsmittel mit leicht abstrahierbaren Wasserstoffatomen, wie primäre Alkohole, eingesetzt werden, so werden sie vorzugsweise im Gemisch mit mindestens einem weiteren Lösungsmittel verwendet, das keine leicht abstrahierbaren Wasserstoffatome besitzt. Vorzugsweise werden die dem Arylradikal gegenüber nicht inerten organischen Lösungsmittel bzw. Lösungsmittelsysteme dabei in einer Menge von höchstens 50 Gew.-%, besonders bevorzugt von höchstens 20 Gew.-% und insbesondere von höchstens 10 Gew.-%, bezogen auf das Gesamtgewicht des Lösungsmittelsystems eingesetzt.

In einer bevorzugten Ausführungsform wird die Verbindung der Formel **2** zusammen mit einem Oxidationsmittel in einem Lösungsmittel oder Lösungsmittelsystem vorgelegt und langsam mit der Verbindung der Formel **1,** gegebenenfalls gelöst in einem Lösungsmittel oder Lösungsmittelsystem, versetzt.

Alternativ, und ebenfalls bevorzugt, kann die Verbindung der Formel **1** als Salz bzw. als Säureaddukt eingesetzt werden. Mögliche Gegenionen der protonierten Form der Verbindung der Formel **1** sind Chlorid, Bromid, lodid, Hydrogensulfat, Sulfat, Tetrafluoroborat, Nitrat, Acetat, Trifluoracetat und ähnliche. In diesem Fall hat es sich als vorteilhaft erwiesen, der vorgelegten Mischung aus Verbindung der Formel **2** und dem Oxidationsmittel in einem Lösungsmittel oder Lösungsmittelsystem eine Base zuzusetzen. Die Menge und Art der Base sollte so gewählt werden, dass bei Zugabe des Säureadduktes der Verbindung der Formel **1** zur Reaktionsmischung durch Deprotonierung zunächst die Verbindung der Formel **1** freigesetzt wird.

Im Rahmen der erfindungsgemäßen Verfahren sind geeignete Basen beispielsweise anorganische Basen, wie Alkalimetallhydroxide, z.B. Lithium-, Natrium- oder Kaliumhydroxid, Erdalkalimetallhydroxide, z.B. Magnesium- oder Calciumhydroxid, Aluminiumhydroxid, oder Alkali- und Erdalkalimetalloxide wie z.B. Natrium-, Magnesium- oder Calciumoxid.

Des Weiteren können Alkali- und Erdalkalicarbonate, z.B. Lithium-, Natrium-, Kalium- oder Calciumcarbonat, Alkali - und Erdalkalihydrogencarbonate, z.B. Lithium-, Natrium- oder Kaliumhydrogencarbonat, und organische Basen, wie Acetate, z.B. Natriumacetat, Alkoholate, z.B. Natriummethanolat, Natriumethanolat, Natrium-tert-butanolat oder Kalium-tert-butanolat und dergleichen; aliphatische und aromatische Amine, wie Diethylamin, Triethylamin, Ethyldiisopropylamin oder Pyridin als Basen eingesetzt werden. Die Alkoholate werden vorzugsweise als wässrige Lösungsmittelsysteme eingesetzt.

Bevorzugte Basen, die zur Deprotonierung der Säureaddukte der Verbindung der Formel **1** geeignet sind, sind beispielsweise Alkali- und Erdalkalimetallcarbonate, - hydrogencarbonate und -hydroxide. Speziell bevorzugt sind Carbonate und Hydrogencarbonate wie Na₂CO₃, K₂CO₃ und NaHCO₃.

Generell ist es für den Reaktionsverlauf günstig, wenn die Verbindung der Formel **1** in der Reaktionsmischung vollständig oder zumindest teilweise in unprotonierter Form vorliegt. Ist dies der Fall, so können zur Oxidation der Verbindung der Formel **1** und der daraus resultierenden Generierung von Arylradikalen mildere Oxidationsmittel eingesetzt werden. Liegt dagegen die Verbindung der Formel **1** als einfaches oder sogar zweifaches Säureaddukt (einfach oder zweifach protoniert) in der Reaktionsmischung vor, so sind zur Oxidation deutlich drastischere Bedingungen notwendig. Diese können zu Nebenreaktionen führen, da unter derartigen Bedingungen auch die Verbindung der Formel **2** sowie das Reaktionsprodukt der Formel **3** oxidiert werden können.

Es hat sich allgemein auch als vorteilhaft erwiesen, die Verbindung der Formel **2** in vollständig oder zumindest teilweise unprotonierter Form in der Reaktion einzusetzen. Wird die Verbindung der Formel **2** als Säureaddukt eingesetzt, so sollte durch Zusatz einer der oben genannten Basen das Säureaddukt in die unprotonierte Form der Verbindung der Formel **2** umgewandelt werden.

In einer alternativen Ausführungsform wird die Verbindung der Formel **2** zusammen mit einem Oxidationsmittel in einem Lösungsmittel oder Lösungsmittelsystem vorgelegt und ein Säureaddukt der Verbindung der Formel **1** zugegeben. Durch den basischen Charakter der Verbindung der Formel **2** wird die Verbindung der Formel **1** aus dem Säureaddukt freigesetzt und kann durch das Oxidationsmittel umgesetzt werden. In dieser Ausführungsform ist es vorteilhaft, wenn die Verbindung der Formel **2** im Überschuss bezüglich des Säureadduktes der Verbindung der Formel **1** eingesetzt wird. Bevorzugt sind 1,5-50 Mol der Verbindung der Formel **2** bezogen auf 1 Mol der Verbindung der Formel **1.**

Unter Säureaddukten sind im Zusammenhang mit den Verbindungen der Formeln 1 und 2 ein- und zweifache Additionsprodukte dieser Verbindungen mit Säuren, insbesondere mit Mineralsäuren wie Hydrogenchlorid, Hydrogenbromid, Hydrogeniodid, Schwefelsäure, Salpetersäure, Tetrafluorborsäure, Essigsäure und Trifluoressigsäure zu verstehen.

Unter "Lösungsmittelsystemen" wird eine Mischung aus mindestens zwei Lösungsmitteln verstanden, die unabhängig voneinander aus den Gruppen wässriger, organischer und/oder anorganischer Lösungsmittel ausgewählt sind. Bevorzugt ist ein weitgehend inertes organisches Lösungsmittel, wie oben beschrieben, eines der verwendeten Lösungsmittel des Lösungsmittelsystems. Speziell bevorzugt ist Acetonitril.

Ein weiteres geeignetes Lösungsmittelsystem ist ein Zweiphasen-Lösungsmittelsystem, welches zwei miteinander im Wesentlichen nicht mischbare Lösungsmittel oder Lösungsmittelsysteme umfasst. "Im Wesentlichen nicht mischbar" bedeutet, dass sich ein erstes Lösungsmittel oder Lösungsmittelsystem, das in geringerer oder gleicher Menge wie ein zweites Lösungsmittel oder Lösungsmittelsystem eingesetzt wird, im zweiten Lösungsmittel oder Lösungsmittelsystem zu höchstens 20 Gew.-%, vorzugsweise zu höchstens 10 Gew.-% und insbesondere zu höchstens 5 Gew.-%, bezogen auf das Gesamtgewicht des ersten Lösungsmittels oder Lösungsmittelsystems, löst. Beispiele sind Systeme, die neben einem wie oben definierten wässrigen Lösungsmittel bzw. Lösungsmittelsystem ein oder mehrere mit Wasser im Wesentlichen nicht mischbare Lösungsmittel enthalten, wie Carbonsäureester, z.B. Ethylacetat, Propylacetat oder Ethylpropionat, offenkettige Ether, wie Diethylether, Dipropylether, Dibutylether, Methylisobutylether und Methyl-tert-butylether, aliphatische Kohlenwasserstoffe, wie Pentan, Hexan, Heptan und Octan sowie Petrolether, halogenierte aliphatische Kohlenwasserstoffe, wie Methylenchlorid, Trichlormethan, Dichlorethan und Trichlorethan, cycloaliphatische Kohlenwasserstoffe, wie Cyclopentan und Cyclohexan, und aromatische Kohlenwasserstoffe, wie Benzol, Toluol, die Xylole, Chlorbenzol, Dichlorbenzole und Mesitylen.

Ein solches Zweiphasen-Lösungsmittelsystem kann außerdem wenigstens einen Phasentransferkatalysator enthalten.

Phasentransferkatalysatoren sind dem Fachmann hinreichend bekannt und umfassen beispielsweise geladene Systeme, wie organische Ammoniumsalze, beispielsweise Tetra-(C₁-C₁₈-alkyl)-ammoniumchloride oder -bromide, wie Tetramethylammoniumchlorid oder -bromid, Tetrabutylammoniumchlorid oder -bromid, Hexadecyltrimethylammoniumchlorid oder -bromid, Octadecyltrimethylammoniumchlorid oder -bromid, Methyltrihexylammoniumchlorid oder -bromid, Methyltrioctylammoniumchlorid oder - bromid oder Benzyltrimethylammoniumhydroxid (Triton B), ferner Tetra-(C₁-C₁₈-alkyl)-phosphoniumchloride oder -bromide, wie Tetraphenylphosphoniumchlorid oder - bromid, [(Phenyl)ₐ₋(C₁-C₁₈-alkyl)_{b}]-phosphoniumchloride oder -bromide, worin a = 1 bis 3 und b = 3 bis 1 und die Summe a + b = 4 ist, und außerdem Pyridiniumsalze, wie Methylpyridinium-chlorid oder -bromid, und ungeladene Systeme, wie Kronenether oder Azakronenether, z.B. 12-Krone-4, 15-Krone-5, 18-Krone-6, Dibenzo-18-Krone-6 oder [2,2,2]-Kryptand (222-Kryptofix), Cyclodextrine, Calixarene, wie [14]-Metacyclophan, Calix[4]aren und p-tert-Butyl-Calix[4]aren, und Cyclophane.

Geeignet ist auch die Durchführung in einem Zweiphasensystem, wobei eine der Phasen wenigstens eines der oben genannten Lösungsmittel bzw. Lösungsmittelsysteme umfasst und die zweite Phase mit der ersten Phase im Wesentlichen nicht mischbar ist. Bevorzugt umfasst die erste Phase wenigstens eines der o.g. protischen Lösungsmittel, wie Wasser, Alkohole oder Diole. Besonders bevorzugt ist die erste Phase ein wässriges Lösungsmittel oder Lösungsmittelystem, oder ein Gemisch aus Wasser mit wenigstens einem mit Wasser mischbaren organischen Lösungsmittel, wie z.B. Alkohole, wie Methanol, Ethanol, Propanol oder Trifluorethanol, Diole, wie Ethylenglykol, Acetonitril, Amide, wie Dimethylformamid, und Ketone, wie Aceton.

Die zweite Phase ist vorzugsweise ausgewählt unter offenkettigen Ethern, wie Diethylether, Dipropylether, Dibutylether, Methylisobutylether und Methyl-tert-butylether, aliphatischen Kohlenwasserstoffen, wie Pentan, Hexan, Heptan und Octan sowie Petrolether, halogenierten aliphatischen Kohlenwasserstoffen, wie Methylenchlorid, Trichlormethan, 1,2-Dichlorethan und Trichlorethan, cycloaliphatischen Kohlenwasserstoffen, wie Cyclopentan und Cyclohexan.

Wird die Verbindung der Formel **1** und/oder die Verbindung der Formel **2** als Säureaddukt eingesetzt, so hat es sich als vorteilhaft erwiesen, der ersten Phase eine der o.g. Basen zuzusetzen. Die Menge der Base wird dabei idealerweise so gewählt, dass sowohl die Verbindung der Formel **1** als auch die Verbindung der Formel **2** in der Reaktionslösung in unprotonierter Form vorliegen.

Im Rahmen der vorliegenden Erfindung versteht man unter nichtpolaren (apolaren) Lösungsmitteln beispielsweise Schwefelkohlenstoff, Tetrachlormethan, die meisten aromatischen und die gesättigten aliphatischen Kohlenwasserstoffe.

Der Begriff "schwach polare Lösungsmittel" bezeichnet beispielsweise Halogen- und manche aromatischen Kohlenwasserstoffe. Die empirische bestimmte und in Einheiten der sog. E_{T}(30)-Skala ausgedrückte Polarität steigt z.B. von apolarem n-Hexan über Toluol, Chloroform, n-Butanol, Aceton, Ethanol, Formamid bis zum stark polaren Wasser.

Im Rahmen der vorliegenden Erfindung versteht man unter "polaren organischen Lösungsmitteln" beispielsweise kurzkettige Nitrile, wie Acetonitril oder Propionitril, Amide, wie Dimethylformamid, kurzkettige ein- oder mehrwertige Alkohole, wie Methanol, Ethanol, Propanol, Isopropanol, Ethylenglykol oder Trifluorethanol, kurzkettige Carbonsäuren, wie Eisessig, Trifluoressigsäure, kurzkettige Ketone, wie Aceton, und Dimethylsulfoxid.

In einer alternativen Ausführungsform werden die Lösungsmittel oder Lösungsmittelsysteme in entgaster (d.h. speziell in von Sauerstoff befreiter) Form eingesetzt. Das Entgasen von Lösungsmitteln oder Lösungsmittelsystemen ist bekannt und kann beispielsweise durch ein- oder mehrfaches Einfrieren des Lösungsmittels oder Lösungsmittelsystems, Auftauen unter Vakuum (zum Entziehen des im Lösungsmittel oder Lösungsmittelsystem gelösten/dispergierten Gases) und Kompensieren mit einem Inertgas, wie Stickstoff oder Argon, erfolgen. Alternativ oder zusätzlich kann das Lösungsmittel oder Lösungsmittelsystem mit Ultraschall behandelt werden. Letztere Vorgehensweise bietet sich insbesondere bei Wasser bzw. wässrigen Lösungsmitteln oder Lösungsmittelsystemen an, da die Ausdehnung von Wasser beim Frieren zu apparativen Problemen führen kann.

Zusätzlich erfolgt in einer bevorzugten Ausführungsform des erfindungs-gemäßen Verfahrens die Durchführung in wenigstens einem Lösungsmittel bzw. Lösungsmittelsystem, das die Oxidation und radikalische Zersetzung der Verbindung der Formel 1 in Stickstoff und ein Arylradikal bewirkt und/oder die Umsetzung in einer anderen Art und Weise begünstigt. "In einer anderen Art und Weise begünstigt" bedeutet beispielsweise, dass das Lösungsmittel bzw. Lösungsmittelsystem die Bildung des Arylradikals fördert.

Lösungsmittel bzw. Lösungsmittelsysteme können die radikalische Zersetzung der Verbindung der Formel 1 in ein Arylradikal beispielsweise dadurch fördern, dass ein Oxidationsmittel wie Sauerstoff in ihnen eine hohe Löslichkeit besitzt. Diese Eigenschaft ist speziell dann von Vorteil, wenn die Reaktion allein mit Sauerstoff oder Luftsauerstoff als Oxidationsmittel durchgeführt werden soll.

Wird Sauerstoff oder Luftsauerstoff als Oxidationsmittel eingesetzt, so umfasst das erfindungsgemäße Verfahren auch die Durchführung unter Gasgemischen, die Sauerstoff in einem Volumenanteil von 0,1-100% enthalten. Sauerstoff kann dabei beispielsweise im Gemisch mit Stickstoff oder Argon eingesetzt werden.

Geeignet sind alternativ auch oxidierende Gase, wie Stickstoffmonoxid, Stickstoffdioxid, Distickstofftetroxid oder Ozon. Diese Gase werden in einem Volumenanteil von 0,1-100% eingesetzt, wobei sie gegebenenfalls mit Sauerstoff, Stickstoff und/oder Argon gemischt vorliegen können.

Reaktionen, in denen gasförmige Oxidationsmittel wie Sauerstoff oder Stickstoffmonoxid zum Einsatz kommen, können entweder unter einer Atmosphäre des entsprechenden Gases oder Gasgemisches (wie oben beschrieben) durchgeführt werden, oder das betreffende Gas wird im Reaktionsverlauf durch die Reaktionsmischung geleitet.

Wird reiner Sauerstoff oder Sauerstoff im Gemisch mit anderen Gasen, wie Luftsauerstoff, als Oxidationsmittel verwendet, so kann die Geschwindigkeit der Umsetzung der Verbindung der Formel **1** zu Stickstoff und einem Arylradikal dadurch beschleunigt werden, dass geeignete Katalysatoren eingesetzt werden. Derartige Katalysatoren sind beispielsweise Phthalocyanin-Derivate, wie sie kürzlich beschrieben wurden von T. Taniguchi, Y. Sugiura, H. Zaimoku, H. Ishibashi, Angew. Chem. Int. Ed. 2010, 49, 10154-10157.

Geeignet sind besonders solche Lösungsmittel bzw. Lösungsmittelsysteme, die keine leicht abstrahierbaren Wasserstoffatome besitzen, da sie ein gebildetes Arylradikal bestmöglich vor Nebenreaktionen schützen.

Im Rahmen des erfindungsgemäßen Verfahrens ist es nicht notwendig, dass die Verbindung der Formel **2** im eingesetzten Lösungsmittel oder Lösungsmittelsystem vollständig löslich ist.

Alternativ verwendet man nicht die reinen Lösungsmittel, sondern Gemische, die die Lösungseigenschaften vereinigen, oder man greift zu Lösungsvermittlern.

Der Begriff "Lösungsvermittler" bezeichnet (grenzflächenaktive) Stoffe, die durch ihre Gegenwart andere, in einem Lösungsmittel oder Lösungsmittelsystem praktisch unlösliche Verbindungen in diesem Lösungsmittel oder Lösungsmittelsystem löslich oder emulgierbar machen. Es gibt Lösungsvermittler, die mit der schwer löslichen Substanz eine Molekülverbindung eingehen und solche, die durch Micellen-Bildung wirken. Man kann auch sagen, dass Lösungsvermittler einem sog. latenten Lösungsmittel sein Lösungsvermögen verleihen. Beispielsweise ist Ethanol (als latentes Lösungsmittel) löst Celluloseacetat schlecht, nach Zusatz von 2-Nitropropan (als Lösungsvermittler) jedoch viel besser.

Die erfindungsgemäße Umsetzung erfolgt, indem man die Verbindung der Formel **1** und eine Verbindung der Formel **2** vorzugsweise in einem geeigneten Lösungsmittel oder Lösungsmittelsystem miteinander unter Reaktionsbedingungen in Kontakt bringt, die eine Zersetzung der Verbindung der Formel **1** in Stickstoff und ein Arylradikal bewirken.

Die Reaktanden können prinzipiell in unterschiedlicher Reihenfolge miteinander in Kontakt gebracht werden. So kann beispielsweise die Verbindung der Formel **2,** gegebenenfalls in einem Lösungsmittel bzw. Lösungsmittelsystem gelöst oder dispergiert, zusammen mit dem Oxidationsmittel vorgelegt und mit der Verbindung der Formel **1,** gegebenenfalls in einem Lösungsmittel bzw. Lösungsmittelsystem gelöst oder dispergiert, versetzt werden.

Umgekehrt kann die Verbindung der Formel **1** zusammen mit der Verbindung der Formel **2,** gegebenenfalls in einem Lösungsmittel bzw. Lösungsmittelsystem gelöst oder dispergiert, vorgelegt und mit dem Oxidationsmittel, gegebenenfalls in einem Lösungsmittel bzw. Lösungsmittelsystem gelöst oder dispergiert, versetzt werden. Im Fall von gasförmigen Oxidationsmitteln wird die Reaktion unter der entsprechenden Gasatmosphäre durchgeführt.

Es hat sich jedoch als vorteilhaft erwiesen, die Verbindung der Formel **2,** gegebenenfalls in einem Lösungsmittel bzw. Lösungsmittelsystem zusammen mit dem Oxidationsmittel vorzulegen und die Verbindung der Formel **1,** gegebenenfalls in einem Lösungsmittel bzw. Lösungsmittelsystem gelöst oder dispergiert, langsam hinzuzugeben. Auf diese Weise wird die Bildung von Homokupplungsprodukten (siehe oben) weitgehend unterdrückt.

In einer alternativen Ausführungsform können die Verbindungen der Formel **1** und der Formel **2** und das Oxidationsmittel zunächst in einem Lösungsmittel oder Lösungsmittelsystem gelöst oder dispergiert werden. In diese Ausführungsform ist es speziell vorteilhaft, wenn die Mischung der Verbindungen der Formel **1** und **2** und des Oxidationsmittels bei einer Temperatur erfolgt, bei der noch keine wesentliche Oxidation der Verbindung der Formel **1** eintritt. Im Folgenden wird das Gemisch der Verbindungen der Formel **1** und **2** und des Oxidationsmittels erhitzt, worauf die oxidative Zersetzung der Verbindung der Formel **1** in Stickstoff und ein Arylradikal einsetzt. Entsprechend geeignete Temperaturbereiche sind von der eingesetzten Verbindung der Formel **1** sowie der Art des Oxidationsmittels abhängig und müssen in Vorversuchen ermittelt werden.

Vorteilhaft ist auch die Zugabe des Gemisches aus den Verbindungen der Formeln **1** und **2,** gegebenenfalls in einem Lösungsmittel bzw. Lösungsmittelsystem gelöst oder dispergiert, zum Oxidationsmittel, das gegebenenfalls in einem Lösungsmittel bzw. Lösungsmittelsystem gelöst oder dispergiert vorgelegt wird.

Die Umsetzung kann sowohl in einem Lösungsmittel bzw. Lösungsmittelsystem als auch in Substanz durchgeführt werden. In letzterem Fall fungiert beispielsweise die Verbindung der Formel **2** selbst als Lösungs- oder Dispergiermittel oder wird, falls sein Schmelzpunkt oberhalb Raumtemperatur (25°C) liegt, als Schmelze zusammen mit dem Oxidationsmittel vorgelegt und dann mit der Verbindung der Formel **1** unter geeigneten Reaktionsbedingungen versetzt. Die bevorzugte Ausführungsform ist jedoch die Durchführung in einem Lösungsmittel bzw. Lösungsmittel-system, das gegebenenfalls mindestens eine Base enthält.

Vorzugsweise wird die Verbindung der Formel **2** direkt als freies Amin eingesetzt. Alternativ kann es auch, entweder vollständig oder teilweise, in Form eines seiner Säureaddukte oder einer Mischung solcher Addukte eingesetzt werden, wobei das Hydrochlorid der Verbindung der Formel **2** besonders bevorzugt ist. Bei der Verwendung der Säureaddukte der Verbindung der Formel **2** ist es vorteilhaft durch Zusatz mindestens einer Base zu gewährleistet, dass die Verbindung der Formel **2** während der Reaktion zumindest teilweise in unprotonierter Form, vorliegt.

Die Durchführung erfolgt beispielsweise so, dass die Verbindung der Formel **2** zusammen mit dem Oxidationsmittel in einem Lösungsmittel oder Lösungsmittelsystem vorgelegt und dann die Verbindung der Formel **1** sukzessive zugegeben wird oder umgekehrt die Verbindung der Formel **1** zusammen mit dem Oxidationsmittel in einem solchen Lösungsmittel oder Lösungsmittelsystem vorgelegt und dann die Verbindung der Formel **2** zugegeben wird, wobei die erste Variante bevorzugt ist. Bezüglich Zugabegeschwindigkeit und Reaktionstemperatur wird auf die folgenden Ausführungen verwiesen. Bei Durchführung in einem Zweiphasensystem kann alternativ die Verbindung der Formel **2** im Lösungsmittel oder Lösungsmittelsystem der einen Phase und die Verbindung der Formel **1** im Lösungsmittel oder Lösungsmittelsystem der zweiten Phase jeweils vorgelegt werden. Hierbei ist es vorteilhaft, wenn das Oxidationsmittel ganz oder zumindest teilweise in der Phase löslich ist, in der sich die Verbindung der Formel **1** befindet.

Wie bereits gesagt, wird in einer bevorzugten Ausführungsform die Verbindung der Formel **1,** zu einer Vorlage aus der Verbindung der Formel **2** und dem Oxidationsmittel gegeben. Die Verbindung der Formel **1** kann sowohl in Substanz als auch in einem Lösungsmittel oder Lösungsmittelsystem gelöst oder dispergiert zugegeben werden. Als Lösungsmittel können hier Wasser oder die oben genannten wässrigen Lösungsmittelsysteme oder polare organische Lösungsmittel eingesetzt werden, wobei im Fall der Verwendung von Säureaddukten der Verbindung der Formel **1** mindestens eine Base zugesetzt werden kann. Die Verbindung der Formel **1** wird dabei vorzugsweise sukzessive (portionsweise oder kontinuierlich) zugegeben. Die sukzessive Zugabe unterdrückt in vielen Fällen die Bildung von Homokupplungsprodukten, d.h. von Produkten, die durch Reaktion von zwei oder mehreren Verbindungen der Formel **1** miteinander entstehen, denn eine geringe Konzentration der Verbindung der Formel **1** im Reaktionsgemisch gewährleistet, dass seine Umsetzung mit der Verbindung der Formel **2** gegenüber der Umsetzung mit sich selbst überwiegt.

Die Geschwindigkeit der Zugabe wird dabei von mehreren Faktoren bestimmt, wie Ansatzgröße, Temperatur, Reaktivität der Edukte und Art des gewählten Oxidationsmittels, das eine Zersetzung der Verbindung der Formel **1** in Stickstoff und ein Arylradikal bewirkt, und kann vom Fachmann im Einzelfall, beispielsweise durch geeignete Vorversuche, bestimmt werden. So verlangt eine geringe Reaktivität der Verbindung der Formel **1** eine langsamere Zugabegeschwindigkeit. Die geringere Reaktivität der Verbindung der Formel **1** kann aber beispielsweise auch durch eine höhere Temperatur und/oder durch die Wahl eines stärkeren Oxidationsmittels, das eine Zersetzung der Verbindung der Formel **1** bewirkt, zumindest teilweise kompensiert werden.

Die beiden bevorzugten Maßnahmen, d.h. der Einsatz der Verbindung der Formel **1** im Unterschuss (bzgl. der Verbindung der Formel **2**) sowie deren schrittweise Zugabe, bewirken einen vorteilhaften Reaktionsverlauf, da sie die Homokupplung der Verbindung der Formel **1** zurückdrängen.

Erfindungsgemäß wird die Verbindung der Formel **1** und die Verbindung der Formel **2** unter Reaktionsbedingung umgesetzt, die eine Zersetzung der Verbindung der Formel **1** in Stickstoff und ein Arylradikal bewirken. Dabei werden bevorzugt Reaktionsbedingungen gewählt, unter denen die Verbindung der Formel **1** durch ein Oxidationsmittel zu Stickstoff und einem Arylradikal zersetzt wird.

Geeignete Bedingungen, unter denen eine Zersetzung der Verbindung der Formel **1** in Stickstoff und ein Arylradikal stattfindet, sind dem Fachmann allgemein bekannt. So kann bereits die Zugabe der Verbindung der Formel **1** zu der Verbindung der Formel **2** die Zersetzung der Verbindung der Formel **1** in Stickstoff und einem Arylradikal bewirken, wenn die Reaktion unter Luftsauerstoff durchgeführt wird, da Sauerstoff als Oxidationsmittel wirkt.

In diesem Fall müssen keine weiteren Verfahrensmaßnahmen ergriffen werden und die Reaktion kann unter den oben beschriebenen Reaktionsbedingungen durchgeführt werden. Reicht das Oxidationspotential der Luftsauerstoffatmosphäre nicht aus, ist es erforderlich, weitere Verfahrensmaßnahmen zu ergreifen, um den Oxidationsschritt einzuleiten. Derartige Maßnahmen sind speziell die Zugabe eines weiteren, stärkeren Oxidationsmittels oder die Durchführung unter reiner Sauerstoffatmosphäre. Aber auch wenn das Oxidationspotential von Luftsauerstoff ausreichen sollte, um die Zersetzung der Verbindung der Formel **1** in Stickstoff und ein Arylradikal einzuleiten, kann es von Vorteil sein, weitere Oxidationsmittel zuzusetzen, die einen Zerfall oder Abbau der Verbindung der Formel **1** in Stickstoff und ein Arylradikal gewährleisten/beschleunigen bzw. gegenüber anderen Reaktionsmöglichkeiten der Verbindung der Formel **1** bevorzugt ablaufen lassen.

Werden zwei oder mehrere der obigen Maßnahmen kombiniert, so ist erfindungsgemäß eine dieser Maßnahmen die Durchführung in Gegenwart wenigstens eines Oxidationsmittels. Geeignet ist insbesondere die Kombination dieser Maßnahme mit der Durchführung in mindestens einem Lösungsmittel oder Lösungsmittelsystem.

Die Reaktionstemperatur wird von mehreren Faktoren bestimmt, wie beispielsweise der Reaktivität der eingesetzten Edukte und der Art des gewählten Oxidationsmittels, das eine Zersetzung der Verbindung der Formel 1 in Stickstoff und ein Arylradikal bewirkt, und kann vom Fachmann im Einzelfall ermittelt werden, beispielsweise durch einfache Vorversuche. Im Allgemeinen führt man die Umsetzung der Verbindung der Formel **1** mit der Verbindung der Formel **2** bei einer Temperatur im Bereich von vorzugsweise -100 °C bis zum Siedepunkt des Reaktionsgemischs, bevorzugt -78 °C bis 200 °C, besonders bevorzugt von -20 °C bis 100 °C und insbesondere von 0 °C bis 80 °C durch. Diese Temperaturen gelten für die Durchführung in Lösung; wird der Versuch hingegen in Substanz durchgeführt und liegt der Schmelzpunkt der Verbindung der Formel **2** über Raumtemperatur, so entspricht die Reaktionstemperatur selbstverständlich mindestens der Temperatur der Schmelze des Reaktionsgemischs.

Vorzugsweise setzt man in dem erfindungsgemäßen Verfahren zur Herstellung von Verbindungen der Formel **3** die Verbindung der Formel **1** in einer Menge von 0,001 bis 5,0 Mol, besonders bevorzugt von 0,002 bis 1,0 Mol (z. B. 0,02 bis 0,5 Mol) und insbesondere von 0,05 bis 0,3 Mol (z.B. 0,05 bis 0,2 Mol), jeweils bezogen auf 1 Mol der Verbindung der Formel **2** ein.

Verbindungen der Formel **1** sind allgemein bekannt und können gemäß gängiger Verfahren hergestellt werden, wie sie beispielsweise in Houben Weyl, "Methoden d. org. Chemie" 10/2, 169 beschrieben sind. So sind sie durch Diazotierung und Reduktion des entsprechenden Anilinderivates erhältlich, beispielsweise, indem man ein solches Anilinderivat mit Nitrit in Gegenwart einer Säure, wie etwa halbkonzentrierte Schwefelsäure, umsetzt, und anschließend mit salzsaurer Zinn(II)chloridlösung reduziert. Sowohl entsprechende Anilinderivate zur Herstellung von Verbindungen der Formel **1** als auch von Verbindungen der Formel **2** sind bekannt oder können nach bekannten Verfahren hergestellt werden, beispielsweise, indem man entsprechend substituierte Nitrobenzole in Gegenwart eines geeigneten Katalysators hydriert oder homogen reduziert (etwa mit Sn(II)-Chlorid / HCl, vgl. Houben Weyl, "Methoden d. org. Chemie" 11/1, 422). Auch die Herstellung aus Azobenzolen und die Substitution geeigneter Benzole mit Ammoniak sind gängige Methoden.

Die Aufarbeitung der erhaltenen Reaktionsgemische und die Isolierung der Verbindungen der Formel **3** erfolgt in üblicher Weise, beispielsweise durch eine extraktive Aufarbeitung, durch Entfernen des Lösungsmittels, z. B. unter vermindertem Druck, oder durch eine Kombination dieser Maßnahmen. Eine weitere Reinigung kann beispielsweise durch Kristallisation, Destillation oder durch Chromatographie erfolgen.

Überschüssige bzw. nicht umgesetzte Edukte (hierbei handelt es sich vor allem um die Verbindung der Formel **2,** die in Bezug auf die Verbindung der Formel **1** bevorzugt im Überschuss eingesetzt wird) werden bei der Aufarbeitung vorzugsweise isoliert und erneut verwendet.

Entsprechend einer bevorzugten Ausführungsform der Erfindung wird das Reaktionsgemisch zur Aufarbeitung mit Wasser verdünnt und mit einem geeigneten, im Wesentlichen nicht wassermischbaren organischen Lösungsmittel mehrmals extrahiert und die kombinierten organischen Phasen eingeengt. Je nach den Säure-Base-Eigenschaften des Produktes wird der pH-Wert vor der Extraktion gegebenenfalls durch Zugabe von Säuren oder Basen geeignet eingestellt. Beispiele für geeignete, im Wesentlichen nicht wassermischbare organische Lösungsmittel sind oben aufgeführt. Das so isolierte Produkt kann anschließend für Verwendungen bereitgehalten oder direkt einer Verwendung zugeführt werden, beispielsweise in einem weiteren Reaktionsschritt eingesetzt werden, oder zuvor weiter aufgereinigt werden.

### Beispiele:

¹H-NMR-Spektren wurden auf 360 und 600 MHz Spektrometern unter Verwendung von CDCl₃ als Lösungsmittel mit CHCl₃ (7.26 ppm) als Standard aufgenommen. Chemische Verschiebungen sind als parts per million (ppm) angegeben. Kopplungskonstanten sind in Hertz (*J*Hz) angegeben. Die folgenden Abkürzungen werden für die Beschreibung der Signale verwendet: s (Singulett), d (Dublett), dd (Dublett von Dublett), ddd (Dublett von Dublett von Dublett), t (Triplett), q (Quadruplett), m (Multiplett). ¹³C-NMR-Spektren wurden bei 90.6 und 150.9 MHz in CDCl₃ mit CHCl₃ (77.0 ppm) als Standard aufgenommen. Chemische Verschiebungen sind als parts per million (ppm) angegeben. ¹⁹F-NMR-Spektren wurden bei 338.8 MHz in CDCl₃ mit C₆F₆ (-164.9 ppm) als Standard aufgenommen. Massespektren wurden auf einem Jeol GC mate II GC-MS-System mit electron impact (EI) aufgenommen. Analytische Dünnschichtchromatographie (DC) wurde auf Merck Kieselgelplatten unter Verwendung von kurzwelligem (254 nm) UV durchgeführt. Für die Flash-Chromatographie wurde Kieselgel (Kieselgel 60, 40-63 µm, Merck) verwendet.

### Abkürzungen:

THF...... Tetrahydrofuran
EtOAc .. Essigsäureethylester

### I. Allgemeine Vorschriften

### I.1 Herstellung von Arylhydrazinen (AAV 1)

Bei Raumtemperatur wird unter Rühren konzentrierte Salzsäure (50 mL, 36%) zu einer Lösung des Anilins (20.0 mmol) in konzentrierter Essigsäure (10 mL) gegeben. Nach Kühlen des Reaktionsgemisches auf 0 °C wird langsam Natriumnitritlösung (1.38 g, 20.0 mmol in 4 mL Wasser) zugegeben und für eine Stunde bei 0 °C gerührt. Die kalte Lösung wird filtriert, erneut auf 0 °C gekühlt und anschließend tropfenweise mit einer vorgekühlten Lösung aus Zinn(II)-chlorid-dihydrat (10.0 g, 44.3 mmol) in konzentrierter Salzsäure (10 mL) versetzt. Nach einer Stunde wird der entstandene Niederschlag abfiltriert und mit gesättigter, wässriger Natriumchloridlösung gewaschen (30 mL). Der so erhaltene Feststoff wird mit Natriumhydroxidlösung (2 m) in Lösung gebracht und mit Diethylether extrahiert (2 × 100 mL). Die vereinigten organischen Phasen werden mit Wasser gewaschen und über Natriumsulfat getrocknet. Das Lösemittel wird am Rotationsverdampfer entfernt und das erhaltene Hydrazin ohne weitere Reinigung eingesetzt.

### I.2 Allgemeine Vorschrift zur Biarylsynthese mit MnO₂(AAV2)

Unter Rühren wird eine Lösung aus dem Arylhydrazinderivat (1.00 mmol) in 2 mL Acetonitril über einen Zeitraum von 60 Minuten zu einer Suspension aus dem Anilinderivat (20.0 mmol) und MnO₂ (5.00 mmol, 435 mg) in 5 mL Acetonitril zugetropft. Nach beendeter Reaktion wird das Reaktionsgemisch über Celite gefiltert und mit Ethylacetat gewaschen. Das Lösemittel wird bei vermindertem Druck entfernt und das erhaltene Produkt im Vakuum getrocknet. Die weitere Aufreinigung der Produkte erfolgt mittels Kugelrohrdestillation und anschließender Säulenchromatographie an Kieselgel.

### I.3 Allgemeine Vorschrift zur Biarylsynthese mit MnO₂ und NaHCO₃ (AAV 3)

Unter Rühren wird das Arylhydrazinhydrochlorid (1.00 mmol) über einen Zeitraum von 10 Minuten portionsweise zu einer Suspension aus dem Anilinderivat (20.0 mmol), MnO₂ (5.00 mmol, 435 mg) und NaHCO₃ (5.00 mmol, 420 mg) in 5 mL Acetonitril gegeben. Nach beendeter Reaktion wird das Reaktionsgemisch über Celite gefiltert und mit Ethylacetat gewaschen. Die Lösung wird mit Wasser und gesättigter, wässriger Natriumchloridlösung gewaschen und über Natriumsulfat getrocknet. Das Lösemittel wird bei vermindertem Druck entfernt und das erhaltene Produkt im Vakuum getrocknet. Die weitere Aufreinigung der Produkte erfolgt mittels Kugelrohrdestillation und anschließender Säulenchromatographie an Kieselgel.

### II. Spezielle Beispiele

### II.1 4'-Chlor-5-fluorbiphenyl-2-amin

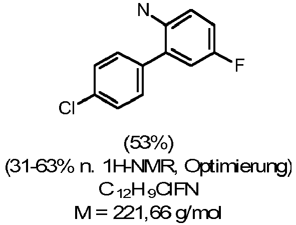

Zur Ermittlung der als AAV 2 beschriebenen Reaktionsbedingungen wurden folgende Optimierungsversuche durchgeführt.

4'-Chlor-5-fluorbiphenyl-2-amin wird aus 4-Chlorphenylhydrazin (1.00 mmol, 143 mg) und 4-Fluoranilin (20.0 mmol, 1.92 mL) entsprechend der allgemeinen Arbeitsvorschrift AAV 2 und den in Tabelle 1 angegebenen Variationen des Oxidationsmittels synthetisiert.

Die in Tabelle 1 angegebenen Ausbeuten wurden mittels ¹H-NMR-Spektrometrie (interner Standard: Diethylterephthalat) bestimmt.

| | |
|---|---|
| Oxidationsmittel | Ausbeute an 4'-Chlor-5-fluorbiphenyl-2-amin [%] (mittels ¹H-NMR und int. Standard Dimethylterephthalat) |
| MnO₂, siehe AAV 2 | 63 |
| KO₂ | 54 |
| Mn(OAc)₃ | 59 |
| KMnO₄ | 56 |
| NalO₄ | 54 |
| K₃[Fe(CN)₆] | 55 |
| H₂O₂ | 36 |
| O₂-Atmosphäre | 31 |
| O₂-Atmosphäre, 0,2 eq. TEMPO | 36 |
| Luft, Raumtemperatur | 33 |
| Luft, 60°C (Zugabe des Hydrazins als Feststoff über 8 h) | 44 |
| Luft, Rückfluss (82°C) (Zugabe des Hydrazins in Lösung über 3 h) | 38 |

Im präparativen Versuch wird 4'-Chlor-5-fluorbiphenyl-2-amin aus 4-Chlorphenylhydrazin (1.00 mmol, 143 mg), 4-Fluoranilin (20.0 mmol, 1.92 mL) und MnO₂ (5.00 mmol, 435 mg) analog der allgemeinen Arbeitsvorschrift AAV 2 synthetisiert. Das erhaltene Rohprodukt wird mittels Säulenchromatographie (Hexan / EtOAc = 10: 1) gereinigt, wodurch 4'-Chlor-5-fluorbiphenyl-2-amin (0.53 mmol, 118 mg, 53%) erhalten wird.

| | |
|---|---|
| *R*_{f}-Wert | 0.6 (Hexan / EtOAc = 4:1) [UV] |
| ¹H-NMR | (360 MHz, CDCl₃): δ(ppm) = 3.54 (br s, 2 H), 6.69 (dd, *J*_{HF} = 4.7 Hz, *J* = 8.5 Hz, 1 H), 6.83 (dd, *J* = 2.8 Hz, *J*_{HF} = 9.0 Hz, 1 H), 6.88 (ddd, *J* = 3.0 Hz, *J*_{HF} = 8.1 Hz, *J* = 8.7 Hz, 1 H), 7.37 (d, *J* = 8.7 Hz, 1 H), 7.42 (d, *J* = 8.7 Hz, 1 H). |
| ¹³C-NMR | (90.6 MHz, CDCl₃): δ(ppm) = 115.3 (d, *J*_{CF} *=* 22.2 Hz, CH), 116.5 (d, *J*_{CF} *=* 22.6 Hz, CH), 116.7 (d, *J*_{CF} *=* 7.7 Hz, CH), 127.4 (d, *J*_{CF} *=* 7.2 Hz, C_{q}), 129.1 (2 × CH), 130.3 (2 × CH), 133.6 (C_{q}), 136.9 (d, *J*_{CF} *=* 1.7 Hz, C_{q}), 139.4 (d, *J*_{CF} *=* 2.2 Hz, C_{q}), 156.4 (d, *J*_{CF} *=* 236.9 Hz, C_{q}). |
| ¹⁹F-NMR | (235 MHz, CDCl₃): δ(ppm) = -116.5. |
| MS (EI) | *m*/*z* (%): 224 (6), 223 (29) [³⁷Cl-M⁺], 222 (18), 221 (100) [³⁵Cl-M⁺], 220 (10), 219 (20), 187 (8), 186 (45), 185 (60), 184 (13), 159 (5), 157 (7), 126 (6), 110 (10), 93 (37). |
| HRMS (EI) | berechnet für C₁₂H₉CIFN [M⁺]: 221.0407, gefunden: 221.0407. |

### II.2 3',4',5'-Trifluorbiphenyl-2-amin

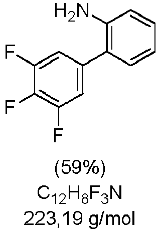

3',4',5'-Trifluorbiphenyl-2-amin wird aus 3,4,5-Trifluorphenylhydrazin (0.90 mmol, 146 mg), Anilin (18.0 mmol, 1.64 mL) und MnO₂ (4.50 mmol, 392 mg) analog der allgemeinen Arbeitsvorschrift AAV 2 synthetisiert. Das erhaltene Rohprodukt wird mittels Säulenchromatographie (Hexan / EtOAc = 10:1 → 4:1) gereinigt und es wird 3',4',5'-Trifluorbiphenyl-2-amin (0.53 mmol, 118 mg, 59%) erhalten.

| | |
|---|---|
| *R*_{f}-Wert | 0.6 (Hexan/EtOAc 4:1) [UV] |
| ¹H-NMR | (360 MHz, CDCl₃): δ(ppm) = 3.79 (br s, 2 H), 6.77 (dd, *J* = 0.8 Hz, *J* = 8.0 Hz, 1 H), 6.83 (dt, *J* = 1.0 Hz, *J* = 7.5 Hz, 1 H), 7.06 (dd, *J* = 1.6 Hz, *J* = 7.6 Hz, 1 H), 7.07-7.13 (m, 2 H), 7.19 (ddd, *J* = 1.6 Hz, *J* = 7.4 Hz, *J* = 8.0 Hz, 1 H). |
| ¹³C-NMR | (151 MHz, CDCl₃): δ(ppm) = 113.2 (dd, *J*_{CF} = 4.5 Hz, *J*_{CF} = 16.4 Hz, 2 CH), 116.0 (CH), 118.9 (CH), 124.4 (d, *J*_{CF} = 1.0 Hz, C_{q}), 129.4 (CH), 130.1 (CH), 135.5 (dt, *J*_{CF} = 4.9 Hz, *J*_{CF} = 7.9 Hz, C_{q}), 138.9 (td, *J*_{CF} = 15.3 Hz, *J*_{CF} = 251.6 Hz, C_{q}), 143.2 (C_{q}), 151.3 (ddd, *J*_{CF} = 4.4 Hz, *J*_{CF} = 9.9 Hz, *J*_{CF} = 250.5 Hz, 2 × C_{q}). |
| ¹⁹F-NMR | (339 MHz, CDCl₃): δ(ppm) = -137.1, -165.4. |
| MS(EI) | *m*/*z* (%): 268 (23), 266 (13), 224 (15), 223 (100) [M⁺], 222 (26), 221 (12), 204 (12), 203 (18), 85 (21), 83 (30). |
| HRMS(EI) | berechnet für C₁₂H₈F₃N [M⁺]: 223.0609, gefunden: 223.0609. |

### II.3 4',5-Dichlorbiphenyl-2-amin

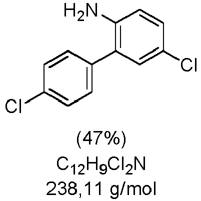

4',5-Dichlorbiphenyl-2-amin wird aus 4-Chlorphenylhydrazin (1.00 mmol, 143 mg), 4-Chloranilin (20.0 mmol, 2.55 g) und MnO₂ (5.00 mmol, 435 mg) analog der allgemeinen Arbeitsvorschrift AAV 2 synthetisiert. Das erhaltene Rohprodukt wird mittels Säulenchromatographie (Hexan / EtOAc = 10:1) gereinigt, wodurch 4',5-Dichlorbiphenyl-2-amin (0.47 mmol, 112 mg, 47%) erhalten wird.

| | |
|---|---|
| *R*_{f}-Wert | 0.6 (Hexan / EtOAc = 4:1) [UV] |
| ¹H-NMR | (360 MHz, CDCl₃): δ(ppm) = 3.72 (br s, 2 H), 6.69 (d, *J* = 8.5 Hz, 1 H), 7.06 (d, *J* = 2.5 Hz, 1 H), 7.11 (dd, *J* = 2.5 Hz, *J* = 8.5 Hz, 1 H), 7.36 (d, *J* = 8.8 Hz, 2 H), 7.42 (d, *J* = 8.7 Hz, 2 H). |
| ¹³C-NMR | (90.6 MHz, CDCl₃): δ(ppm) = 116.9 (CH), 123.4 (C_{q}), 128.5 (CH), 129.2 (2 × CH), 129.8 (CH), 130.3 (2 × CH), 131.5 (C_{q}), 133.7 (C_{q}), 136.7 (C_{q}), 141.9 (C_{q}). |
| MS (EI) | m/z (%): 241 (10) [³⁷Cl₂-M⁺], 240 (11), 239 (29) [³⁷Cl-³⁵Cl-M⁺], 238 (19), 237 (100) [³⁵Cl₂-M⁺], 203 (12), 202 (26), 201 (31), 167 (60), 166 (18), 139 (11), 100 (17). |
| HRMS (EI) | berechnet für C₁₂H₉Cl₂N [M⁺]: 237.0112, gefunden: 237.0112. |

### II.4 5-Brom-4'-chlorbiphenl-2-amin

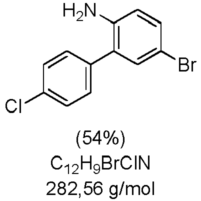

5-Brom-4'-chlorbiphenyl-2-amin wird aus 4-Chlorphenylhydrazin-hydrochlorid (1.00 mmol, 179 mg), 4-Bromanilin (20.0 mmol, 3.44 g), NaHCO₃ (5.00 mmol, 420 mg) und MnO₂ (5.00 mmol, 435 mg) analog der allgemeinen Arbeitsvorschrift AAV 3 synthetisiert. Das erhaltene Rohprodukt wird mittels Säulenchromatographie (Hexan / EtOAc = 10:1) gereinigt, wodurch 5-Brom-4'-chlorbiphenyl-2-amin (0.54 mmol, 152 mg, 54%) erhalten wird.

| | |
|---|---|
| *R*_{f}-Wert | 0.5 (Hexan / EtOAc = 4:1) [UV] |
| ¹H-NMR | (600 MHz, CDCl₃): δ(ppm) = 3.77 (br s, 2 H), 6.64 (d, *J =* 8.5 Hz, 1 H), 7.20 (d, *J =* 2.4 Hz, 1 H), 7.24 (dd, *J =* 2.4 Hz, *J =* 8.5 Hz, 1 H), 7.36 (d, *J =* 8.6 Hz, 2 H), 7.42 (d, *J =* 8.6 Hz, 2 H). |
| ¹³C-NMR | (151 MHz, CDCl₃): δ(ppm) = 117.4 (C_{q}), 129.2 (2 × CH), 130.3 (2 × CH), 130.6 (C_{q}), 131.4 (CH), 132.1 (C_{q}), 132.7 (CH), 133.7 (C_{q}), 136.4 (C_{q}). Ein CH-Signal fehlt aufgrund von Überlappung. |
| MS(EI) | *m*/*z*(%): 285 (23) [³⁷Cl-⁸¹Br-M⁺], 284 (10), 283 (100) [³⁷Cl-⁷⁹Br-M⁺; ³⁵Cl-⁸¹Br -M⁺], 282 (10), 281 (66) [³⁵Cl-⁷⁹Br-M⁺], 201 (12), 168 (10), 167 (73), 166 (19), 140 (11), 139 (12), 83 (27). |
| HRMS(EI) | berechnet für C₁₂H₉BrClN [M⁺]: 280.9607, gefunden: 280.9606. |

### II.5 2'-Chlor-5-fluorbihenl-2-amin

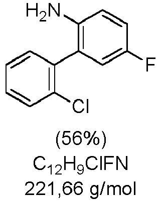

2'-Chlor-5-fluorbiphenyl-2-amin wird aus 2-Chlorphenylhydrazin (1.00 mmol, 143 mg), 4-Fluoranilin (20.0 mmol, 1.92 mL) und MnO₂ (5.00 mmol, 435 mg) analog der allgemeinen Arbeitsvorschrift AAV 2 synthetisiert. Das erhaltene Rohprodukt wird mittels Säulenchromatographie (Hexan / EtOAc = 10:1) gereinigt, wodurch 2'-Chlor-5-fluor-biphenyl-2-amin (0.56 mmol, 124 mg, 56%) erhalten wird.

| | |
|---|---|
| *R*_{f}-Wert | 0.4 (Hexan / EtOAc = 4:1) [UV] |
| ¹H-NMR | (600 MHz, CDCl₃): δ(ppm) = 3.47 (br s, 2 H), 6.73 (dd, *J*_{HF} *=* 4.8 Hz, *J =* 8.8 Hz, 1 H), 6.81 (dd, *J =* 3.0Hz, *J*_{HF} *=* 8.9 Hz, 1 H), 6.93 (dt, *J =* 3.0 Hz, *J*_{HF} *=* 8.4 Hz, *J =* 8.5 Hz, 1 H), 7.30-7.36 (m, 3 H), 7.48-7.52 (m, 1 H). |
| ¹³C-NMR | (90.6 MHz, CDCl₃): δ(ppm) = 115.7 (d, *J*_{CF} *=* 22.2 Hz, CH), 116.7 (d, *J*_{CF} *=* 7.8 Hz, CH), 116.8 (d, *J*_{CF} *=* 22.7 Hz, CH), 126.5 (d, *J*_{CF} *=* 7.3 Hz, C_{q}), 127.3 (CH), 129.5 (CH), 130.0 (CH), 131.7 (CH), 133.7 (C_{q}), 136.8 (d, *J*_{CF} *=*1.6 Hz, C_{q}), 139.6 (C_{q}), 156.1 (d, *J*_{CF} *=* 237.2 Hz, C_{q}). |
| ¹⁹F-NMR: | (339 MHz, CDCl₃): δ(ppm) = -129.6. |
| MS(EI) | *m*/*z*(%): 252 (15), 250 (9), 223 (13) [³⁷Cl-M⁺], 221 (39) [³⁵Cl-M⁺], 187 (13), 186 (100), 185 (64), 184 (13), 157 (9), 92 (13). |
| HRMS(EI) | berechnet für C₁₂H₉ClFN [M⁺]: 221.0407, gefunden: 221.0407. |

### II.6 4'-Chlorbiphenyl-2-amin

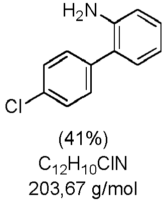

4'-Chlorbiphenyl-2-amin wird aus 4-Chlorphenylhydrazin (1.00 mmol, 143 mg), Anilin (20.0 mmol, 1.82 mL) und MnO₂ (5.00 mmol, 435 mg) analog der allgemeinen Arbeitsvorschrift AAV 2 synthetisiert. Das erhaltene Rohprodukt wird mittels Säulenchromatographie (Hexan / EtOAc = 10:1 → 4:1) gereinigt und es wird 4'-Chlorbiphenyl-2-amin (0.41 mmol, 83.9 mg, 41%) erhalten.

| | |
|---|---|
| R_{f}-Wert | 0.6 (Hexan / EtOAc = 4:1) [UV] |
| ¹H-NMR | (600 MHz, CDCl₃): δ(ppm) = 4.09 (br s, 2 H), 6.80 (dd, *J* = 1.1 Hz, *J* = 8.0 Hz, 1 H), 6.85 (dt, *J* = 1.1 Hz, *J* = 7.5 Hz, 1 H), 7.10 (dd, *J* = 1.6 Hz, *J* = 7.6 Hz, 1 H), 7.15-7.21 (m, 1 H), 7.40 (s, 4 H). |
| ¹³C-NMR | (90.6 MHz, CDCl₃): δ(ppm) = 115.7 (CH), 118.8 (CH), 126.3 (C_{q}), 128.8 (CH), 129.0 (2 × CH), 130.3 (CH), 130.4 (2 × CH), 133.1 (C_{q}), 137.9 (C_{q}), 143.4 (C_{q}). |
| MS (EI) | m/z (%): 205 (29) [³⁷Cl-M⁺], 204 (10), 203 (100) [³⁵Cl-M⁺], 202 (12), 169 (17), 168 (56), 167 (37), 166 (14), 83 (29). |
| HRMS (EI) | berechnet für C₁₂H₁₀ClN [M⁺]: 203.0502, gefunden: 203.0502. |
| II.7 | 2'-Chlorbiphenyl-2-amin |

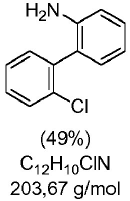

2'-Chlorbiphenyl-2-amin wird aus 2-Chlorphenylhydrazin (1.00 mmol, 143 mg), Anilin (20.0 mmol, 1.82 mL) und MnO₂ (5.00 mmol, 435 mg) analog der allgemeinen Arbeitsvorschrift AAV 2 synthetisiert. Das erhaltene Rohprodukt wird mittels Säulenchromatographie (Hexan / EtOAc = 10:1 → 4:1) gereinigt und es wird 2'-Chlorbiphenyl-2-amin (0.49 mmol, 98.7 mg, 49%) erhalten.

| | |
|---|---|
| *R*_{f}-Wert | 0.4 (Hexan / EtOAc = 4:1) [UV] |
| ¹H-NMR | (600 MHz, CDCl₃): δ(ppm) = 3.83 (br s, 2 H), 6.83 (dd, *J =* 0.9 Hz, *J =* 8.0 Hz, 1 H), 6.86 (dt, *J =* 1.1 Hz, *J =* 7.5 Hz, 1 H), 7.07 (dd, *J =* 1.5 Hz, *J =* 7.4 Hz, 1 H), 7.22 (ddd, *J =* 1.6 Hz, *J =* 7.4 Hz, *J =* 8.0 Hz, 1 H), 7.29-7.36 (m, 3 H), 7.48-7.52 (m, 1 H). |
| ¹³C-NMR | (151 MHz, CDCl₃): δ(ppm) = 115.9 (CH), 118.8 (CH), 125.7 (C_{q}), 127.2 (CH), 129.1 (CH), 129.1 (CH), 129.9 (CH), 130.4 (CH), 131.9 (CH), 133.9 (C_{q}), 137.8 (C_{q}), 143.1 (C_{q}). |
| MS(EI) | *m*/*z* (%): 205 (16) [³⁷Cl-M⁺], 203 (53) [³⁵Cl-M⁺], 178 (21), 169 (20), 168 (100), 167 (95), 166 (17), 161 (11), 140 (10), 139 (15), 137 (13), 125 (10), 123 (10), 112 (11), 11 (15), 109 (14), 99 (10), 97 (24), 96 (10), 95 (19), 85 (20), 84 (11), 83 (23), 83 (25), 82 (13), 81 (35), 71 (35), 70 (16), 69 (59), 68 (10), 67 (12), 57 (55), 56 (10), 55(32), 44 (17), 43 (34). |
| HRMS(EI) | berechnet für C₁₂H₁₀ClN [M⁺]: 203.0502, gefunden: 203.0501. |

### II.8 5-Brom-2'-chlorbiphenyl-2-amin

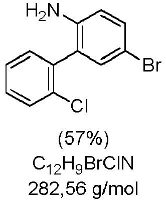

5-Brom-2'-chlorbiphenyl-2-amin wird aus 2-Chlorphenylhydrazin (1.00 mmol, 143 mg), 4-Bromanilin (20.0 mmol, 3.44 g) und MnO₂ (5.00 mmol, 435 mg) analog der allgemeinen Arbeitsvorschrift AAV 2 synthetisiert. Das erhaltene Rohprodukt wird mittels Säulenchromatographie (Hexan / EtOAc = 10:1) gereinigt, wodurch 5-Brom-2'-chlorbiphenyl-2-amin (0.57 mmol, 162 mg, 57%) erhalten wird.

| | |
|---|---|
| *R*_{f}-Wert | 0.4 (Hexan / EtOAc = 4:1) [UV] |
| ¹H-NMR | (600 MHz, CDCl₃): δ(ppm) = 3.77 (br s, 2 H), 6.69 (d, *J =* 8.6 Hz, 1 H), 7.18 (d, *J =* 2.3 Hz, 1 H), 7.27-7.36 (m, 4 H), 7.47-7.52 (m, 1 H). |
| ¹³C-NMR | (90.6 MHz, CDCl₃): δ(ppm) = 129.7 (C_{q}), 136.8 (CH), 146.8 (C_{q}), 146.9 (CH), 149.1 (CH), 149.5 (CH), 151.3 (CH), 151.3 (CH), 152.4 (CH), 153.3 (C_{q}), 156.0 (C_{q}), 162.0 (C_{q}). |
| MS(EI) | *m*/*z* (%): 285 (23), 284 (12) [³⁷Cl-M⁺], 283 (90), 282 (10) [³⁵Cl-M⁺], 281 (73), 248 (73), 247 (40), 246 (69), 245 (34), 168 (37), 167 (100), 166 (71), 164 (10), 140 (28), 139 (52), 138 (10), 101 (13), 83 (40), 83 (23), 82 (20), 69 (21), 63 (11). |
| HRMS(EI) | berechnet für C₁₂H₁₀ClN [M⁺]: 280.9607, gefunden: 280.9607. |

### II.9 4'-Cyano-5-fluorbiphenyl-2-amin

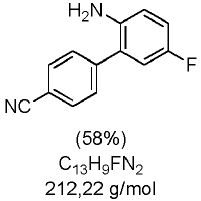

4'-Cyano-5-fluorbiphenyl-2-amin wird aus 4-Cyanophenylhydrazin (1.00 mmol, 133 mg), 4-Fluoranilin (20.0 mmol, 1.92 mL) und MnO₂ (5.00 mmol, 435 mg) analog der allgemeinen Arbeitsvorschrift AAV 2 synthetisiert. Das erhaltene Rohprodukt wird mittels Säulenchromatographie (Hexan / EtOAc = 10:1) gereinigt, wodurch 4'-Cyano-5-fluorbiphenyl-2-amin (0.58 mmol, 123 mg, 58%) erhalten wird.

| | |
|---|---|
| R_{f}-Wert | 0.2 (Hexan / EtOAc = 4:1) [UV] |
| ¹H-NMR | (600 MHz, CDCl₃): δ(ppm) = 3.68 (br s, 2 H), 6.73 (dd, *J*_{HF} = 4.7 Hz, *J* = 8.7 Hz, 1 H), 6.84 (dd, *J* = 2.9 Hz, *J*_{HF} = 9.0 Hz, 1 H), 6.93 (dt, *J* = 2.9 Hz, *J* = 8.4 Hz, *J*_{HF} = 8.4 Hz, 1 H), 7.59 (d, *J* = 8.2 Hz, 2 H), 7.75 (d, *J* = 8.3 Hz, 2 H). |
| ¹³C-NMR | (90.6 MHz, CDCl₃): δ(ppm) = 111.4 (C_{q}), 116.2 (d, *J*_{CF} = 18.3 Hz, CH), 116.4 (d, *J*_{CF} = 18.9 Hz, CH), 117.1 (d, *J*_{CF} = 7.6 Hz, CH), 118.6 (C_{q}), 126.3 (d, *J*_{CF} = 11.3 Hz, C_{q}), 129.7 (2 × CH), 132.7 (2 × CH), 139.4 (d, *J*_{CF} = 2.0 Hz, C_{q}), 143.5 (d, *J*_{CF} = 1.7 Hz, C_{q}), 156.4 (d, *J*_{CF} = 237.4 Hz, C_{q}). |
| ¹⁹F-NMR: | (339 MHz, CDCl₃): δ(ppm) = -128.6. |
| MS(EI) | m/z (%): 213 (15), 212 (100), [³⁵Cl-M⁺], 211 (38), 210 (18), 185 (5), 184 (9), 158 (4), 157 (4), 85 (9), 83 (12). |
| HRMS(EI) | berechnet für C₁₃H₉ F N₂ [M⁺]: 212.0750, gefunden: 212.0750. |

### II.10 5-Brom-4'-fluorbiphenyl-2-amin

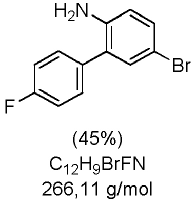

5-Brom-4'-fluorbiphenyl-2-amin wird aus 4-Fluorphenylhydrazin (1.00 mmol, 126 mg), 4-Bromanilin (20.0 mmol, 3.44 g) und MnO₂ (5.00 mmol, 435 mg) analog der allgemeinen Arbeitsvorschrift AAV 2 synthetisiert. Das erhaltene Rohprodukt wird mittels Säulenchromatographie (Hexan / EtOAc = 10:1) gereinigt, wodurch 5-Brom-4'-fluorbiphenyl-2-amin (0.45 mmol, 119 mg, 45%) erhalten wird.

| | |
|---|---|
| *R*_{f}-Wert | 0.5 (Hexan / EtOAc = 4:1) [UV] |
| ¹H-NMR | (600 MHz, CDCl₃): δ(ppm) = 3.93 (br s, 2 H), 6.65 (d, *J =* 8.5 Hz, 1 H), 7.13 (t, *J =* 8.8 Hz, *J*_{HF} *=* 8.8 Hz, 2 H), 7.21 (d, *J =* 2.3 Hz, 1 H), 7.24 (dd, *J =* 2.3 Hz, *J =* 8.5 Hz, 1 H), 7.38 (dd, *J*_{HF} *=* 5.4 Hz, *J =* 8.8 Hz, 2 H). |
| ¹³C-NMR | (90.6 MHz, CDCl₃): δ(ppm) = 110.6 (C_{q}), 115.9 (d, *J*_{CF} =21.4 Hz, 2 x CH), 117.4 (CH), 128.7 (C_{q}), 130.7 (d, *J*_{CF} =8.03 Hz, 2 x CH), 131.2 (CH), 132.8 (CH), 133.94 (d, *J*_{CF} =3.4 Hz, C_{q}), 142.2 (C_{q}), 162.32 (d, *J*_{CF} *=* 247.3 Hz, C_{q}). |
| ¹⁹F-NMR: | (339 MHz, CDCl₃): δ(ppm) = -117.3. |
| MS(EI) | *m*/*z* (%): 327 (10), 268 (13), 267 (81) [⁸¹Br-M⁺], 266 (23), 265 (91) [⁷⁹Br-M⁺], 264 (12), 252 (43), 250 (23), 235 (27), 233 (16), 219 (16), 186 (27), 185 (100), 184 (23), 167 (19), 166 (16), 158 (11), 157 (21), 139 (11), 133 (13), 93 (22), 92 (37), 85 (19), 83 (29). |
| HRMS(EI) | berechnet für C₁₂H₉BrFN [M⁺]: 264.9902, gefunden: 264.9903. |

### II.11 3',4'-Dichlor-5-fluorbiphenyl-2-amin

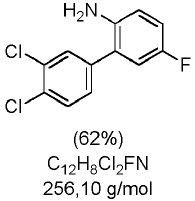

3',4'-Dichlor-5-fluorbiphenyl-2-amin wird aus 3,4-Dichlorphenylhydrazin (1.00 mmol, 177 mg), 4-Fluoranilin (20.0 mmol, 1.92 mL) und MnO₂ (5.00 mmol, 435 mg) analog der allgemeinen Arbeitsvorschrift AAV 2 synthetisiert. Das erhaltene Rohprodukt wird mittels Säulenchromatographie (Hexan / EtOAc = 10:1) gereinigt, wodurch 3',4'-Dichlor-5-fluorbiphenyl-2-amin (0.62 mmol, 158 mg, 62%) erhalten wird.

| | |
|---|---|
| R_{f}-Wert | 0.5 (Hexan / EtOAc = 4:1) [UV] |
| ¹H-NMR | (360 MHz, CDCl₃): δ(ppm) = 3.65 (br s, 2 H), 6.70 (dd, *J*_{HF} = 4.8 Hz, *J* = 8.8 Hz, 1 H), 6.82 (dd, *J* = 2.9 Hz, *J*_{HF} = 9.1 Hz, 1 H), 6.89 (ddd, *J* = 3.0 Hz, *J*_{HF} = 8.1 Hz, *J* = 8.7 Hz, 1 H), 7.29 (dd, *J* = 2.1 Hz, *J* = 8.3 Hz, 1 H), 7.52 (d, *J* = 8.2 Hz, 1 H), 7.55 (d, *J* = 2.1 Hz, 1 H). |
| ¹³C-NMR | (90.6 MHz, CDCl₃): δ(ppm) = 115.8 (d, *J*_{CF} = 22.3 Hz, CH), 116.4 (d, *J*_{CF} = 22.8 Hz, CH), 116.9 (d, *J*_{CF} = 7.7 Hz, CH), 126.0 (d, *J*_{CF} = 7.3 Hz, C_{q}), 128.3 (CH), 130.9 (CH), 131.8 (CH), 133.1 (C_{q}), 138.5 (C_{q}), 138.5 (C_{q}), 139.3 (d, *J*_{CF} = 2.1 Hz, C_{q}), 156.3 (d, *J*_{CF} = 237.4 Hz, C_{q}). |
| ¹⁹F-NMR | (339 MHz, CDCl₃): δ(ppm) = -129.0. |
| MS(EI) | m/z (%):258 (12), 257 (11) [³⁷Cl-M⁺], 256 (82) [M⁺], 255 (19) [³⁵Cl-M⁺], 254 (100), 251 (22), 250 (12), 221 (12), 220 (15), 219 (33), 186 (10), 185 (78), 184 (19), 157 (12), 109 (10), 91 (11), 57 (13), 55 (11). |
| HRMS(EI) | berechnet für C₁₂H₈Cl₂FN [M⁺]: 255.0018, gefunden: 255.0017. |

### II.12 4'-Chlor-5-methoxybiphenyl-2-amin

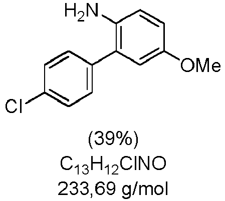

4'-Chlor-5-methoxybiphenyl-2-amin wird aus 4-Chlorphenylhydrazin (1.00 mmol, 143 mg), p-Anisidin (20.0 mmol, 2.46 g) und MnO₂ (5.00 mmol, 435 mg) analog der allgemeinen Arbeitsvorschrift AAV 2 synthetisiert. Das erhaltene Rohprodukt wird mittels Säulenchromatographie (Hexan / EtOAc = 10:1) gereinigt, wodurch 4'-Chlor-5-methoxybiphenyl-2-amin (0.39 mmol, 91.2 mg, 39%) erhalten wird.

| | |
|---|---|
| R_{f}-Wert | 0.4 (Hexan / EtOAc = 4:1) [UV] |
| ¹H-NMR | (360 MHz, CDCl₃): δ(ppm) = 3.21 (br s, 2 H), 3.76 (s, 3 H), 6.69 (d, *J* = 2.8 Hz, 1 H), 6.72 (d, *J* = 8.5 Hz, 1 H), 6.77 (dd, *J* = 2.8 Hz, *J* = 8.7 Hz, 1 H), 7.36-7.44 (m, 4 H). |
| ¹³C-NMR | (90.6 MHz, CDCl₃): δ(ppm) = 55.8 (CH₃), 114.7 (CH), 115.7 (CH), 117.2 (CH), 127.6 (C_{q}), 129.0 (2 x CH), 130.4 (2 x CH), 133.2 (C_{q}), 136.8 (C_{q}), 137.8 (C_{q}), 152.9 (C_{q}). |
| MS (EI) | m/z (%): 239 (8), 237 (11), 235 (29) [³⁷Cl-M⁺], 234 (14), 233 (93) [³⁵Cl-M⁺], 220 (33), 219 (16), 218 (100), 203 (11), 190 (14), 183 (10), 167 (9), 155 (9), 154 (11), 128 (11), 127 (19), 113 (7), 99 (7), 86 (44), 84 (62). |
| HRMS (EI) | berechnet für C₁₃H₁₂ClNO [M⁺]: 233.0608, gefunden: 233.0601. |

### II.13 4'-Chlor-5-methlbihenl-2-amin

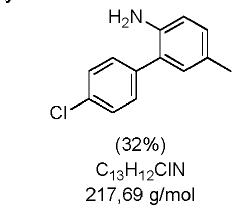

4'-Chlor-5-methylbiphenyl-2-amin wird aus 4-Chlorphenylhydrazin (1.00 mmol, 143 mg), p-Toluidin (20.0 mmol, 2.14 g) und MnO₂ (5.00 mmol, 435 mg) analog der allgemeinen Arbeitsvorschrift AAV 2 synthetisiert. Das erhaltene Rohprodukt wird mittels Säulenchromatographie (Hexan / EtOAc = 10:1) gereinigt, wodurch 4'-Chlor-5-methyl-biphenyl-2-amin (0.32 mmol, 69.2 mg, 32%) erhalten wird.

| | |
|---|---|
| *R*_{f}-Wert | 0.7 (Hexan / EtOAc = 4:1) [UV] |
| ¹H-NMR | (360 MHz, CDCl₃): δ(ppm) = 2.27 (s, 3 H), 3.67 (br s, 2 H), 6.69 (d, *J* = 8.1 Hz, 1 H), 6.89-6.94 (m, 1 H), 6.94-7.01 (m, 1 H), 7.39 (s, 4 H). |
| ¹³C-NMR | (90.6 MHz, CDCl₃): δ(ppm) = 20.4 (CH₃), 116.0 (CH), 126.5 (C_{q}), 128.2 (C_{q}), 128.9 (2 × CH), 129.3 (CH), 130.4 (2 × CH), 130.8 (CH), 133.0 (C_{q}), 138.0 (C_{q}), 140.7 (C_{q}). |
| MS(EI) | *m*/*z* (%): 252 (100), 250 (54), 236 (14), 235 (46), 233 (30), 219 (29) [³⁷Cl-M⁺], 218 (12), 217 (46) [³⁵Cl-M⁺], 216 (18), 203 (13), 180 (11), 83 (13). |
| HRMS(EI) | berechnet für C₁₃H₁₂ClN [M⁺]: 217.0658, gefunden: 217.0658. |

### II.14 4'-Chlor-5-cyanobiphenyl-2-amin

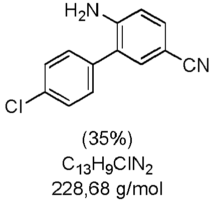

4'-Chlor-5-cyanobiphenyl-2-amin wird aus 4-Chlorphenylhydrazin-hydrochlorid (1.00 mmol, 179 mg), 4-Aminobenzonitril (20.0 mmol, 1.18 g), NaHCO₃ (5.00 mmol, 420 mg) und MnO₂ (5.00 mmol, 435 mg) analog der allgemeinen Arbeitsvorschrift AAV 3 synthetisiert. Das erhaltene Rohprodukt wird mittels Säulenchromatographie (Hexan / EtOAc = 10:1) gereinigt, wodurch 4'-Chlor-5-cyanobiphenyl-2-amin (0.39 mmol, 91.2 mg, 35%) erhalten wird.

| | |
|---|---|
| *R*_{f}-Wert | 0.3 (Hexan / EtOAc = 4:1) [UV] |
| ¹H-NMR | (600 MHz, CDCl₃): δ(ppm) = 4.20 (br s, 2 H), 6.74 (d, *J =* 8.4 Hz, 1 H), 7.34 (d, *J =* 8.3 Hz, 2 H), 7.36 (d, *J =* 2.0 Hz, 1 H), 7.42 (dd, *J =* 2.0 Hz, *J =* 8.4 Hz, 1 H), 7.46 (d, *J =* 8.3 Hz, 2 H). |
| ¹³C-NMR | (151 MHz, CDCl₃): δ(ppm) = 100.8 (C_{q}), 115.2 (CH), 119.8 (C_{q}), 126.1 (C_{q}), 129.5 (2 × CH), 130.2 (2 × CH), 132.9 (CH), 134.3 (C_{q}), 134.3 (CH), 135.5(C_{q}), 147.5(C_{q}). |
| MS(EI) | *m*/*z* (%):230 (35) [³⁷Cl-M⁺], 229 (17), 228 (100) [³⁵Cl-M⁺], 227 (10), 194 (8), 193 (49), 192 (49), 166 (9), 164 (10), 96 (14), 82 (10). |
| HRMS (EI) | berechnet für C₁₃H₉ClN₂ [M⁺]: 228.0454, gefunden: 228.0455. |

### II.15 5-Fluor-4'-methoxybiphenyl-2-amin

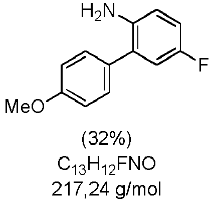

5-Fluor-4'-methoxybiphenyl-2-amin wird aus 4-Methoxyphenylhydrazin-hydrochlorid (1.00 mmol, 175 mg), 4-Fluoranilin (20.0 mmol, 1.92 mL), NaHCO₃ (5.00 mmol, 420 mg) und MnO₂ (5.00 mmol, 435 mg) analog der allgemeinen Arbeitsvorschrift AAV 3 synthetisiert. Das erhaltene Rohprodukt wird mittels Säulenchromatographie (Hexan / EtOAc = 10:1) gereinigt, wodurch 5-Fluor-4'-methoxybiphenyl-2-amin (0.32 mmol, 69.2 mg, 32%) erhalten wird.

| | |
|---|---|
| R_{f}-Wert | 0.3 (Hexan / EtOAc = 4:1) [UV] |
| ¹H-NMR | (360 MHz, CDCl₃): δ(ppm) = 3.85 (br s, 3 H), 6.68 (dd, *J*_{HF} = 4.9 Hz, *J* = 9.2 Hz, 1 H), 6.80-6.88 (m, 2 H), 6.98 (d, *J* = 8.8 Hz, 1 H), 7.36 (d, *J* = 8.9 Hz, 1 H). |
| ¹³C-NMR | (151 MHz, CDCl₃): δ(ppm) = 55.3 (CH₃), 114.3 (2 × CH), 114.5 (d, *J*_{CF} = 22.2 Hz, CH), 116.4 (d, *J*_{CF} = 7.8 Hz, CH), 116.7 (d, *J*_{CF} = 22.2 Hz, CH), 128.5 (d, *J*_{CF} = 7.3 Hz, C_{q}), 130.0 (2 × CH), 130.8 (C_{q}), 139.6 (d, *J*_{CF} = 2.3 Hz, C_{q}), 156.4 (d, *J*_{CF} = 236.2 Hz, C_{q}), 159.1 (C_{q}). |
| ¹⁹F-NMR | (339 MHz, CDCl₃): δ(ppm) = -129.6. |
| MS(EI) | m/z (%): 218 (15), 217 (100) [M⁺], 216 (10), 202 (28), 186 (8), 185 (8), 174 (10), 172 (10), 147 (12), 146 (10). |
| HRMS(EI) | berechnet für C₁₃H₁₂FNO [M⁺]: 217.0903, gefunden: 217.0902. |

### II.16 5-Fluorbiphenyl-2-amin

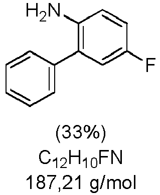

5-Fluorbiphenyl-2-amin wird aus Phenylhydrazin (1.00 mmol, 99.0 µL), 4-Fluoranilin (20.0 mmol, 1.92 mL) und MnO₂ (5.00 mmol, 435 mg) analog der allgemeinen Arbeitsvorschrift AAV 2 synthetisiert. Das erhaltene Rohprodukt wird mittels Säulenchromatographie (Hexan / EtOAc = 10:1) gereinigt, wodurch 5-Fluorbiphenyl-2-amin (0.33 mmol, 62.2 mg, 33%) erhalten wird.

| | |
|---|---|
| R_{f}-Wert | 0.3 (Hexan / EtOAc = 4:1) [UV] |
| ¹H-NMR | (360 MHz, CDCl₃): δ(ppm) = 3.85 (br s, 2 H), 6.72 (dd, *J* = 4.9 Hz, *J* = 9.1 Hz, 1 H), 6.85-6.90 (m, 2 H), 7.33-7.38 (m, 1 H), 7.42-7.46 (m, 4 H). |
| ¹³C-NMR | (90.6 MHz, CDCl₃): δ(ppm) = 114.8 (d, *J*_{CF} =22.2 Hz, CH), 116.4 (d, *J*_{CF} = 7.7 Hz, CH), 116.6 (d, *J*_{CF} =22.5 Hz, CH), 127.6 (s, CH), 128.7 (d, *J*_{CF} = 7.0 Hz, C_{q}), 128.9 (4xCH), 138.6 (d, *J*_{CF} *=* 1.7 Hz, C_{q}), 139.6 (d, *J*_{CF} *=* 2.3 Hz, C_{q}), 156.3 (d, *J*_{CF} =235.7 Hz, C_{q}). |
| ¹⁹F-NMR | (339 MHz, CDCl₃): δ(ppm) = -129.7. |
| MS(EI) | m/z (%): 188 (13), 187 (100) [M⁺], 186 (73), 185 (31), 184 (7), 166 (3), 157 (4), 133 (4), 93 (6), 92 (5). |
| HRMS(EI) | berechnet für C₁₂H₁₀FN [M⁺]: 187.0797, gefunden: 187.0797. |

### II.17 5-Fluor-4'-methylbiphenyl-2-amin

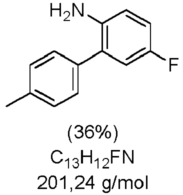

5-Fluor-4'-methylbiphenyl-2-amin wird aus 4-Methylphenylhydrazin (1.00 mmol, 122 mg), 4-Fluoranilin (20.0 mmol, 1.92 mL) und MnO₂ (5.00 mmol, 435 mg) analog der allgemeinen Arbeitsvorschrift AAV 2 synthetisiert. Das erhaltene Rohprodukt wird mittels Säulenchromatographie (Hexan / EtOAc = 10:1) gereinigt, wodurch 5-Fluor-4'-methylbiphenyl-2-amin (0.36 mmol, 72.2 mg, 36%) erhalten wird.

| | |
|---|---|
| *R*_{f}-Wert | 0.5 (Hexan / EtOAc = 4:1) [UV] |
| ¹H-NMR | (600 MHz, CDCl₃): δ(ppm) = 2.40 (s, 3 H), 3.65 (br s, 2 H), 6.68 (dd, *J =* 9.2 Hz, *J*_{HF} *=* 4.8 Hz, 1 H), 6.82-6.88 (m, 2 H), 7.26 (d, *J =* 7.8 Hz, 2 H), 7.32 (d, *J =* 8.0 Hz, 2 H). |
| ¹³C-NMR | (90.6 MHz, CDCl₃): δ(ppm) = 21.2 (CH₃), 114.6 (d, *J*_{CF} =22.2 Hz, CH), 116.4 (d, *J*_{CF} *=* 7.7 Hz, CH), 116.6 (d, *J*_{CF} =22.3 Hz, CH), 128.7 (2 × CH), 129.6 (2 × CH), 135.6 (C_{q}), 136.0 (C_{q}), 137.4 (C_{q}), 139.5 (d, *J*_{CF} *=* 2.1 Hz, C_{q}), 156.4 (d, *J*_{CF} =236.4 Hz, C_{q}). |
| MS (EI) | *m*/*z* (%): 272 (12), 270 (43), 268 (21), 252 (11), 235 (25), 233 (13), 202 (28) [M⁺+H], 201 (100) [M⁺], 200 (67), 199 (10), 198 (16), 186 (35), 185 (52), 99 (11), 85 (18), 83 (26), 44 (12). |
| HRMS (EI) | berechnet für C₁₃H₁₂FN [M⁺]: 201.0954, gefunden: 201.0955. |

### II.18 4'-Fluorbiphenyl-2-amin

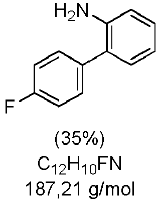

4'-Fluorbiphenyl-2-amin wird aus 4-Fluorphenylhydrazin (1.00 mmol, 126 mg), Anilin (20.0 mmol, 1.82 mL) und MnO₂ (5.00 mmol, 435 mg) analog der allgemeinen Arbeitsvorschrift AAV 2 synthetisiert. Das erhaltene Rohprodukt wird mittels Säulenchromatographie (Hexan / EtOAc = 10:1 → 4:1) gereinigt, wodurch 4'-Fluorbiphenyl-2-amin (0.35 mmol, 65.2 mg, 35%) erhalten wird.

| | |
|---|---|
| *R*_{f}-Wert | 0.5 (Hexan / EtOAc = 4:1) [UV] |
| ¹H-NMR | (360 MHz, CDCl₃): δ(ppm) = 3.78 (br s, 2 H), 6.77 (dd, *J =* 1.0 Hz, *J =* 8.0 Hz, 1 H), 6.82 (dt, *J =* 1.2 Hz, *J =* 7.5 Hz, 1 H), 7.06-7.19 (m, 4 H), 7.41 (dd, *J =* 5.4 Hz, *J =* 8.9 Hz, 2 H). |
| ¹³C-NMR | (90.6 MHz, CDCl₃): δ(ppm) = 115.7 (d, *J*_{CF} =20.9 Hz, 2 × CH), 118.9 (CH), 126.8 (C_{q}), 128.6 (CH), 130.4 (CH), 130.5 (CH), 130.8 (d, *J*_{CF} =8.0 Hz, 2 × CH), 135.3 (d, *J*_{CF} =3.3 Hz, C_{q}), 143.2 (C_{q}), 162.1 (d, *J*_{CF} =246.3 Hz, C_{q}). |
| MS(EI) | *m*/*z* (%): 272 (22), 270 (77), 268 (44), 235 (45), 233 (28), 219 (16), 217 (11), 203 (13), 187 (43) [M⁺], 186 (20), 185 (12), 85 (55), 83 (100), 49 (11), 48 (12), 47 (23), 44 (65). |
| HRMS(EI) | berechnet für C₁₂H₁₀FN [M⁺]: 187.0797, gefunden: 187.0797. |

### II.19 Biphenyl-2-amin

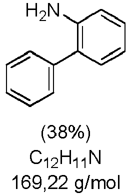

Biphenyl-2-amin wird aus Phenylhydrazin (1.00 mmol, 99 µl), Anilin (20.0 mmol, 1.82 mL) und MnO₂ (5.00 mmol, 435 mg) analog der allgemeinen Arbeitsvorschrift AAV 2 synthetisiert. Das erhaltene Rohprodukt wird mittels Säulenchromatographie (Hexan / EtOAc = 10:1 → 4:1) gereinigt und es wird Biphenyl-2-amin (0.38 mmol, 64.2 mg, 38%) erhalten.

| | |
|---|---|
| *R*_{f}-Wert | 0.6 (Hexan / EtOAc = 4:1) [UV] |
| ¹H-NMR | (600 MHz, CDCl₃): δ(ppm) = 3.75 (br s, 2 H), 6.76-6.78 (m, 1 H), 6.83 (dt, *J =* 1.2 Hz, *J =* 7.5 Hz, 1 H), 7.11-7.18 (m, 2 H), 7.32-7.36 (m, 1 H), 7.45 (m, 4 H). |

### III. Wirkstoffsynthese

### III.1 2-Chlor-N-(4'-chlorbiphenyl-2-yl)-nicotinamid (Boscalid®)

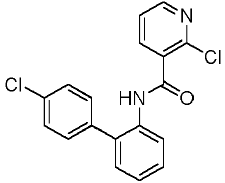

Zu einer Lösung von 4'-Chlorbiphenyl-2-amin (0.28 mmol, 58 mg) und Triethylamin (1.40 mmol, 0.20 ml) in Dichlormethan (4.4 ml) wurde langsam bei 0°C eine Lösung von 2-Chlornicotinsäurechlorid (0.41 mmol, 72 mg) in Methylenchlorid (0.9 ml) gegeben. Man ließ das Gemisch 3 h auf Raumtemperatur auftauen, rührte für eine weitere Stunde und erwärmte anschließend 2 h zum Rückfluss. Die organische Phase wurde mit Wasser und gesättigter Kochsalzlösung gewaschen und über Natriumsulfat getrocknet. Nach dem Einengen im Vakuum wurde das Rohprodukt mittels Säulenchromatographie (Kieselgel, Hexan / EtOAc = 3:1) gereinigt und man erhielt 2-Chlor-*N-*(4'-chlorbiphenyl-2-yl)-nicotinamid (0.25 mmol; 85 mg; 87%).

| | |
|---|---|
| *R*_{f}-Wert | 0.4 (Hexan / EtOAc = 3:2) [UV]. |
| ¹H-NMR | (600 MHz, CDCl₃): δ = 7.26-7.28 (m, 2 H), 7.34 (d, *J* = 8.4 Hz, 2 H), 7.35 (m, 1 H), 7.43 (d, *J* = 8.5 Hz, 2 H), 7.45-7.48 (m, 1 H), 8.13 (dd, *J* = 1.9 Hz, *J* = 7.7 Hz, 1 H), 8.14-8.17 (m, 1 H), 8.41 (d, *J* = 8.2 Hz, 1 H), 8.44 (dd, *J* = 1.9 Hz, *J* = 4.7 Hz, 1 H). |

### III.2 3-Difluormethyl-1-methyl-1H-pyrazol-4-carbonsäure-(3',4'-dichlor-5-fluor-biphenyl-2-yl)-amid (Bixafen®)

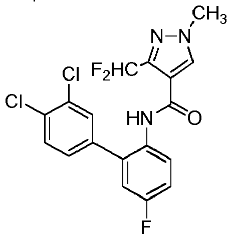

Eine Lösung von 3',4'-Dichlor-5-fluorbiphenyl-2-amin (0.21 mmol, 53 mg) und 3-Difluormethyl-1-methyl-1*H-*pyrazol-4-carbonsäurechlorid (0.25 mmol, 48 mg) in THF (1 mL) wurde mit Triethylamin (0.41 mmol, 0.06 mL) behandelt. Das Gemisch wurde 16 h auf 60 °C erwärmt. Nach dem Einengen im Vakuum wurde das Rohprodukt mittels Säulenchromatographie (Kieselgel, Hexan / EtOAc = 3:2) gereinigt und man erhielt 3-Difluormethyl-1-methyl-1H-pyrazol-4-carbonsäure-(3',4'-dichlor-5-fluor-biphenyl-2-yl)-amid (0.18 mmol, 73 mg, 85%).

| | |
|---|---|
| *R*_{f}-Wert | 0.1 (Hexan / EtOAc = 3:2) [UV]. |
| ¹H-NMR | (600 MHz, CDCl₃): δ = 3.91 (s, 3 H), 6.67 (t, *J*_{HF} =54.2 Hz, 1 H), 6.97 (dd, *J =* 2.9 Hz, *J*_{HF} *=* 8.7 Hz, 1 H), 7.12 (ddd, *J =* 3.0 Hz, *J*_{HF} *=* 8.0 Hz, *J =* 9.0 Hz, 1 H), 7.20 (dd, *J =* 2.1 Hz, *J*= 8.2 Hz, 1 H), 7.47 (d, *J =* 2.0 Hz, 1 H), 7.50 (d, *J =* 8.2 Hz, 1 H), 7.72 (s, 1 H), 7.90 (s, 1 H), 8.09 (dd, *J*_{HF} *=*5.3 Hz, *J* = 9.0 Hz, 1 H). |
| ¹³C-NMR | (90,6 MHz, CDCl₃): δ = 39.5 (CH₃), 111.4 (t, *J*_{CF} =233.3, CH), 115.6 (d, *J*_{CF} =22.0 Hz, CH), 116.4 (C_{q}), 116.7 (d, *J*_{CF} =23.1 Hz, CH), 125.6 (d, *J*_{CF} = 8.0 Hz, C_{q}), 128.4 (CH), 130.5 (d, *J*_{CF} =3.0 Hz, C_{q}), 130.9 (CH), 131.0 (CH), 132.6 (C_{q}), 133.1 (C_{q}), 133.9 (d, *J*_{CF} *=* 7.9 Hz, C_{q}), 135.8 (C_{q}), 137.1 (d, *J*_{CF} *=* 1.6 Hz, C_{q}), 142.5 (t, *J*_{CF} *=* 29.0 Hz, C_{q}), 159.5 (C_{q}), 159.6 (d, *J*_{CF} = 247.4 Hz, C_{q}). |
| ¹⁹F-NMR | (339 MHz, CDCl₃): δ = -112.1, -119.7. |
| MS (EI) | *m*/*z* (%): 417 (5) [³⁷Cl₂-M⁺], 416 (6), 415 (26) [³⁷Cl-³⁵Cl-M⁺], 414 (9), 413 (43) [³⁵Cl₂-M⁺], 219 (6), 184 (6), 160 (28), 159 (100), 139 (8), 137 (6), 83 (8), 43 (12). |
| HRMS (EI) | berechnet für C₁₈H₁₂Cl₂F₃N₃O [M⁺]: 413.0310, gefunden: 413.0309. |

### III.3 3-Difluormethyl-1-methyl-1H-pyrazol-4-carbonsäure-(3',4',5'-trifluorbiphenyl-2-yl)-amid (Xemium®)

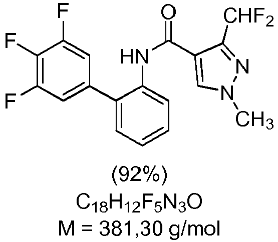

3',4',5'-Trifluorbiphenyl-2-amin (0.45 mmol, 100 mg) und Pyridin (0.81 mmol, 65 µL) werden in 1 mL Toluol gelöst und die Lösung wird auf 55 °C erwärmt. Es wird eine Lösung aus 3-Difluormethyl-1-methyl-1H-pyrazol-4-carbonyl chlorid (0.45 mmol, 87.9 mg) in 300 µL Toluol über 10 Minuten zugetropft und die Reaktionsmischung weiter bei 55 °C gerührt. Nach beendeter Reaktion wird die Lösung auf 70 °C erwärmt und es wird mit 2n HCl, gesättigter Natriumhydrogencarbonatlösung und Wasser gewaschen. Nachdem die Lösung abgekühlt ist wird das Lösemittel bei vermindertem Druck entfernt und das erhaltene Rohprodukt wird mittels Säulenchromatographie an Kieselgel (Hexan / EtOAc = 2:1) gereinigt, wodurch 3-Difluormethyl-1-methyl-1H-pyrazol-4-carbonsäure-(3',4',5'-trifluorbiphenyl-2-yl)-amid (0.41 mmol, 158 mg, 92%) als weißer Feststoff erhalten wird.

| | |
|---|---|
| *R*_{f}-Wert | 0.4 (Hexan / EtOAc = 1:1) [UV] |
| ¹H-NMR | (360 MHz, CDCl₃): δ(ppm) = 3.92 (s, 3 H), 6.65 (t, *J =* 54.2 Hz, 1 H), 6.92-7.06 (m, 2 H), 7.19-7.25 (m, 2 H), 7.38-7.47 (m, 1 H), 7.81 (br s, 1 H), 7.95 (s, 1 H), 8.19 (d, *J =* 8.2 Hz, 1 H). |
| ¹³C-NMR | (151 MHz, CDCl₃): δ(ppm) = 39.5 (CH₃), 111.6 (t, *J*_{CF} =232.9 Hz, CH), 113.7 (dd, *J*_{CF} =4.7 Hz, *J*_{CF} *=* 16.6 Hz, 2 × CH), 116.5 (C_{q}), 123.4 (CH), 125.2 (CH), 129.2 (CH), 130.0 (CH), 131.2 (C_{q}), 134.1 (dt, *J*_{CF} =4.9 Hz, *J*_{CF} =7.9 Hz, C_{q}), 134.5 (CH), 136.1 (C_{q}), 139.5 (td, *J*_{CF} =15.3 Hz, *J*_{CF} =252.5 Hz, C_{q}), 142.3 (t, *J*_{CF} =29.3 Hz, C_{q}), 151.2 (ddd, *J*_{CF} =4.3 Hz, *J*_{CF} =10.0 Hz, *J*_{CF} =251.2 Hz, 2 × C_{q}), 159.4 (C_{q}). |
| ¹⁹F-NMR: | (339 MHz, CDCl₃): δ(ppm) = -111.8, -136.9, -164.7. |
| MS(EI) | *m*/*z* (%): 382 (20), 381 (96) [M⁺], 222 (5), 221 (11), 160 (35), 159 (100), 139 (10), 83 (5), 44(4), 43 (14). |
| HSMS(EI) | berechnet für C₁₈H₁₂F₅N₃O [M⁺]: 381.0901, gefunden: 381.0901. |

## Patentansprüche

1. Verfahren zur Herstellung einer Verbindung der Formel 3 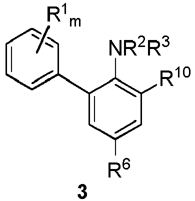 wobei eine Verbindung der Formel 1 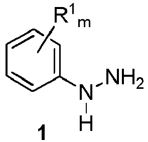 mit einer Verbindung der Formel 2 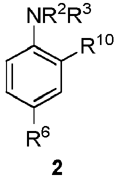 umgesetzt wird, wobei
m für 0, 1, 2, 3, 4 oder 5 steht;
jedes R¹ jeweils unabhängig für Halogen, Alkyl, Haloalkyl, Hydroxy, Hydroxyalkyl, Alkoxy, Haloalkoxy, Alkylthio, Cycloalkyl, Haloalkylthio, Alkenyl, Alkinyl, Amino, Nitro, Cyano, -SO₃R⁵, -SO₂NH₂, -SO₂NHR⁴,-SO₂NR⁴R⁵, -COOR⁴, -CONHR⁴, -CONR⁴R⁵,-COR⁴, -OCOR⁴,-NR⁴R⁵,-NR⁴COR⁵, -NR⁴SO₂R⁵, Alkylcarbonyl, Haloalkylcarbonyl, Alkenylcarbonyl, Alkoxycarbonyl, Haloalkoxycarbonyl, Alkenyloxycarbonyl, Alkylsulfonyl, Haloalkylsulfonyl, Alkylimino, Aryl, Aryloxy, Arylcarbonyl, Arylalkyl, Heteroarylalkyl, Arylalkoxycarbonyl, Arylalkylimino oder Heteroaryl steht;
R² und R³ für Wasserstoff stehen,
n jeweils unabhängig für 1, 2, 3, 4, 5, 6, 7, 8, 9 oder 10 steht;
R⁴ jeweils unabhängig für Wasserstoff, Alkyl, Cycloalkyl, Haloalkyl, Arylalkyl, Heteroarylalkyl, Aryl oder Heteroaryl steht;
R⁵ jeweils unabhängig für Wasserstoff, Alkyl, Cycloalkyl, Haloalkyl, Arylalkyl, Heteroarylalkyl, Aryl oder Heteroaryl steht;
R⁶ jeweils unabhängig für Wasserstoff, Halogen, Alkyl, Alkenyl, Alkinyl, Cycloalkyl, Arylalkyl, Heteroarylalkyl, Haloalkyl, Hydroxyalkyl, Alkoxyalkyl, Aryloxyalkyl, Heteroaryloxyalkyl, Aminoalkyl, -(CH₂)ₙ-NR⁴R⁵,-COOH, -CHO, -CN, -COR⁴, Alkylcarbonyl, Haloalkylcarbonyl, Cycloalkylcarbonyl, Arylalkylcarbonyl, Alkenylcarbonyl, Arylcarbonyl, Heteroarylcarbonyl, -COOR⁴, Alkoxycarbonyl, Haloalkoxycarbonyl, Cycloalkoxycarbonyl, Arylalkoxycarbonyl, Alkenyloxycarbonyl, Aryloxycarbonyl, Heteroaryloxycarbonyl, -CONHR⁴, -CONR⁴R⁵, Amino, Nitro, -NHR⁴, -NR⁴R⁵, 1-Pyrrolidino, 1-Piperidino, 1-Morpholino, Alkylimino, Cycloalkylimino, Haloalkylimino, Arylalkylimino, -NR⁴COR⁵, NR⁴COOR⁵,-NR⁴SO₂R⁵, Hydroxy, Alkoxy, Haloalkoxy, Cycloalkoxy, Arylalkyloxy, Aryloxy, Heteroaryloxy, -OCOR⁴, Alkylcarbonyloxy, Haloalkylcarbonyloxy, Cycloalkylcarbonyloxy, Arylalkylcarbonyloxy, Arylcarbonyloxy, Heteroarylcarbonyloxy, -OCONR⁴R⁵, -O-(CH₂)ₙ-OR⁴, -O-(CH₂)ₙ-NR⁴R⁵, -O-(CH₂)ₙ-NR⁴COR⁵, -O-(CH₂)ₙ-NR⁴COOR⁵, -O-(CH₂)ₙ-COOR⁴, -O-(CH₂)ₙ-CONHR⁴, -O-(CH₂)ₙ-CONR⁴R⁵, -O-(CH₂)ₙ-SO₃R⁴, -O-(CH₂)ₙ-SO₂R⁴, -O-(CH₂)ₙ-CN, -SH, Alkylthio, Haloalkylthio, Cycloalkylthio, Arylalkylthio, Arylthio, Heteroarylthio, Alkylsulfonyl, Haloalkylsulfonyl, Cycloalkylsulfonyl, Arylalkylsulfonyl, Arylsulfonyl, Heteroarylsulfonyl, -SO₂NH₂, -SO₂NHR⁴, -SO₂NR⁴R⁵, -SO₃R⁵, Aryl oder Heteroaryl steht;
R¹⁰ jeweils unabhängig für Wasserstoff, Halogen, Alkyl, Haloalkyl, Hydroxyalkyl, Cycloalkyl, Arylalkyl, Heteroarylalkyl, -(CH₂)_{q}-NR⁴R⁵, -(CH₂)_{q}-NR⁴COR⁵, -(CH₂)_{q}-NR⁴COOR⁵, -(CH₂)_{q}-COOR⁴, -(CH₂)_{q}-CONHR⁴,-(CH₂)_{q}-CONR⁴R⁵, -(CH₂)_{q}-SO₃R⁴, -(CH₂)_{q}-CN, Aryl oder Heteroaryl steht;
q jeweils unabhängig für 1, 2, 3, 4, oder 5 steht;
wobei der Alkylrest in Alkoxy, Alkycarbonyl, Alkycarbonyloxy, Alkoxycarbonyl, Alkylsulfonyl, Hydroxyalkyl, Alkoxyalkyl, Aryloxyalkyl, Heteroaryloxyalkyl, Aminoalkyl, Arylalkylcarbonyl, Arylalkoxycarbonyl, Arylalkyloxy, Arylalkyl, Heteroarylalkyl, Arylalkylcarbonyloxy, Alkylthio, Arylalkylthio und Arylalkylsulfonyl gegebenenfalls 1, 2 oder 3 Substituenten trägt, die ausgewählt sind unter Halogen, Alkyl, Haloalkyl, Cycloalkyl, Alkoxy und Haloalkoxy;
wobei Cycloalkyl gegebenenfalls 1, 2 oder 3 Substituenten trägt, die ausgewählt sind unter Alkyl, Alkoxy und Halogen;
wobei der Cycloalkylrest in Cycloalkoxy und Cycloalkylthio gegebenenfalls 1, 2 oder 3 Substituenten trägt, die ausgewählt sind unter Alkyl und Halogen;
wobei der Cycloalkylrest in Cycloalkylcarbonyl, Cycloalkoxycarbonyl, Cycloalkylcarbonyloxy und Cycloalkylsulfonyl gegebenenfalls 1, 2 oder 3 Substituenten trägt, die ausgewählt sind unter Halogen, Alkyl, Haloalkyl, Cycloalkyl, Alkoxy und Haloalkoxy;
wobei der Alkenylrest in Alkenylcarbonyl und Alkenyloxycarbonyl gegebenenfalls 1, 2 oder 3 Substituenten trägt, die ausgewählt sind unter Halogen, Alkyl, Haloalkyl, Cycloalkyl, Alkoxy und Haloalkoxy;
wobei der Cycloalkylidenrest in Cycloalkylimino gegebenenfalls 1, 2 oder 3 Substituenten trägt, die ausgewählt sind unter Halogen, Alkyl, Haloalkyl, Cycloalkyl, Alkoxy und Haloalkoxy; und
wobei der Alkylenrest in Alkylimino gegebenenfalls 1, 2 oder 3 Substituenten trägt, die ausgewählt sind unter Halogen, Alkyl, Haloalkyl, Cycloalkyl, Alkoxy und Haloalkoxy;
**dadurch gekennzeichnet, dass** die Umsetzung in Gegenwart mindestens eines Oxidationsmittels durchgeführt wird, wobei das Oxidationsmittel Sauerstoff, Luftsauerstoff, MnO₂, Mn(OAc)₃, KMnO₄, NalO₄, K₃[Fe(CN)₆], KO₂ oder H₂O₂ ist.

2. Verfahren nach Anspruch 1, **dadurch gekennzeichnet, dass** die Umsetzung in Gegenwart von mindestens einem Lösungsmittel durchgeführt wird.

3. Verfahren nach einem der voranstehenden Ansprüche, **dadurch gekennzeichnet, dass** die Umsetzung in Gegenwart von mindestens einer Base, mindestens einem Lösungsmittel und mindestens einem Oxidationsmittel durchgeführt wird.

4. Verfahren nach einem der voranstehenden Ansprüche, **dadurch gekennzeichnet, dass** das Oxidationsmittel Sauerstoff oder Luftsauerstoff ist.

5. Verfahren nach einem der Ansprüche 1 bis 3, **dadurch gekennzeichnet, dass** das Oxidationsmittel MnO₂, Mn(OAc)₃, KMnO₄, NaIO₄, K₃[Fe(CN)₆], KO₂ oder H₂O₂ ist.

6. Verfahren nach einem der voranstehenden Ansprüche, **dadurch gekennzeichnet, dass** R¹ für Halogen, C₁-C₄-Alkyl oder C₁-C₄-Alkoxy steht.

7. Verfahren nach einem der voranstehenden Ansprüche, **dadurch gekennzeichnet, dass** R¹⁰ für Wasserstoff steht.

8. Verfahren nach einem der voranstehenden Ansprüche **dadurch gekennzeichnet, dass** R⁶ für Wasserstoff, Halogen, CN, C₁-C₄-Alkyl oder C₁-C₄-Alkoxy steht.

9. Verfahren nach einem der voranstehenden Ansprüche **dadurch gekennzeichnet, dass** m für 0, 1, 2, oder 3 steht.

10. Verfahren nach einem der voranstehenden Ansprüche, **dadurch gekennzeichnet, dass** R¹ Fluor, Chlor, Brom oder Methoxy ist, R², R³ und R¹⁰ Wasserstoffatome sind, R⁶ Wasserstoff, Fluor, Chlor, Brom, CN, Methoxy oder Ethoxy ist und m gleich 0, 1, 2 oder 3 ist.

11. Verfahren nach einem der voranstehenden Ansprüche, **dadurch gekennzeichnet, dass** die Umsetzung im Temperaturbereich von -20 bis 100°C durchgeführt wird.

12. Verfahren nach Anspruch 11, **dadurch gekennzeichnet, dass** die Umsetzung im Temperaturbereich von -10 °C bis 70°C durchgeführt wird.

13. Verfahren nach einem der voranstehenden Ansprüche, **dadurch gekennzeichnet, dass** die Umsetzung unter Bestrahlung oder Ultraschall durchgeführt wird.

14. Verfahren nach einem der Ansprüche 1 bis 3 und 5 bis 13, **dadurch gekennzeichnet, dass** die Umsetzung in Gegenwart mindestens eines Oxidationsmittels unter Schutzgas durchgeführt wird.

## Claims

1. A process for preparing a compound of the formula 3 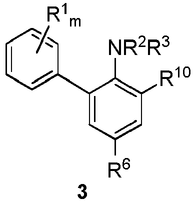 by reacting a compound of the formula 1 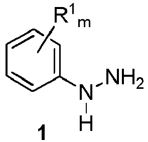 with a compound of the formula 2 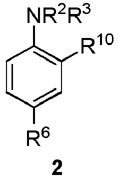 where
m is 0, 1, 2, 3, 4 or 5;
each R¹ is independently halogen, alkyl, haloalkyl, hydroxyl, hydroxyalkyl, alkoxy, haloalkoxy, alkylthio, cycloalkyl, haloalkylthio, alkenyl, alkynyl, amino, nitro, cyano,-SO₃R⁵, -SO₂NH₂, -SO₂NHR⁴, -SO₂NR⁴R⁵, -COOR⁴,-CONHR⁴, -CONR⁴R⁵, -COR⁴, -OCOR⁴, -NR⁴R⁵,-NR⁴COR⁵, -NR⁴SO₂R⁵, alkylcarbonyl, haloalkylcarbonyl, alkenylcarbonyl, alkoxycarbonyl, haloalkoxycarbonyl, alkenyloxycarbonyl, alkylsulfonyl, haloalkylsulfonyl, alkylimino, aryl, aryloxy, arylcarbonyl, arylalkyl, heteroarylalkyl, arylalkoxycarbonyl, arylalkylimino or heteroaryl;
R² and R³ are hydrogen,
n at each instance is independently 1, 2, 3, 4, 5, 6, 7, 8, 9 or 10;
R⁴ at each instance is independently hydrogen, alkyl, cycloalkyl, haloalkyl, arylalkyl, heteroarylalkyl, aryl or heteroaryl;
R⁵ at each instance is independently hydrogen, alkyl, cycloalkyl, haloalkyl, arylalkyl, heteroarylalkyl, aryl or heteroaryl;
R⁶ at each instance is independently hydrogen, halogen, alkyl, alkenyl, alkynyl, cycloalkyl, arylalkyl, heteroarylalkyl, haloalkyl, hydroxyalkyl, alkoxyalkyl, aryloxyalkyl, heteroaryloxyalkyl, aminoalkyl, -(CH₂)ₙ-NR⁴R⁵, -COOH, -CHO, -CN, -COR⁴, alkylcarbonyl, haloalkylcarbonyl, cycloalkylcarbonyl, arylalkylcarbonyl, alkenylcarbonyl, arylcarbonyl, heteroarylcarbonyl, -COOR⁴, alkoxycarbonyl, haloalkoxycarbonyl, cycloalkoxycarbonyl, arylalkoxycarbonyl, alkenyloxycarbonyl, aryloxycarbonyl, heteroaryloxycarbonyl,-CONHR⁴, -CONR⁴R⁵, amino, nitro, -NHR⁴, -NR⁴R⁵, 1-pyrrolidino, 1-piperidino, 1-morpholino, alkylimino, cycloalkylimino, haloalkylimino, arylalkylimino, -NR⁴COR⁵, NR⁴COOR⁵, -NR⁴SO₂R⁵, hydroxyl, alkoxy, haloalkoxy, cycloalkoxy, arylalkyloxy, aryloxy, heteroaryloxy, -OCOR⁴, alkylcarbonyloxy, haloalkylcarbonyloxy, cycloalkylcarbonyloxy, arylalkylcarbonyloxy, arylcarbonyloxy, heteroarylcarbonyloxy, -OCONR⁴R⁵, -O-(CH₂)ₙ-OR⁴, -O-(CH₂)ₙ-NR⁴R⁵, -O-(CH₂)-NR⁴COR⁵, -O-(CH₂)ₙ-NR⁴COOR⁵, -O-(CH₂)ₙ-COOR⁴, -O-(CH₂)ₙ-CONHR⁴, -O-(CH₂)-CONR⁴R⁵, -O-(CH₂)ₙ-SO₃R⁴,-O-(CH₂)ₙ-SO₂R⁴, -O-(CH₂)ₙ-CN, -SH, alkylthio, haloalkylthio, cycloalkylthio, arylalkylthio, arylthio, heteroarylthio, alkylsulfonyl, haloalkylsulfonyl, cycloalkylsulfonyl, arylalkylsulfonyl, arylsulfonyl, heteroarylsulfonyl, -SO₂NH₂,-SO₂NHR⁴, -SO₂NR⁴R⁵, -SO₃R⁵, aryl or heteroaryl;
R¹⁰ at each instance is independently hydrogen, halogen, alkyl, haloalkyl, hydroxyalkyl, cycloalkyl, arylalkyl, heteroarylalkyl,-(CH₂)_{q}-NR⁴R⁵, -(CH₂)_{q}-NR⁴COR⁵, -(CH₂)_{q}-NR⁴COOR⁵, -(CH₂)_{q}-COOR⁴, -(CH₂)_{q}-CONHR⁴,-(CH₂)_{q}-CONR⁴R⁵, -(CH₂)q-SO₃R⁴, -(CH₂)_{q}-CN, aryl or heteroaryl;
q at each instance is independently 1, 2, 3, 4, or 5;
where the alkyl radical in alkoxy, alkylcarbonyl, alkylcarbonyloxy, alkoxycarbonyl, alkylsulfonyl, hydroxyalkyl, alkoxyalkyl, aryloxyalkyl, heteroaryloxyalkyl, aminoalkyl, arylalkylcarbonyl, arylalkoxycarbonyl, arylalkyloxy, arylalkyl, heteroarylalkyl, arylalkylcarbonyloxy, alkylthio, arylalkylthio and arylalkylsulfonyl optionally bears 1, 2 or 3 substituents selected from halogen, alkyl, haloalkyl, cycloalkyl, alkoxy and haloalkoxy;
where cycloalkyl optionally bears 1, 2 or 3 substituents selected from alkyl, alkoxy and halogen;
where the cycloalkyl in cycloalkoxy and cycloalkylthio optionally bears 1, 2 or 3 substituents selected from alkyl and halogen;
where the cycloalkyl radical in cycloalkylcarbonyl, cycloalkoxycarbonyl, cycloalkylcarbonyloxy and cycloalkylsulfonyl optionally bears 1, 2 or 3 substituents selected from halogen, alkyl, haloalkyl, cycloalkyl, alkoxy and haloalkoxy;
where the alkenyl radical in alkenylcarbonyl and alkenyloxycarbonyl optionally bears 1, 2 or 3 substituents selected from halogen, alkyl, haloalkyl, cycloalkyl, alkoxy and haloalkoxy;
where the cycloalkylidene radical in cycloalkylimino optionally bears 1, 2 or 3 substituents selected from halogen, alkyl, haloalkyl, cycloalkyl, alkoxy and haloalkoxy;
and where the alkylene radical in alkylimino optionally bears 1, 2 or 3 substituents selected from halogen, alkyl, haloalkyl, cycloalkyl, alkoxy and haloalkoxy;
which comprises performing the reaction in the presence of at least one oxidizing agent, where the oxidizing agent is oxygen, atmospheric oxygen, MnO₂, Mn(OAc)₃, KMnO₄, NaIO₄, K₃[Fe(CN)₆], KO₂ or H₂O₂.

2. The process according to claim 1, wherein the reaction is performed in the presence of at least one solvent.

3. The process according to either of the preceding claims, wherein the reaction is performed in the presence of at least one base, at least one solvent and at least one oxidizing agent.

4. The process according to any of the preceding claims, wherein the oxidizing agent is oxygen or atmospheric oxygen.

5. The process according to any of claims 1 to 3, wherein the oxidizing agent is MnO₂, Mn(OAc)₃, KMnO₄, NaIO₄, K₃[Fe(CN)₆], KO₂ or H₂O₂.

6. The process according to any of the preceding claims, wherein R¹ is halogen, C₁-C₄-alkyl or C₁-C₄-alkoxy.

7. The process according to any of the preceding claims, wherein R¹⁰ is hydrogen.

8. The process according to any of the preceding claims, wherein R⁶ is hydrogen, halogen, CN, C₁-C₄-alkyl or C₁-C₄-alkoxy.

9. The process according to the preceding claims, wherein m is 0, 1, 2 or 3.

10. The process according to any of the preceding claims, wherein R¹ is fluorine, chlorine, bromine or methoxy, R², R³ and R¹⁰ are each hydrogen atoms, R⁶ is hydrogen, fluorine, chlorine, bromine, CN, methoxy or ethoxy and m is 0, 1, 2 or 3.

11. The process according to any of the preceding claims, wherein the reaction is performed within the temperature range from -20 to 100°C.

12. The process according to claim 11, wherein the reaction is performed within the temperature range from -10°C to 70°C.

13. The process according to any of the preceding claims, wherein the reaction is performed under irradiation or ultrasound.

14. The process according to any of claims 1 to 3 and 5 to 13, wherein the reaction is performed in the presence of at least one oxidizing agent under protective gas.

## Revendications

1. Procédé de fabrication d'un composé de la formule 3 : 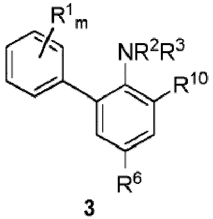 dans lequel un composé de la formule 1 : 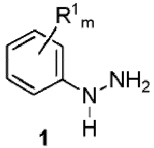 est mis en réaction avec un composé de la formule 2 : 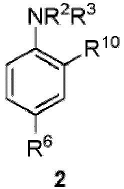 dans lesquelles
m représente 0, 1, 2, 3, 4 ou 5 ;
les R¹ représentent chacun indépendamment halogène, alkyle, haloalkyle, hydroxy, hydroxyalkyle, alcoxy, haloalcoxy, alkylthio, cycloalkyle, haloalkylthio, alcényle, alcynyle, amino, nitro, cyano, -SO₃R⁵, -SO₂NH₂, -SO₂NHR⁴, -SO₂NR⁴R⁵, -COOR⁴, -CONHR⁴, -CONR⁴R⁵, -COR⁴,-OCOR⁴, -NR⁴R⁵, -NR⁴COR⁵, -NR⁴SO₂R⁵, alkylcarbonyle, haloalkylcarbonyle, alcénylcarbonyle, alcoxycarbonyle, haloalcoxycarbonyle, alcényloxycarbonyle, alkylsulfonyle, haloalkylsulfonyle, alkylimino, aryle, aryloxy, arylcarbonyle, arylalkyle, hétéroarylalkyle, arylalcoxycarbonyle, arylalkylimino ou hétéroaryle ;
R² et R³ représentent hydrogène,
les n représentent chacun indépendamment 1, 2, 3, 4, 5, 6, 7, 8, 9 ou 10 ;
les R⁴ représentent chacun indépendamment hydrogène, alkyle, cycloalkyle, haloalkyle, arylalkyle, hétéroarylalkyle, aryle ou hétéroaryle ;
les R⁵ représentent chacun indépendamment hydrogène, alkyle, cycloalkyle, haloalkyle, arylalkyle, hétéroarylalkyle, aryle ou hétéroaryle ;
les R⁶ représentent chacun indépendamment hydrogène, halogène, alkyle, alcényle, alcynyle, cycloalkyle, arylalkyle, hétéroarylalkyle, haloalkyle, hydroxyalkyle, alcoxyalkyle, aryloxyalkyle, hétéroaryloxyalkyle, aminoalkyle, -(CH₂)ₙ-NR⁴R⁵, -COOH,-CHO, -CN, -COR⁴, alkylcarbonyle, haloalkylcarbonyle, cycloalkylcarbonyle, arylalkylcarbonyle, alcénylcarbonyle, arylcarbonyle, hétéroarylcarbonyle,-COOR⁴, alcoxycarbonyle, haloalcoxycarbonyle, cycloalcoxycarbonyle, arylalcoxycarbonyle, alcényloxycarbonyle, aryloxycarbonyle, hétéroaryloxycarbonyle, -CONHR⁴, -CONR⁴R⁵, amino, nitro, -NHR⁴, -NR⁴R⁵, 1-pyrrolidino, 1-pipéridino, 1-morpholino, alkylimino, cycloalkylimino, haloalkylimino, arylalkylimino, -NR⁴COR⁵, NR⁴COOR⁵,-NR⁴SO₂R⁵, hydroxy, alcoxy, haloalcoxy, cycloalcoxy, arylalkyloxy, aryloxy, hétéroaryloxy, -OCOR⁴, alkylcarbonyloxy, haloalkylcarbonyloxy, cycloalkylcarbonyloxy, arylalkylcarbonyloxy, arylcarbonyloxy, hétéroarylcarbonyloxy, -OCONR⁴R⁵, -O-(CH₂)ₙ-OR⁴, -O-(CH₂)ₙ-NR⁴R⁵, -O-(CH₂)ₙ-NR⁴COR⁵, -O-(CH₂)ₙ-NR⁴COOR⁵, -O-(CH₂)ₙ-COOR⁴, -O-(CH₂)ₙ-CONHR⁴, -O-(CH₂)ₙ-CONR⁴R⁵, -O-(CH₂)ₙ-SO₃R⁴, -O-(CH₂)ₙ-SO₂R⁴, -O-(CH₂)ₙ-CN,-SH, alkylthio, haloalkylthio, cycloalkylthio, arylalkylthio, arylthio, hétéroarylthio, alkylsulfonyle, haloalkylsulfonyle, cycloalkylsulfonyle, arylalkylsulfonyle, arylsulfonyle, hétéroarylsulfonyle, -SO₂NH₂, -SO₂NHR⁴, -SO₂NR⁴R⁵, -SO₃R⁵, aryle ou hétéroaryle ;
les R¹⁰ représentent chacun indépendamment hydrogène, halogène, alkyle, haloalkyle, hydroxyalkyle, cycloalkyle, arylalkyle, hétéroarylalkyle, -(CH₂)_{q}-NR⁴R⁵, -(CH₂)_{q}-NR⁴COR⁵, -(CH₂)_{q}-NR⁴COOR⁵, -(CH₂)_{q}-COOR⁴, -(CH₂)_{q}-CONHR⁴, -(CH₂)_{q}-CONR⁴R⁵, -(CH₂)_{q}-SO₃R⁴, -(CH₂)_{q}-CN, aryle ou hétéroaryle ;
les q représentent chacun indépendamment 1, 2, 3, 4 ou 5 ;
le radical alkyle dans alcoxy, alkylcarbonyle, alkylcarbonyloxy, alcoxycarbonyle, alkylsulfonyle, hydroxyalkyle, alcoxyalkyle, aryloxyalkyle, hétéroaryloxyalkyle, aminoalkyle, arylalkylcarbonyle, arylalcoxycarbonyle, arylalkyloxy, arylalkyle, hétéroarylalkyle, arylalkylcarbonyloxy, alkylthio, arylalkylthio et arylalkylsulfonyle portant éventuellement 1, 2 ou 3 substituants, qui sont choisis parmi halogène, alkyle, haloalkyle, cycloalkyle, alcoxy et haloalcoxy ;
le cycloalkyle portant éventuellement 1, 2 ou 3 substituants, qui sont choisis parmi alkyle, alcoxy et halogène ;
le radical cycloalkyle dans cycloalcoxy et cycloalkylthio portant éventuellement 1, 2 ou 3 substituants, qui sont choisis parmi alkyle et halogène ;
le radical cycloalkyle dans cycloalkylcarbonyle, cycloalcoxycarbonyle, cycloalkylcarbonyloxy et cycloalkylsulfonyle portant éventuellement 1, 2 ou 3 substituants, qui sont choisis parmi halogène, alkyle, haloalkyle, cycloalkyle, alcoxy et haloalcoxy ; le radical alcényle dans alcénylcarbonyle et alcényloxycarbonyle portant éventuellement 1, 2 ou 3 substituants, qui sont choisis parmi halogène, alkyle, haloalkyle, cycloalkyle, alcoxy et haloalcoxy ; le radical cycloalkylidène dans cycloalkylimino portant éventuellement 1, 2 ou 3 substituants, qui sont choisis parmi halogène, alkyle, haloalkyle, cycloalkyle, alcoxy et haloalcoxy ; et
le radical alkylène dans alkylimino portant éventuellement 1, 2 ou 3 substituants, qui sont choisis parmi halogène, alkyle, haloalkyle, cycloalkyle, alcoxy et haloalcoxy ;
**caractérisé en ce que** la réaction est réalisée en présence d'au moins un oxydant, l'oxydant étant l'oxygène, l'oxygène de l'air, MnO₂, Mn(OAc)₃, KMnO₄, NaIO₄, K₃[Fe(CN)₆], KO₂ ou H₂O₂.

2. Procédé selon la revendication 1, **caractérisé en ce que** la réaction est réalisée en présence d'au moins un solvant.

3. Procédé selon l'une quelconque des revendications précédentes, **caractérisé en ce que** la réaction est réalisée en présence d'au moins une base, d'au moins un solvant et d'au moins un oxydant.

4. Procédé selon l'une quelconque des revendications précédentes, **caractérisé en ce que** l'oxydant est l'oxygène ou l'oxygène de l'air.

5. Procédé selon l'une quelconque des revendications 1 à 3, **caractérisé en ce que** l'oxydant est MnO₂, Mn(OAc)₃, KMnO₄, NaIO₄, K₃[Fe(CN)₆], KO₂ ou H₂O₂.

6. Procédé selon l'une quelconque des revendications précédentes, **caractérisé en ce que** R¹ représente halogène, alkyle en C₁-C₄ ou alcoxy en C₁-C₄.

7. Procédé selon l'une quelconque des revendications précédentes, **caractérisé en ce que** R¹⁰ représente hydrogène.

8. Procédé selon l'une quelconque des revendications précédentes, **caractérisé en ce que** R⁶ représente hydrogène, halogène, CN, alkyle en C₁-C₄ ou alcoxy en C₁-C₄.

9. Procédé selon l'une quelconque des revendications précédentes, **caractérisé en ce que** m représente 0, 1, 2 ou 3.

10. Procédé selon l'une quelconque des revendications précédentes, **caractérisé en ce que** R¹ représente fluor, chlore, brome ou méthoxy, R², R³ et R¹⁰ représentent des atomes d'hydrogène, R⁶ représente hydrogène, fluor, chlore, brome, CN, méthoxy ou éthoxy, et m représente 0, 1, 2 ou 3.

11. Procédé selon l'une quelconque des revendications précédentes, **caractérisé en ce que** la réaction est réalisée dans la plage de température allant de -20 à 100 °C.

12. Procédé selon la revendication 11, **caractérisé en ce que** la réaction est réalisée dans la plage de température allant de -10 °C à 70 °C.

13. Procédé selon l'une quelconque des revendications précédentes, **caractérisé en ce que** la réaction est réalisée sous exposition à un rayonnement ou exposition à des ultrasons.

14. Procédé selon l'une quelconque des revendications 1 à 3 et 5 à 13, **caractérisé en ce que** la réaction est réalisée en présence d'au moins un oxydant sous un gaz de protection.
